(19)

Europäisches
Patentamt
European
Patent Office
Office européen
des brevets

(11) **EP 1 782 767 A1**

(12) **EUROPEAN PATENT APPLICATION**
published in accordance with Art. 158(3) EPC

(43) Date of publication:
**09.05.2007 Bulletin 2007/19**

(51) Int Cl.:
*A61F 7/08* *(2006.01)*  *A61F 13/00* *(2006.01)*
*A61F 13/02* *(2006.01)*  *C09K 5/16* *(2006.01)*

(21) Application number: 05765729.8

(22) Date of filing: **14.07.2005**

(86) International application number:
**PCT/JP2005/013017**

(87) International publication number:
**WO 2006/006664 (19.01.2006 Gazette 2006/03)**

(84) Designated Contracting States:
**DE FR GB**

(30) Priority: **14.07.2004 JP 2004207846**

(71) Applicant: **Mycoal Products Corporation**
**Tochigi-chi, Tochigi 328-0067 (JP)**

(72) Inventor: **DODO, T.,**
**MYCOAL PRODUCTS CORPORATION**
**Tochigi-shi, Tochigi 3280067 (JP)**

(74) Representative: **Thun, Clemens**
**Mitscherlich & Partner**
**Sonnenstrasse 33**
**80331 München (DE)**

(54) **FLEXIBLE HEATING ELEMENT**

(57) To provide a flexible heat generating body which is simple in structure, has excellent adaptability to the body of a user, is able to be fixed to any portion of the body upon sticking or winding, does not cause separation or coming off even upon stretching in response to the movement of the body, reaches a temperature region of use relatively rapidly and is able to give controlled heat energy.

A flexible heat generating body provided with a flexible exothermic part which is prepared by mediating a plural number of a heat generating composition molded body resulting from molding a heat generating composition containing surplus water as a connecting substance between a substrate and a covering material and heat sealing the periphery of the heat generating composition molded body, thereby constituting a sectional exothermic part containing the heat generating composition molded body and a sectioned part as heat sealed, which is **characterized in that** the heat generating composition contains, as essential components, an iron powder, a carbon component, a reaction accelerator and water and does not contain a flocculant aid, a flocculant, an agglomeration aid, a dry binder, a dry binding agent, a dry binding material, a sticky raw material, a thickener and an excipient; that the surplus water has a water mobility value of from 0.01 to 20;

The water in the heat generating composition does not function as a barrier layer; that the heat generating composition molded body has a volume of from 0.01 to 30 cm$^3$; that a ratio of the capacity of the sectional exothermic part to the volume of the heat generating composition molded body is from 0.6 to 1.0; that the sectioned exothermic part has a maximum height of from 0.1 to 10 mm; that the sectioned part has a width of from 0.3 to 30 mm; that a flexibility holding member is provided in at least one side of the flexible exothermic part; that a bending resistance in at least one direction of the flexible exothermic part is not more than 100 mm; that a rate of bending resistance in at least one direction of the flexible exothermic part is 50 or more; that a bending resistance in at least one direction of the flexibility holding member is not more than 200 mm; that a bending resistance in at least the longitudinal direction of the flexible heat generating body is not more than 200 mm; that the substrate is substantially planar and does not have a pocket, an accommodating division or an accommodating zone; and that the substrate and/or the covering material has permeability to air in at least a part thereof.

EP 1 782 767 A1

# FIG. 2 (a)

# FIG. 2 (b)

**Description**

[Technical Field]

The present invention relates to a pliable and freely stretchable flexible heat generating body which is flexible as a whole, is pliable, is free from an uncomfortable feeling in wearing, is well fitting to the body, well follows the movement of the body, is able to freely wear an arbitrary desired place of the body and when installed in a bending part, is free from separation. The invention preferably relates to a flexible heat generating body which is used for relaxation of pains of respective sites of the body, is persistent and simply imparts comfortable warmth.

[Background Art]

In conventional body warmers, flexible heat generating bodies in which raw materials capable of causing heat generation (for example, an iron powder, active carbon, vermiculite, salt, and a water absorptive resin) are packaged by a porous film or a non-woven fabric alone or a composite body thereof are used as a chemical body warmer. Then, a number of flexible heat generating bodies are used as a body warmer which is subjected to sticking processing at need and stuck to the body. Also, in the foregoing flexible heat generating bodies of a sticking type, porous films, or non-woven fabrics or other fiber raw materials for the purpose of protecting them or improving a touch feeling to the skin are used as a packaging material.
On the other hand, a chemical body warmer is used as means for remedy of symptoms including pains, muscular or joint stiffness, neuralgia, and rheumatism. As a method for fixing this, a sticking type, a winding type, and so on are employed.
As the winding type, Patent Document 1 discloses a freely stretchable body warmer in which a chemical body warmer is installed in a sheet-like non-stretchable portion composed of a stretchable portion and a non-stretchable portion of a flexible material.
Also, Patent Document 2 discloses a thermal body wrap having a heat cell fixed to a flexible material.
In the foregoing conventional body warmers, since porous materials for the purpose of improving a touch feeling to the skin such as non-woven fabrics and other fiber raw materials do not have stretchability, the use for bending parts of the body is difficult so that winding by a bandage, etc. was required. Also, especially in the sticking body warmer, since it does not expand and contract as described previously, it is not fitting to the movement of the body so that there was a problem that even sticking, it causes separation and falling.
Also, a throwaway body warmer itself is poor in flexibility so that when making it go along the body, it produces a tight feeling and further becomes hard during the use. Thus, the fitness to the body is poor, and a feeling for use is not good.
[Patent Document 1] Utility Model Registration No. 2595980
[Patent Document 2] JP-A-2001-513394

[Disclosure of the Invention]

[Problems that the Invention is to Solve]

An object of the invention is to provide a flexible heat generating body which is simple in structure, has excellent adaptability to the body of a user, is able to be fixed to any portion of the body upon sticking or winding, does not cause separation or coming off even upon stretching in response to the movement of the body, reaches a temperature region of use relatively rapidly and is able to give controlled heat energy.

[Means for Solving the Problems]

As set forth in claim 1, a flexible heat generating body of the invention is a flexible heat generating body provided with a flexible exothermic part which is prepared by mediating a plural number of a heat generating composition molded body resulting from molding a heat generating composition containing surplus water as a connecting substance between a substrate and a covering material and heat sealing the periphery of the heat generating composition molded body, thereby constituting a sectional exothermic part containing the heat generating composition molded body and a sectioned part as heat sealed, which is characterized in that:

> 1) the heat generating composition contains, as essential components, an iron powder, a carbon component, a reaction accelerator and water and does not contain a flocculant aid, a flocculant, an agglomeration aid, a dry binder, a dry binding agent, a dry binding material, a sticky raw material, a thickener and an excipient;
> 2) the surplus water has a water mobility value of from 0.01 to 20;

3) the water in the heat generating composition does not function as a barrier layer;

4) the heat generating composition molded body has a volume of from 0.01 to 30 cm$^3$;

5) a ratio of the capacity of the sectional exothermic part to the volume of the heat generating composition molded body is from 0.6 to 1.0;

6) the sectioned exothermic part has a maximum height of from 0.1 to 10 mm;

7) the sectioned part has a width of from 0.3 to 30 mm;

8) a flexibility holding member is provided in at least one side of the flexible exothermic part;

9) a bending resistance in at least one direction of the flexible exothermic part is not more than 100 mm;

10) a rate of bending resistance in at least one direction of the flexible exothermic part is 50 or more;

11) a bending resistance in at least one direction of the flexibility holding member is not more than 200 mm;

12) a bending resistance in at least the longitudinal direction of the flexible heat generating body is not more than 200 mm;

13) the substrate is substantially planar and does not have a pocket, an accommodating division or an accommodating zone; and

14) the substrate and/or the covering material has permeability to air in at least a part thereof.

Also, a flexible heat generating body as set forth in claim 2 is characterized in that in the flexible heat generating body as set forth in claim 1, the flexibility holding member is constituted of a non-stretchable portion and a stretchable portion.

Also, a flexible heat generating body as set forth in claim 3 is characterized in that in the flexible heat generating body as set forth in claim 2, the flexibility holding member is constituted of a sheet-like material in which a non-stretchable portion and a stretchable portion are integrally formed in a sheet-like form; and the flexible exothermic part is provided in the non-stretchable portion of the sheet-like material.

Also, a flexible heat generating body as set forth in claim 4 is characterized in that in the flexible heat generating body as set forth in claim 1, the flexible exothermic part is sandwiched by the flexibility holding members constituted of a flexible material; and a part of the flexibility holding member is constituted of a stretchable material.

Also, a flexible heat generating body as set forth in claim 5 is characterized in that in the flexible heat generating body as set forth in claim 1, the flexibility holding member is constituted of a longitudinal non-stretchable raw material; and plural notch parts are alternately provided in the crossing direction to the longitudinal direction of the non-stretchable raw material, thereby forming the stretchable portion.

Also, a flexible heat generating body as set forth in claim 6 is characterized in that in the flexible heat generating body as set forth in claim 5, the alternate notch parts have a rate of extension in the notch direction of from 1.1 to 10 times and a tensile strength of 3 N/50 mm or more.

Also, a flexible heat generating body as set forth in claim 7 is characterized in that in the flexible heat generating body as set forth in claim 5, the alternate notch parts are provided in the vicinity of either side of the flexible exothermic part.

Also, a flexible heat generating body as set forth in claim 8 is characterized in that in the flexible heat generating body as set forth in claim 2, the stretchable portion is constituted of a film to which stretchability is imparted by an elastomer, an expanded body, a non-woven fabric, a fabric, a laminate thereof, or a flexible material body of a scrim-supported laminate.

Also, a flexible heat generating body as set forth in claim 9 is characterized in that in the flexible heat generating body as set forth in claim 1, the flexible heat generating body is constituted such that when wound up around a prescribed site, the both end parts thereof are superposed on each other.

Also, a flexible heat generating body as set forth in claim 10 is characterized in that in the flexible heat generating body as set forth in claim 1, a thermal buffer sheet is provided in a portion coming into contact with an adherend of the flexible exothermic part.

Also, a flexible heat generating body as set forth in claim 11 is characterized in that in the flexible heat generating body as set forth in claim 1, a fixing measure for fixing to an adherend is provided in the flexible heat generating body.

Also, in the flexible heat generating body, it is preferable that the fixing measure is an adhesive layer; and that the adhesive layer contains at least one member selected from additional components consisting of a water retaining agent, a water absorptive polymer, a pH adjusting agent, a surfactant, an organosilicon compound, a hydrophobic polymer compound, a pyroelectric substance, an antioxidant, an aggregate, a fibrous material, a moisturizer, a functional substance, and a mixture thereof.

[Advantages of the Invention]

In the light of the above, according to the flexible heat generating body of the invention, the following advantages are brought.

1. Since the flexible heat generating body of the invention is freely stretchable, it can be stuck to any portion of the body. In particular, the flexible heat generating body of the invention can be applied to bending portions of the body, back, waist, upper arm, lower arm, upper leg and lower leg, knee portion of foot, and elbow portion of arm, and even when such a portion is bent and stretched, there is no possibility of occurrence of separation and coming off.

2. Since the flexible heat generating body of the invention can be constituted of a simple structure by only installing a flexible heat generating body in a sheet-like material having flexibility as a whole, the flexibility can be kept in each portion. Thus, even when following bending and stretching of the body, a force is applied to the sheet-like material, the sheet causes neither breakage nor coming off. In addition, since the flexible exothermic part is installed in the non-stretchable portion, even when the stretchable portion expands and contracts in response to the movement of the body, no stress is loaded in the installing portion of the exothermic part, and there is no possibility that the exothermic part comes off from the sheet.

3. Since the flexible exothermic part has plural sectional exothermic parts, a rough "rugged" feeling and a stimulus are largely reduced. Furthermore, it has excellent adaptability to the body of a user, uniformly supplies heat and enhances comfort.

4. The flexible heat generating body of the invention has excellent adaptability to the body of a user such that the sectional exothermic part gives a controlled persistent temperature and reaches a temperature region of use relatively rapidly.

[Best Modes for Carrying Out the Invention]

The flexible heat generating body of the invention is premised on a flexible heat generating body provided with a flexible exothermic part which is prepared by mediating a plural number of a heat generating composition molded body resulting from molding a heat generating composition containing surplus water as a connecting substance between a substrate and a covering material and heat sealing the periphery of the heat generating composition molded body, thereby constituting a sectional exothermic part containing the heat generating composition molded body and a sectioned part as heat sealed.

A capacity of the sectional exothermic part or a volume of the heat generating composition molded body is usually from 0. 015 to 500 $cm^3$, preferably from 0. 04 to 30 $cm^3$, more preferably from 0.1 to 30 $cm^3$, further preferably from 1 to 30 $cm^3$, and still further preferably from 3 to 20 $cm^3$.

In the sectional exothermic part, when the sectional exothermic part which is an accommodating region of the heat generating composition is filled with the heat generating composition molded body, a volume ratio of the volume of the heat generating composition molded body which is an occupying region of the heat generating composition molded body to the capacity of the sectional exothermic part which is an accommodating region of the heat generating composition is usually from 0. 6 to 1, preferably from 0. 7 to 1, more preferably from 0.8 to 1, and further preferably from 0.9 to 1.0.

Furthermore, a width of the sectioned part which is a space between the sectional exothermic parts is not limited so far as sectioning can be achieved. It is usually from 0.1 to 50 mm, preferably from 0.3 to 50 mm, more preferably from 0.3 to 50 mm, further preferably from 0.3 to 40 mm, still further preferably from 0.5 to 30 mm, even further preferably from 1.0 to 20 mm, and even still further preferably from 3 to 10 mm.

Incidentally, the heat generating composition molded body or the sectional exothermic part may have any shape. The shape may be a planar shape, and examples thereof include a circular shape, an elliptical shape, a polygonal shape, a star shape, and a flower shape. Also, the shape may be a three-dimensional shape, and examples thereof include a polygonal pyramidal shape, a conical shape, a frustum shape, a spherical shape, a parallelepiped shape, a cylindrical shape, a semi-pillar shape, a semicylindroid shape, a semicylidrical shape, a pillar shape, and a cylindroid shape. Furthermore, in these shapes, the corner may be rounded, thereby processing the corner in a curvilinear or curved state, or the central part may be provided with a concave.

Furthermore, the "volume of the heat generating composition molded body of the invention" as referred to herein means a volume of the heat generating composition molded body or compressed heat generating composition molded body.

Furthermore, the "capacity of the sectional exothermic part" as referred to herein means an internal capacity of the sectional exothermic part having a heat generating composition molded body accommodated therein.

The flexible exothermic part of the invention is provided with a flexibility holding member made of a flexible raw material in at least one side thereof.

The flexible material which constitutes the flexibility holding member is not limited so far as it has flexibility and is able to hold an exothermic part which is constituted of sectional exothermic parts.

The flexible material is not limited so far as it has flexibility and may have any of extensibility, non-extensibility, stretchability or non-stretchability. Examples thereof include films, sheets, expanded bodies, non-woven fabrics, fabrics, and knitted fabrics, each of which is made of a synthetic resin or a natural material. Examples of the synthetic resin include polyethylene, polypropylene, polyamides (for example, nylons), polyesters, polyvinyl chloride, polyvinylidene chloride, polyurethane, polystyrene, ethylene-vinyl acetate copolymer saponified products, and ethylene-vinyl acetate copolymers.

Examples of the natural raw material include paper and cotton.

Furthermore, the flexible material may have elasticity. According to this, in the case of using as a substrate, a cushioning material, etc., it is more suitable.

Though the thickness of the flexible material is not limited, it is preferably from 5 μm to 10 mm, more preferably from 5 μm to 4 mm, further preferably from 0.1 mm to 4 mm, still further preferably from 0.5 mm to 2 mm, and even further preferably from 0.5 mm to 1.5 mm.

The expanded body is not particularly limited so far as it is preferably made of a plastic, has elasticity and meets a prescribed tensile strength. Examples thereof include expanded polyurethane, expanded polyolefins (for example, expanded polyethylene), and expanded polyacrylates. Of these, expanded polyurethane and expanded polyethylene, both of which have a large tensile strength, are preferable. In particular, when the expanded body is soft and elastic, a touch is good, and in the case of using as a substrate coming into contact with the skin or the like, it is suitable. The "elasticity" as referred to herein refers to properties that the expanded body is shrunk against a pushing force, whereas when the force is eliminated, it is returned to the original state.

In the case where the flexibility holding member of the invention has stretchability, examples thereof include ones in which the flexibility holding member is non-stretchable and is constituted as a stretchable raw material by providing plural notch parts as disposed alternately with an angle against the longitudinal direction, preferably a perpendicular direction to the longitudinal direction and penetrating in the thickness direction and combinations thereof.

A bending resistance in at least one direction of the flexible exothermic part is usually not more than 100 mm, preferably not more than 80 mm, more preferably not more than 50 mm, further preferably not more than 30 mm, and still further preferably not more than 20 mm. Furthermore, a rate of bending resistance in at least one direction of the flexible exothermic part is usually not more than 50, preferably not more than 40, and more preferably not more than 30. The bending resistance and rate of bending resistance are kept at least at a temperature between 20°C and 60°C. A bending resistance in at least one direction of the flexibility holding member is usually not more than 200 mm, preferably not more than 100 mm, more preferably not more than 80 mm, further preferably not more than 60 mm, and still further preferably not more than 50 mm.

A bending resistance of the heat generating composition molded body and/or the flexible heat generating body is usually not more than 200 mm, preferably not more than 100 mm, more preferably not more than 80 mm, further preferably not more than 60 mm, and still further preferably not more than 50 mm. Furthermore, the bending resistance of the substrate and the covering material is kept at least at a temperature between 20°C and 60°C, too.

In the flexible heat generating body of the invention, in the case where heat generating composition molded bodies of a parallelepiped shape are provided at intervals such that an absolute value of a difference between bending resistances in the two directions as intersecting directions becomes maximum, the flexible heat generating body is very pliable in one direction and rigid in the other direction. Accordingly, it is possible to make the flexible heat generating body well go along the body so that it is optimum for taking warmth of the body or relieving or remedying various symptoms.

To provide strength and weakness in bending resistance in the longitudinal direction and the short direction of the flexible exothermic part is determined on what direction is provided the sectional exothermic part. Such may be selected depending upon the utility.

When a flexible heat generating body in which rectangular exothermic parts in which sectional exothermic parts are provided in a striped form in the longitudinal direction are installed in the longitudinal flexibility holding member adaptive to the respective longitudinal directions thereof is taken as an example, by making the bending resistance of the exothermic parts in the longitudinal direction high and making the bending resistance in the short direction substantially orthogonal thereto extremely low, since at the time of installing, the longitudinal direction is rigid, the flexible heat generating body is installed therein while relying thereupon, whereas since the short direction is soft, it can be easily fitted to a warmth taking part.

The "bending resistance" as referred to in the invention exhibits rigidity (tension or nerve) or flexibility and follows the A method according to JIS L1096 (45° cantilever method), except for using a heat generating body itself as a sample. That is, a heat generating body is placed on a horizontal table having a smooth surface and having a slope at an angle of 45° in one end thereof such that one side thereof coincides with a scale base line. Next, the heat generating body is slowly slid toward the slope by an appropriate method, and when a central point of the one end of the heat generating body comes into contact with the slope A, the position of the other end is read by a scale. The bending resistance is exhibited by a length (mm) for which the heat generating body moves. Respective five sheets of heat generating body are measured, and the bending resistance (calculated down to the integral place) is expressed by an average value of lengths measured in the length direction and the width direction, or in one direction and the orthogonal direction thereto. However, in the measurement, in the case of measuring an adhesive layer-provided heat generating body such that the adhesive side is faced at the horizontal table side, while the adhesive side provided with a separator is faced at the horizontal table side. In any way, a measured value in the side at which a minimum bending resistance is measured is employed.

Furthermore, in the measurement, the following must be taken into consideration.

(1) A heat generating composition-incorporated exothermic part of the heat generating body is to retain on the horizontal table to an extent of 5 mm or more in width x 20 mm or more in length. However, the length is to cross a region where the heat generating composition is present or to cross linearly a region where the heat generating composition is present and a region where the heat generating composition is not present.

(2) In the case of an adhesive layer-provided heat generating body, a plastic film having a bending resistance of not more than 30 mm, or a limp and soft film such as a limp film having a thickness of not more than 50 μm, and preferably not more than 25 μm and a plastic film in which wrinkles are formed by lightly crumpling is to be used as a separator of the adhesive layer and provided along the adhesive layer. Furthermore, with respect to the bending resistance of the substrate and/or the covering material, a specimen of 100 mm x 200 mm is prepared, and a bending resistance in the 200 mm direction is employed.

In the invention, a bending resistance in at least one direction is usually not more than 100 mm, preferably not more than 80 mm, more preferably not more than 50 mm, further preferably not more than 30 mm, and still further preferably not more than 20 mm.

A rate of bending resistance of the heat generating body or exothermic part in the invention is a rate of bending resistance to the full length of the heat generating body or exothermic part in one direction and is calculated according to the following expression.

$$\texttt{(Rate of bending resistance)} = \texttt{(A/B)} \times \texttt{100}$$

Wherein A represents a bending resistance of the heat generating body or exothermic part in one direction; and B represents the full length of the heat generating body or exothermic part in the foregoing one direction.

In the invention, a rate of bending resistance in at least one direction is usually not more than 50, preferably not more than 40, and more preferably not more than 30.

A ratio of bending resistance in the invention is a ratio of a bending resistance in one direction to a smaller bending resistance in bending resistances in the directions orthogonal thereto in the plane orthogonal to the thickness direction of the heat generating body or exothermic part. The ratio of bending resistance is preferably 2 or more.

In the invention, in the case of a heat generating body having sectional exothermic parts provided at intervals in the striped form, a heat generating body provided with sectional exothermic parts of a parallelepiped shape at intervals in the striped form in which a maximum absolute value of a difference between bending resistances in the two directions as intersecting directions, a heat generating body further provided with an adhesive layer, and a heat generating body provided with adhesive layers at intervals in the striped form are very flexible in one direction and rigid in one direction.

Thus, these heat generating bodies relieve symptoms such as stiff shoulders, lower-back pain, and muscular fatigue and especially exhibit efficacy for relieving a symptom of menstrual pain. In addition, these heat generating bodies are able to be wound in a size substantially equal to the width dimension in the width direction of the heat generating body, become compact and are convenient for accommodation. Furthermore, in the case of a separator-provided heat generating body, by using a separator having a low bending resistance, winding is possible.

Furthermore, in the case of providing a heat generating body along the body, the body includes many two-dimensional curves, and in shoulders, legs, abdomen, waist, arms, and the like, one direction is substantially linear, and the other two directions are formed of a substantially curved surface. Accordingly, since the heat generating body of the invention which is able to form a substantially linear surface in one direction and a curved surface in the other two directions is able to form a two-dimensional curved surface, it is able to well follow the body and is optimum for warming of the body and relaxation or treatment of various symptoms.

Furthermore, in the heat generating body of the invention, by adjusting the size or space of the convex sectional exothermic part, an exothermic part which is flexible and exhibits a uniform temperature distribution or an exothermic part exhibiting a pattern-like temperature distribution is obtainable. By the pattern-like temperature distribution, it is possible to improve a meridian effect of the warming part.

In the flexible heat generating body having sectional exothermic parts, a minimum bending resistance of the bending resistance on the surface orthogonal to the thickness direction is preferably not more than 50 mm, more preferably not more than 40 mm, further preferably not more than 30 mm, and still further preferably from 5 to 30 mm.

The bending resistance and bending resistance ratio are kept at least at a temperature between 20°C and 60°C.

The "water retention" as referred to herein is a value as measured and calculated in the following method. That is, about 1 g of a sample fiber as prepared by cutting into a length of about 5 cm and well opening is dipped in pure water, and after elapsing 20 minutes (at 20 °C), water among the fibers is removed using a centrifuge by revolution at 2,000 rpm. A weight (W1) of the thus prepared sample is measured. Next, the sample is dried in a vacuum dryer at 80 °C until it becomes constant in weight, thereby measuring a weight (W2). A water retention is calculated according to the following expression.

[Water retention (%)] = [(W1 - W2)/W2] x 100

In the invention, the water retention is preferably 20 % or more.

In the tensile test of the invention, the packaging material is cut into a size of 2.5 cm in width x about 20 cm in length according to JIS L1096. A sample is applied with a tensile force sufficient for eliminating all relaxations in ends of the small piece without applying a load to a load cell, nipped by a chuck with a chuck interval of 10 cm, and placed in a unit. Next, the temperature of the sample is stabilized at a desired test temperature.

(1) Judgment test of non-elastic body:

After stabilizing the sample at 25 °C, the chuck interval is elongated by 5 mm at a cross head speed of about 50 cm/min, and the sample is then taken out from the unit.

In the case where the length after elongation is longer than that before the elongation, a permanent set is generated, and therefore, such a sample is defined as a non-elastic body.

Furthermore, a sample which generates a deviation from the linear function relation between elongation and tensile strength and is admitted to fall outside the elastic deformation is also defined as a non-elastic body.

Furthermore, in an anisotropic sample, a sample which is admitted to be non-elastic in at least one direction is defined as a non-elastic body.

(2) Breaking strength at 25 °C:

After stabilizing at a test temperature of 25 °C, a unit is operated until the sample is broken, and when broken, a strength of the sample is read from a chart and defined as a breaking strength at 25 °C.

(3) Breaking strength at 90 °C:

After stabilizing at a test temperature of 90 °C, a unit is operated until the sample is broken, and when broken, a strength of the sample is read from a chart and defined as a breaking strength at 90 °C.

(4) Breaking elongation at 90 °C:

After stabilizing at a test temperature of 90 °C, a unit is operated until the sample is broken, and when broken, an elongation of the sample is read from a chart and defined as a breaking elongation at 90 °C.

According to JIS L1096, a sample having a size of 2.5 cm in width x 20 cm in length is nipped by a chuck with a chuck interval of 10 cm and elongated at a cross heat speed of about 50 cm/min until the chuck interval is increased by 5 mm by a tensile test at the circumferential temperature. When a sample after the test generates a permanent set in the elongation direction, or a sample which generates a deviation from the linear function relation between elongation and tensile strength and is admitted to fall outside the elastic deformation is also defined as a non-elastic body. Furthermore, in an anisotropic sample, a sample which is admitted to be non-elastic in at least one direction is defined as a non-elastic body.

The term "elastic" as referred to herein means a characteristic of a material such that when receiving a tensile force, the material is elongated or widened in the direction of the force, and when eliminating the force, it is returned to the original dimension.

More concretely, the term "elastic" means a directional characteristic such that an element or a structure receives a percentage strain H % exceeding 50 % and is then recovered within about 10 % of the original length Lb.

The percentage strain H % as used in this specification is defined as follows.

$$H \% = [(Lx - Lb)/Lb] \times 100$$

In the foregoing expression, Lx represents a length when elongated; and Lb represents an original length.

In order to make consistent comparison, it is preferable that the recovery of the element or structure is measured within 30 seconds after relieving from the length Lf at the time of elongation. When the element or structure is not recovered to the range within about 10 % within 30 seconds after relieving from 50 % of a percentage strain H %, it is considered that such an element or structure is all non-elastic. The non-elastic element or structure also include an element or structure which when receiving 50 % of a percentage strain H %, breaks and/or deforms permanently or reversibly.

The term "non-shrink properties at 90 °C" as referred to herein means that after holding at 90 °C for 3 minutes and then returning to room temperature, the length does not become shorter than the original length. In more detail, the term "non-shrink properties at 90 °C" means that after holding at 90 °C for 3 minutes and then returning to room temperature, a shrinkage factor is preferably not more than 15 %, more preferably not more than 10 %, further preferably not more than 8 %, still further preferably not more than 5 %, and even further preferably not more than 1 %.

This shrinkage factor is defined as follows.

$$S = [(Lb - L90)/Lb] \times 100$$

In the foregoing expression, S represents a shrinkage factor (%); Lb represents an original length; and L90 represents a length after holding at 90 °C for 3 minutes and then returning to room temperature.

In particular, it is preferable that in the case of laminating a heat generating composition molded body (also including a heat generating composition compressed body in the invention) on a packaging material not having an accommodating pocket by molding with a die, further covering a packaging material thereon and then sealing the laminate to prepare an exothermic part or heat generating body having a sectional exothermic part, a laminate of a thermoplastic resin-made fibrous material and a thermoplastic resin-made film-like material is used for at least one of the packaging materials.

The substrate is substantially planar and does not have a pocket, an accommodating division or an accommodating zone. Incidentally, the "accommodating division" and "accommodating zone" as referred to herein mean a member for accommodating the heat generating composition molded body. In more detail, the "pocket" as referred to herein means an accommodating pocket which is provided in advance in the substrate or the covering material for the purpose of accommodating the heat generating composition molded body and is a pocket as described in JP-T-2001-507593. For the time being, since irregularities which are not used for accommodating the heat generating composition molded body are not a pocket, even when such irregularities are present in the substrate, it is to be noted that this substrate is a substantially planar substrate.

Furthermore, the substrate and/or the cover material has permeability to air in at least a part thereof.

The permeability to air is not limited so far as the heat generation is kept. In the case of using the substrate and/or the cover material for usual heat generation, the air permeability is usually from 50 to 10,000 $g/m^2/24$ hr, preferably from 70 to 5,000 $g/m^2/24$ hr, more preferably from 100 to 2, 000 $g/m^2/24$ hr, and further preferably from 100 to 700 $g/m^2/24$ hr in terms of a moisture permeability by the Lyssy method.

When this moisture permeability is less 50 $g/m^2/24$ hr, the heat value is small and a sufficient thermal effect is not obtained, and therefore, such is not preferable. On the other hand, when it exceeds 10,000 $g/m^2/24$ hr, the exothermic temperature is high so that a problem in safety may possibly be generated, and therefore, such is not preferable.

However, the invention does not exclude the case where the moisture permeability exceeds 10,000 $g/m^2/24$ hr or is closed to a moisture permeability in the opened system depending upon the utility.

Incidentally, the substrate and the covering material in the invention are not distinguished from each other due to the material constitution, but a material on which the heat generating composition molded body is laminated is defined as a substrate, whereas a material which is subsequently put on the substrate or the heat generating composition molded body is defined as a covering material. Functions such as permeability to air are not concerned.

The heat generating composition of the invention is a moldable heat generating composition and contains, as essential components, an iron powder, a carbon component, a reaction accelerator and water but does not contain a flocculant aid, a flocculant, an agglomeration aid, a dry binding material, a dry binding agent, a dry binder, a sticky raw material, a thickener and an excipient.

The surplus water functions as a connecting substance to impart moldability the heat generating composition. The surplus water has a water mobility value of from 0.01 to 20.

Furthermore, in the invention, the water in the heat generating composition does not function as a barrier layer and causes an exothermic reaction upon contact with air.

Incidentally, in the invention, what water does not function as a barrier layer and causes an exothermic reaction upon contact with air means that water in a heat generating composition does not function as a barrier layer which is an air intercepting layer and immediately after the production of a heat generating composition, comes into contact with air, thereby immediately causing an exothermic reaction.

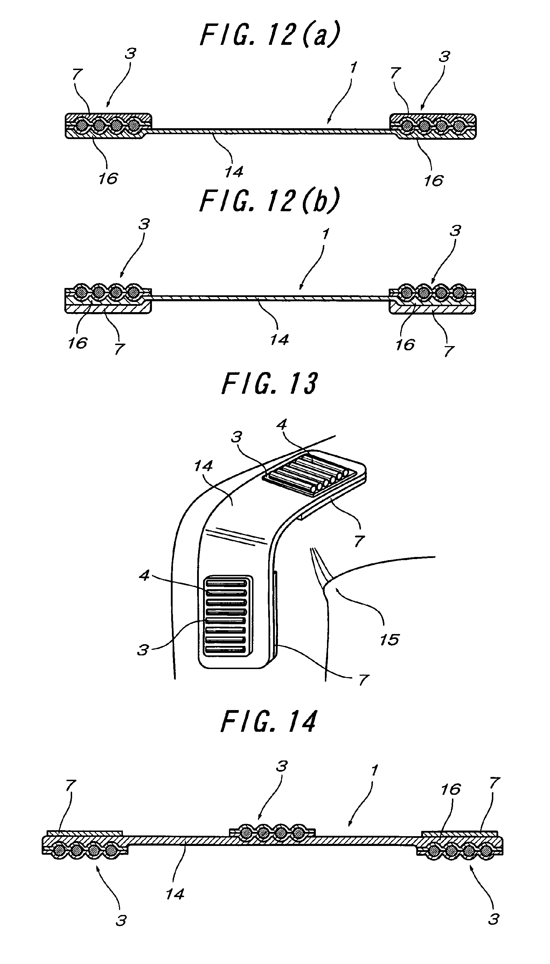

The "water mobility value" as referred to herein is a value showing an amount of surplus water which can transfer to the outside of the heat generating composition in water present in the heat generating composition. This water mobility value will be described below with reference to Figs. 13 to 17.

**EP 1 782 767 A1**

As shown in Fig. 13, a filter paper 18 of No. 2 (second class of JIS P3801) in which eight lines are drawn radiating from the central point with an interval of 45° is placed on a stainless steel plate 22 as shown in Figs. 14 and 15; a template 19 having a size of 150 mm in length x 100 mm in width and having a hollow cylindrical hole 20 having a size of 20 mm in inner diameter x 8 mm in height is placed in the center of the filter paper 18; a sample 21 is placed in the vicinity of the hollow cylindrical hole 20; and a stuffer plate 14 is moved on and along the template 19 and inserted into the hollow cylindrical hole 20 while stuffing the sample 21, thereby leveling the sample (force-in die molding).

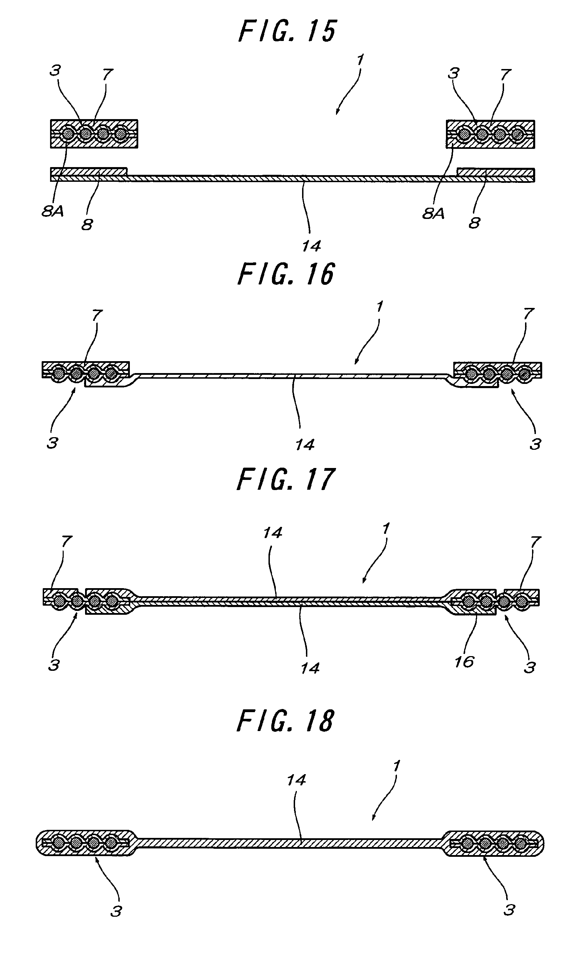

Next, as shown in Fig. 16, a non-water absorptive 70 μm-thick polyethylene film 17 is placed so as to cover the hole 20, and a flat plate 16 made of stainless steel having a size of 5 mm in thickness x 150 mm in length x 150 mm in width is further placed thereon and held for 5 minutes such that an exothermic reaction is not caused.

Thereafter, a shown in Fig. 17, the filter paper 18 is taken out, and an oozed-out locus of the water or aqueous solution is read as a distance 23 (unit: mm) from a periphery 24 as an edge of the hollow cylindrical hole to an oozed-out tip along the radiating lines. Similarly, a distance 23 from each of the lines is read, and eight values in total are obtained. Each of the eight values (a, b, c, d, e, f, g and h) which are read out is defined as a measured water content value. An arithmetic average value of the eight measured water content values is defined as a water content value (mm) of the sample.

Furthermore, the water content for the purpose of measuring a real water content value is defined as a compounded water content of the heat generating composition corresponding to the weight of the heat generating composition having a size of 20 mm in inner diameter x 8 mm in height or the like, similar measurement is conducted only with water corresponding to that water content, and a value as calculated in the same manner is defined as a real water content value (mm). A value obtained by dividing the water content value by the real water content value and then multiplying with 100 is a water mobility value.

That is, the water mobility value is represented by the following expression.

$$\text{(Water mobility value)} = \{[\text{Water content value (mm)}]/ [(\text{Real water content value (mm)})] \times 100$$

With respect to the same sample, five points are measured, and the five water mobility values are averaged, thereby defining an average value thereof as a water mobility value of the sample.

In the invention, a heat generating body can be formed only by laminating a heat generating composition molded body obtained by molding a heat generating composition having surplus water with a water mobility value of from 0.01 to 20 on a substrate, covering a covering material thereon, and sealing at least the periphery of the heat generating composition molded body. After accommodating it in a packaging material such as a substrate and a covering material, it is not necessary to add water. Accordingly, since the process is remarkably simplified, the invention is superior in view of the costs.

In the invention, the water mobility value (0 to 100) is preferably from 0.01 to 20, more preferably from 0.01 to 18, further preferably from 0.01 to 15, still further preferably from 0.01 to 13, even further preferably from 1 to 13, and even still further preferably from 3 to 13.

In a heat generating body using a heat generating composition molded body obtained by molding a moldable heat generating composition containing surplus water as a connecting substance according to the invention, the heat generating composition contains an appropriate amount of surplus water expressed by a water mobility value of from 0.01 to 20 as the connecting substance without using a flocculant aid, a dry binding agent, a flocculating agent, etc.

It is assumed that when the amount of surplus water in the heat generating composition is appropriate, the surplus water causes hydration against hydrophilic groups in the components of the composition due to a bipolar mutual action or hydrogen bond, etc. and that it is present even in the surroundings of hydrophobic groups while having high structural properties. Thus, it is assumed that the heat generating composition becomes in a state of a mud ball, thereby revealing moldability. This is connecting water as a connecting substance in some meaning. Besides, there is water in a state called as free water which can freely move, and it is thought that when the surplus water increases, the structure is softened, whereby the free water increases. Furthermore, controlling factors which an iron powder causes an oxidation reaction are an amount of existing water and a feed amount of oxygen to the surface of the iron powder. It is said that in a degree of water adsorbing film (less than 100 angstroms), the water is not sufficient and that the oxidation rate is small. When the adsorbing film becomes about 1 μm, the water content becomes sufficient. Furthermore, since the thickness of the water film is thin, feed of oxygen onto the surface of the iron powder becomes easy, whereby the oxidation rate becomes large. It is assumed that when the film becomes thicker to an extent that the adsorbing film exceeds 1 μm, the feed amount of oxygen is reduced. The present inventors have obtained knowledge that the water mobility value expressing the optimal water content at which moldability and oxidation rate in fixed levels or more are revealed is from 0.01 to 20, leading to accomplishment of the invention.

That is, by using an appropriate amount of surplus water, the respective component particles are coupled with each other by a surface tension of water, moldability is generated in the heat generating composition, and the water does not substantially function as a barrier layer. Thus, the heat generating composition comes into contact with air to generate heat. In addition, by using a heat generating composition using an active iron powder or an active heat generating composition using an active iron powder, the heat generating composition becomes a heat generating composition having remarkably excellent exothermic rising properties and high moldability. Furthermore, heat generation occurs without causing transfer of the water in the heat generating composition molded body as produced by a molding and laminating system into a packaging material or water absorptive sheet. In addition, by providing plural sectional exothermic parts of the heat generating composition molded body as sectioned by seal parts, it is possible to provide a heat generating body which has flexibility itself, is excellent in installation in places where flexibility is required, such as various places of a human body and curved bodies, and is excellent in feeling for use.

Furthermore, in the substrate, the covering material and the heat generating composition molded body, by temporarily adhering at least the covering material and the heat generating composition molded body to each other via a sticky layer and then heat sealing the periphery of the heat generating composition molded body and the surroundings of the heat generating body, certainty of heat seal is improved so that it becomes possible to design to make the production speed of a heat generating body high and make the heat seal width small.

The "moldability" as referred to in the invention exhibits that a molded body of the heat generating composition having a cavity or concave die shape is formed by force-through molding using a trimming die having a cavity or cast molding using a concave die, whereby after molding including mold release, the molding shape of the heat generating composition molded body is held.

When the moldability is revealed, since the shape is held until the heat generating composition molded article is at least covered by a covering material and a seal part is formed between the substrate and the covering material, sealing can be achieved in the periphery of the shape with a desired shape. Also, since so-called "spots" which are a collapsed piece of the heat generating composition are not scattered in the seal part, the sealing can be achieved without causing cutting in seal. The presence of the spots causes insufficient sealing.

1) Measurement device:

With respect to the measurement device, a stainless steel-made molding die (a plate having a size of 2 mm in thickness x 200 mm in length x 200 mm in width and having a cavity as treated by R5 in four corners of 60 mm in length x 40 mm in width in a central part thereof) and a fixable leveling plate are disposed above a travelable endless belt, and magnets (two magnets having a size of 12.5 mm in thickness x 24 mm in length x 24 mm in width are disposed in parallel) are disposed under the endless belt.

The magnets should cover a region of the leveling plate and the vicinity thereof and a region larger than a region covered by a cut side (40 mm) vertical to the advancing direction of the cavity of the molding die.

2) Measurement method:

With respect to the measurement method, a stainless steel plate having a size of 1 mm in thickness x 200 mm in length x 200 mm in width is placed on the endless belt of the measurement device, a polyethylene film having a size of 70 μm in thickness x 200 mm in length x 200 mm in width is placed thereon, and a stainless steel-made molding die is further placed thereon.

Thereafter, a leveling plate is fixed in a position of the cavity of the molding die of 50 mm far from the end portion in the advancing direction of the endless belt, 50 g of a heat generating composition is then placed in the vicinity of the leveling plate between the leveling plate and the cavity, and the heat generating composition is filled in the cavity of the molding die while leveling it by moving the endless belt at 1.8 m/min. After the molding die has completely passed through the leveling plate, the traveling of the endless belt is stopped. Next, the molding die is removed, and a heat generating composition molded body as laminated on the polyethylene film is observed.

3) Judgment method:

With respect to the judgment method, in the surroundings of the heat generating composition molded body, in the case where any collapsed piece of the heat generating composition molded body exceeding a maximum length of 800 μm is not present and the number of collapsed pieces of the heat generating composition molded body having a maximum length of from 300 to 800 μm is not more than 5, it is to be noted that the heat generating composition has moldability.

The moldability is an essential property for a heat generating composition to be used in the molding system. If the heat generating composition does not have moldability, it is impossible to produce a heat generating body by the molding system.

The heat generating composition is not limited so far as it is a heat generating composition which contains, as essential components, an iron powder, a carbon component, a reaction accelerator and water, does not contain a flocculant aid, a flocculant, an agglomeration aid, a dry binding material, a dry binding agent, a dry binder, a sticky raw material, a thickener and an excipient, contains surplus water so as to have a water mobility value of from 0.01 to 20, has mobility due to the surplus water, with the water in the heat generating composition not functioning as a barrier layer, and is capable of causing an exothermic reaction upon contact with air.

Furthermore, in the heat generating composition of the invention or the like, although there is no particular limitation for the compounding ratio thereof, it is preferred to select the compounding ratio such that the amount of the reaction accelerator is from 1.0 to 50 parts by weight, the amount of water is from 1.0 to 60 parts by weight, the amount of the carbon component is from 1.0 to 50 parts by weight, the amount of the water retaining agent is from 0.01 to 10 parts by weight, the water absorptive polymer is from 0.01 to 20 parts by weight, the amount of the pH adjusting agent is from 0.01 to 5 parts by weight, and the amount of the hydrogen formation inhibitor is from 0.01 to 12 parts by weight, respectively based on 100 parts by weight of the iron powder; and that the heat generating composition has a water mobility value of from 0.01 to 20.

In addition, the following components may be added in compounding ratios as described below to the iron powder to the heat generating composition. That is, the amount of the metal other than iron is from 1.0 to 50 parts by weight, the amount of the metal oxide other than iron oxide is from 1.0 to 50 parts by weight, the amount of the surfactant is from 0.01 to 5 parts by weight, the amount of each of the hydrophobic polymer compound, the aggregate, the fibrous material, the functional substance, the organosilicon compound and the pyroelectric substance is from 0.01 to 10 parts by weight, the amount of each of the moisturizer, the fertilizer component and the heat generating aid is from 0.01 to 10 parts by weight, and the amount of the acidic substance is from 0.01 to 1 part by weight based on 100 parts by weight of the iron powder. Incidentally, a magnetic material may further be compounded, and its compounding ratio may be properly determined depending upon the desire.

Incidentally, these compounding ratios can also be applied in a reaction mixture and a heat generating mixture. Furthermore, a water mobility value of the reaction mixture is usually less than 0.01.

As the water, one from a proper source may be employed.

Its purity and kind and the like are not particularly limited.

In the case of the heat generating composition, the content of water is preferably from 1 to 70 % by weight, more preferably from 1 to 60 % by weight, further preferably from 7 to 60 % by weight, still further preferably from 10 to 50 % by weight, and even further preferably from 20 to 50 % by weight of the heat generating composition.

Furthermore, in the case of the reaction mixture or heat generating mixture prior to the contact treatment with an oxidizing gas, the content of water is preferably from 0.5 to 20 % by weight, more preferably from 3 to 20 % by weight, further preferably from 4 to 20 % by weight, and still further preferably from 4 to 15 % by weight of the reaction mixture or heat generating mixture.

The carbon component is not particularly limited so far as it contains carbon as a component. Examples thereof include carbon black, graphite, active carbon, carbon nanotubes, carbon nanohorns, and flullerenes. Carbon which has become conductive by doping or the like is also employable. There are enumerated active carbons as prepared from coconut shell, wood, charcoal, coal, bone carbon, etc. and carbons as prepared from other raw materials such as animal products, natural gases, fats, oils, and resins. In particular, active carbons having an adsorption retaining ability are preferable.

Furthermore, it is not always required that the carbon component is present alone. In the case where an iron powder containing the carbon component and/or covered by the carbon component is used in the heat generating composition, it is to be noted that the heat generating composition contains the carbon component even though the carbon component is not present alone.

The reaction accelerator is not particularly limited so far as it is able to promote the reaction of the heat generating substance. Examples thereof include metal halides, nitrates, acetates, carbonates, and metal sulfates. Examples of metal halides include sodium chloride, potassium chloride, magnetic chloride, calcium chloride, ferrous chloride, ferric chloride, sodium bromide, potassium bromide, ferrous bromide, ferric bromide, sodium iodide, and potassium iodide. Examples of nitrates include sodium nitrate and potassium nitrate. Examples of acetates include sodium acetate. Examples of carbonates include ferrous carbonate. Examples of metal sulfates include potassium sulfate, sodium sulfate, and ferrous sulfate.

The water retaining agent is not limited so far as it is able to retain water. Examples thereof include porous materials derived from plants having high capillary function and hydrophilicity such as wood meal, pulp powder, active carbon, saw dust, cotton cloth having a number of cotton fluffs, short fiber of cotton, paper dust, and vegetable materials, water-containing magnesium silicate based clay minerals such as active clay and zeolite, pearlite, vermiculite, silica based porous substances, coralline stone, and volcanic ash based substances (for example, terraballoon, *shirasu* balloon, and *taisetsu* balloon). In order to increase a water retaining ability and enhance a shape holding ability of such a water retaining agent, the water retaining agent may be subjected to a processing treatment such as baking and/or pulverization.

The water absorptive polymer is not particularly limited so far as it is a resin having a crosslinking structure and having a water absorption magnification of ion-exchanged water of 3 times or more of the dead weight. Furthermore, a water absorptive polymer the surface of which is crosslinked may be employed. Conventionally known water absorptive polymers and commercial products may also be employed.

Examples of the water absorptive polymer include poly(meth)acrylic acid crosslinked materials, poly(meth)-acrylic acid salt crosslinked materials, sulfonic group-containing poly(meth)acrylic ester crosslinked materials, polyoxyalkylene group-containing poly(meth)acrylic ester crosslinked materials, poly(meth)acrylamide crosslinked materials, crosslinked materials of a copolymer of a (meth)acrylic acid salt and a (meth)acrylamide, crosslinked materials of a copolymer of a hydroxyalkyl (meth)acrylate and a (meth)acrylic acid salt, polydioxolane crosslinked materials, crosslinked polyethylene oxide, crosslinked polyvinylpyrrolidone, sulfonated polystyrene crosslinked materials, crosslinked polyvinylpyridine, saponification products of a starch-poly (meth) acrylonitrile graft copolymer, starch-poly(meth)acrylic acid (salt) graft crosslinked copolymers, reaction products of polyvinyl alcohol and maleic anhydride (salt), crosslinked polyvinyl alcohol sulfonic acid salts, polyvinyl alcohol-acrylic acid graft copolymers, and polyisobutylene maleic acid (salt) crosslinked polymers. These water absorptive polymers may be used alone or in combination with two or more kinds thereof.

Of these water absorptive polymers, water absorptive polymers having biodegradation properties are not limited so far as they are a biodegradable water absorptive polymer. Examples thereof include polyethylene oxide crosslinked materials, polyvinyl alcohol crosslinked materials, carboxymethyl cellulose crosslinked materials, alginic acid crosslinked materials, starch crosslinked materials, polyamino acid crosslinked materials, and polylactic acid crosslinked materials.

The pH adjusting agent is not limited so far it is able to adjust the pH. Examples thereof include alkali metal weak acid salts and hydroxides and alkaline earth metal weak acid salts and hydroxides such as $Na_2CO_3$, $NaHCO_3$, $Na_3PO_4$, $Na_2HPO_4$, $Na_5P_3O_{10}$, NaOH, KOH, $Ca(OH)_2$, $Mg(OH)_2$, and $Ca_3(PO_4)_2$.

The hydrogen formation inhibitor is not limited so far as it is able to inhibit the formation of hydrogen. Examples thereof include one member or two or more members selected from the group consisting of sulfur compounds, oxidizing agents, alkaline substances, sulfur, antimony, selenium, phosphorus, and tellurium. Incidentally, examples of sulfur compounds include compounds with an alkali metal or an alkaline earth metal, metal sulfides such as calcium sulfide, metal sulfites such as sodium sulfite, and metal thiosulfates such as sodium thiosulfate.

Examples of the oxidizing agent include nitrates, oxides, peroxides, halogenated oxygen acid salts, permanganates, and chromates.

The aggregate is not limited so far as it is useful as a filler and/or is useful for making the heat generating composition porous. Examples thereof include fossilized coral (for example, coral fossil and weathered coral fossil), bamboo charcoal, *bincho* charcoal, silica-alumina powders, silica-magnesia powders, kaolin, crystalline cellulose, colloidal silica, pumice, silica gel, silica powders, mica powders, clays, talc, synthetic resin powders or pellets, foamed synthetic resins such as foamed polyesters or polyurethanes, diatomaceous earth, alumina, and cellulose powder. Incidentally, it is to be noted that kaolin and crystalline cellulose are not contained in the heat generating composition of the invention.

The fibrous material is an inorganic fibrous material and/or an organic fibrous material. Examples thereof include rock wool, glass fibers, carbon fibers, metal fibers, pulps, papers, non-woven fabrics, woven fabrics, natural fibers such as cotton and hemp, regenerated fibers such as rayon, semi-synthetic fibers such as acetates, synthetic fibers, and pulverized products thereof.

The functional substance is not limited so far as it is a substance having any function. Examples thereof include at least one member selected from minus ion emitting substances and far infrared ray radiating substances. The minus ion emitting substance is not limited so far as it emits a minus ion as a result either directly or indirectly, and examples thereof include ferroelectric substances such as tourmaline, fossilized coral, granite, and calcium strontium propionate, and ores containing a radioactive substance such as radium and radon. The far infrared ray radiating substance is not limited so far as it radiates far infrared rays. Examples thereof include ceramics, alumina, zeolite, zirconium, and silica.

The surfactant includes anionic surfactants, cationic surfactants, nonionic surfactants, and ampholytic surfactants. Especially, nonionic surfactants are preferable, and examples thereof include polyoxyethylene alkyl ethers, alkylphenolethylene oxide adducts, and higher alcohol phosphoric acid esters.

The organosilicon compound is not limited so far as it is a compound having at least an Si-O-R bond and/or an Si-N-R bond and/or an Si-R bond. The organosilicon compound is in the form of a monomer, a lowly condensed product, a polymer, etc. Examples thereof include organosilane compounds such as methyltriethoxysilane; and dimethylsilicone oil, polyorganosiloxane, or silicone resin compositions containing the same.

The pyroelectric substance is not limited so far as it has pyroelectricity. Examples thereof include tourmaline, hemimorphic ores, and pyroelectric ores. Tourmaline or achroite which is a kind of tourmaline is especially preferable. Examples of the tourmaline include dravite, schorl, and elbaite.

The moisturizer is not limited so far as it is able to hold moisture. Examples thereof include hyaluronic acid, collagen, glycerin, and urea.

The fertilizer component is not limited so far as it is a component containing at least one of three elements of nitrogen, phosphorus and potassium. Examples thereof include a bone powder, urea, ammonium sulfate, calcium perphosphate, potassium chloride, and calcium sulfate.

The hydrophobic polymer compound is not limited so far as it is a polymer compound having a contact angle with water of 40° or more, preferably 50° or more, and more preferably 60° or more in order to improve the draining in the composition. The shape of the hydrophobic polymer compound is not limited, and examples thereof include powdery, particulate, granular, and tablet shapes. Examples of the hydrophobic polymer compound include polyolefins such as polyethylene and polypropylene, polyesters, and polyamides.

Examples of the heat generating aid include metal powders, metal salts, and metal oxides such as Cu, Mn, $CuCl_2$, $FeCl_2$, manganese dioxide, cupric oxide, triiron tetroxide, and mixtures thereof.

As the metal oxide other than iron oxide, any material can be employed so far as it does not hinder the oxidation of iron by an oxidizing gas, and examples thereof include manganese dioxide and cupric oxide.

The acidic substance may be any of an inorganic acid, an organic acid, or an acidic salt. Examples thereof include hydrochloric acid, sulfuric acid, nitric acid, acetic acid, oxalic acid, citric acid, malic acid, maleic acid, chloroacetic acid, iron chloride, iron sulfate, iron oxalate, iron citrate, aluminum chloride, ammonium chloride, and hypochlorous acid.

As the "iron powder" as referred to herein, usual iron powders, iron alloy powders and active iron powders such as iron powders comprising particles, a surface of each of which is at least partially covered with an oxygen-containing film , and iron alloy powders comprising particles , a surface of each of which is at least partially covered with an oxygen-containing film, are preferable. Incidentally, the "iron oxide film" as referred to herein is a film made of oxygen-containing iron such as iron oxide, hydroxide or oxyhydroxide. Furthermore, the "active iron powder" as referred to herein is a powder in which an iron oxide film is formed at least locally on the surface of an iron powder, from which an oxidation reaction promoting effect is obtained by a local cell as formed between an iron matrix and an iron oxide film or a pit inside and outside the iron oxide film.

The iron powder is not limited, and examples thereof include cast iron powders, atomized iron powders, electrolyzed iron powders, reduced iron powders, sponge iron powders, and iron alloy powders thereof. In addition, the iron powder may contain carbon or oxygen, and an iron powder containing 50 % or more of iron and other metals may be employed. The kind of the metal which is contained as an alloy, etc. is not particularly limited so far as the iron component works as a component of the heat generating composition. Examples of such a metal include metals such as aluminum, manganese, copper, nickel, silicon, cobalt, palladium, and molybdenum, and semiconductors. The metal of the invention includes a semiconductor. Such a metal or alloy may be contained only in the surface or the interior, or may be contained in both the surface and the interior.

In the iron powder of the invention, the content of the metal other than iron is usually from 0.01 to 50 % by weight, and preferably from 0.1 to 10 % by weight based on the whole of the iron powder.

Examples of the iron powder having an oxygen-containing film on at least a part of the surface of the iron include:

(A) an active iron powder in which the surface of an iron component is at least partially oxidized, which is obtained by contact treating the essential components of the heat generating composition or the essential components to which acidic substances or other necessary components are added with an oxidizing gas, thereby partially oxidizing the iron component;

(B) an active iron powder in which the content of wustite is from 2 to 50 % by weight in terms of an X-ray peak intensity ratio to iron;

(C) an iron powder having an iron oxide film having a thickness of 3 nm or more on the surface thereof; and

(D) a mixture of an active iron powder and an iron powder other than an active iron powder.

With respect to (A), although the mechanism is not elucidated in detail, it is assumed that upon contact between the oxidizing gas and the components, not only an iron oxide film, namely, an oxygen-containing film is formed on the surface of the iron powder due to the oxidation of the components, especially the oxidation of the iron powder, but also the surface of active carbon is oxidized and/or the oxidized iron component is adhered, whereby hydrophilicity is imparted or improved, and coupling between the components or structurization takes place through the mediation of water.

That is, it is assumed that some kind of a change in the function occurs such that an iron oxide film is formed on the surface of the iron powder, the shape of the iron powder particle becomes irregular, a strain is generated due to the oxidation, or a water-containing pit is formed, whereby the iron powder is activated and exothermic rising properties are improved.

Furthermore, the case where magnetite ($Fe_3O_4$) is present in the iron oxide film is preferable because the conductivity is excellent, and the case where hematite ($Fe_2O_3$) is present in the iron oxide film is also preferable because the iron oxide film becomes porous. Moreover, it is assumed that the carbon component is oxidized on the surface thereof and becomes a carbon component which is rich in oxides on the surface thereof, whereby the hydrophilicity increases and the activity increases.

The thickness of the iron oxide film which is an oxygen-containing film covering the surface of the iron powder, as measured by the Auger electron spectroscopy, is usually 3 nm or more, preferably from 3 nm to 100 $\mu$m, more preferably from 30 nm to 100 $\mu$m, further preferably from 30 nm to 50 $\mu$m, still further preferably from 30 nm to 1 $\mu$m, even further preferably from 30 nm to 500 nm, and even still further preferably from 50 nm to 300 nm.

When the thickness of the oxygen-containing film of iron is 3 nm or more, the thickness of the oxygen-containing film of iron is able to exhibit a promoting effect of the oxidation reaction, and upon contact with an oxidizing gas such as air, is able to immediately initiate the oxidation reaction. When the thickness of the oxygen-containing film of iron is 100 $\mu$m or more, though the heat generation time may possibly be shortened, such is applicable depending upon the utility.

Furthermore, according to the active iron powder, by using a reaction mixture containing, as essential components, an iron powder, a reaction accelerator and water and having a water content of from 0. 5 to 20 % by weight and a water mobility value showing a surplus water content of less than 0.01, the reaction rate at the time of the contact treatment with an oxidizing gas can be raised, thereby achieving a time required for regulating a temperature rise of the reaction mixture at 1 °C or more within 10 minutes. By shortening a time required for arrival at a prescribed temperature or higher, proper activation can be achieved, and unnecessary oxidation on the iron powder can be prevented.

Furthermore, the heat generating composition prepared by adding a carbon component, etc. to a heat generating mixture as produced by contact treating the reaction mixture with an oxidizing gas or adjusting the water content so as to have a water mobility value of from 0.01 to 50 is properly tacky, has excellent moldability and is able to be applied with a molding method such as a force-through die molding method and a cast molding method, whereby heat generating bodies of various shapes can be produced. In particular, a heat generating composition having a water mobility value of from 0.01 to 20 is excellent because it initiates an exothermic reaction immediately after contacting with air, has excellent exothermic rising properties and has excellent moldability.

The contact treatment method of the reaction mixture with an oxidizing gas is not particularly limited so far as it is able to contact treat a reaction mixture containing, as essential components, an iron powder, a reaction accelerator and water and having a water content of from 0.5 to 20 % by weight and a water mobility value of less than 0.01 with an oxidizing gas and regulate a temperature rise of the reaction mixture at 1 °C or more.

Specific examples thereof include:

(1) a process for producing a heat generating mixture containing an iron powder having an iron oxide film on the surface thereof by subjecting a reaction mixture of an iron powder, a reaction accelerator and water in an oxidizing gas atmosphere to a self-exothermic reaction, thereby partially oxidizing the iron powder;

(2) a process for producing a heat generating mixture by subjecting a reaction mixture of an iron powder, a reaction accelerator, an acidic substance and water in an oxidizing gas atmosphere to a self-exothermic reaction;

(3) a process for producing a heat generating mixture by subjecting a reaction mixture of an iron powder, a reaction accelerator, a carbon component and water in an oxidizing gas atmosphere to a self-exothermic reaction;

(4) a process for producing a heat generating mixture by subjecting a reaction mixture of an iron powder, a reaction accelerator, an acidic substance, a carbon component and water in an oxidizing gas atmosphere to a self-exothermic reaction;

(5) a process for producing a heat generating mixture containing a partially oxidized iron powder by carrying out the method as set forth above in any one of (1) to (4), wherein the reaction mixture or heat generating mixture as set forth above in any one of (1) to (4) contains a component other than the foregoing components;

(6) a process for producing a heat generating mixture by carrying out the method as set forth above in any one of (1) to (5) under circumstances heated so as to have temperature of at least 10 °C higher than the circumferential temperature;

(7) a process for producing a heat generating mixture by carrying out the method as set forth above in any one of (1) to (6) by blowing an oxidizing gas;

(8) a process for producing a heat generating mixture by carrying out the method as set forth above in (7) by blowing the oxidizing gas heated so as to have a temperature of at least 10 °C higher than the circumferential temperature;

(9) a process for producing a heat generating composition by carrying out the method as set forth above in any one of (1) to (8) by contact treating with an oxidizing gas until the temperature exceeds a maximum temperature which is a maximum point of temperature rise by the exothermic reaction;

(10) a process for producing a heat generating mixture by carrying out the method as set forth above in any one of (1) to (8) by contact treating with an oxidizing gas until the temperature exceeds a maximum temperature by the exothermic reaction and drops by at least 10 to 20 °C from the maximum temperature;

(11) a process for producing a heat generating composition by carrying out the method as set forth above in any one of (1) to (8) by contact treating with an oxidizing gas until the temperature exceeds a maximum temperature which is a maximum point of temperature rise by the exothermic reaction and after intercepting the oxidizing gas, holding it until the temperature of at least the reaction mixture drops by at least 10 to 20 °C from the maximum temperature; and

(12) a process for producing a heat generating mixture by heating the reaction mixture or heat generating mixture as set forth above in any one of (1) to (5) under oxidizing gas circumstances while regulating a temperature rise at 1 °C or more.

In addition, a heat generating mixture as prepared by adding other components to the heat generating mixture and further treating with an oxidizing gas may be employed.

Incidentally, the circumstances of the reaction mixture at the time of contact treatment with an oxidizing gas are not limited so far as the reaction mixture is brought into contact with an oxidizing gas under circumstances of 0 °C or higher and a temperature rise of the reaction mixture is regulated at 1 °C or more within 10 minutes. In the case where the contact treatment is carried out in an open system, the circumstances may be either the state that the reaction mixture is present in a lid-free vessel or the state that an oxidizing gas such as air comes into a vessel through an air-permeable sheet-like material such as non-woven fabrics.

Furthermore, the contact treatment with an oxidizing gas may be carried out with or without stirring in a fluidized or non-fluidized state and may be carried out in a batch or continuous system.

Examples of the final heat generating composition include:

1) a heat generating composition containing, as a heat generating composition raw material, a heat generating mixture produced in the process as set forth above in any one of (1) to (12);

2) a heat generating composition obtained by adding other components to the heat generating composition as set forth above in 1); and

3) a heat generating composition obtained by adjusting the water content of the heat generating composition as set forth above in 1) or 2).

The order of the timing of adding other components than the essential components and the timing of adjusting the water content is not limited.

Here, the water content in the reaction mixture and also the heat generating mixture prior to the treatment with an oxidizing gas is usually from 0. 5 to 20 % by weight, preferably from 1 to 15 % by weight, more preferably from 2 to 10 % by weight, further preferably from 3 to 10 % by weight, and still further preferably from 6 to 10 % by weight.

The temperature of the reaction mixture after the contact with an oxidizing gas is not limited so far as the temperature rise is regulated at 1 °C or more. The temperature of the reaction mixture after the contact with an oxidizing gas is preferably from 1 to 80 °C, more preferably from 1 to 70 °C, further preferably from 1 to 60 °C, and still further preferably from 1 to 40 °C.

The circumferential temperature at the time of contact between the reaction mixture and the oxidizing gas is not limited so far as the temperature of the reaction mixture is raised to a prescribed temperature or higher. The circumferential temperature at the time of contact between the reaction mixture and the oxidizing gas is preferably 0 °C or higher, more preferably from 0 to 250 °C, further preferably from 10 to 200 °C, still further preferably from 20 to 150 °C, even further preferably from 25 to 100 °C, and even still further preferably from 25 to 50 °C.

The time of contact between the reaction mixture and the oxidizing gas is not limited so far as the time required for regulating a temperature rise at 1 °C or more is within 10 minutes. The time of contact between the reaction mixture and the oxidizing gas is preferably from one second to 10 minutes, more preferably from one second to 7 minutes, further preferably from one second to 5 minutes, still further preferably from 2 seconds to 5 minutes, even further preferably from 2 seconds to 3 minutes, and even still further preferably from 2 seconds to one minute.

The temperature of the oxidizing gas is not limited so far as the foregoing circumferential temperature is kept.

As the "oxidizing gas" as referred to herein, any gas can be used as the oxidizing gas so far as it is oxidizing. Examples thereof include an oxygen gas, air, and mixed gases of an inert gas (for example, a nitrogen gas, an argon gas, and a helium gas) and an oxygen gas. Although the mixed gas is not limited so far as it contains oxygen, mixed gases containing 10 % or more of an oxygen gas are preferable, and of these, air is especially preferable. If desired, a catalyst such as platinum, palladium, iridium, and compounds thereof can also be used.

The oxidation reaction can be carried out under stirring in an oxidizing gas atmosphere optionally under a pressure and/or upon irradiation of ultrasonic waves.

The optimal condition of the oxidation reaction may be properly experimentally determined.

An amount of the oxidizing gas to be used is not limited but may be adjusted depending upon the kind of the oxidizing gas, the kind and particle size of the iron powder, the water content, the treatment temperature, the treatment method, and the like.

In the case of an open system, there is no limitation so far as a necessary amount of oxygen can be taken in.

In order to prevent fly of the reaction mixture or contamination of dusts, etc., the system may be surrounded by an air-permeable raw material such as non-woven fabrics and woven fabrics. So far as the system is in an air-permeable state, it is to be noted that the system is an open system.

In the case where air is used in the system of blowing an oxidizing gas, for example, the amount of air is preferably from 0.01 to 1,000 L/min, more preferably from 0.01 to 100 L/min, and further preferably from 0.1 to 50 L/min per 200 g of the iron powder under one atmosphere. In the case of other oxidizing gas, the amount of the oxidizing gas may be converted on the basis of the case of air.

If desired, a peroxide may be added. Examples of the peroxide include hydrogen peroxide and ozone.

Here, so far as the iron powder is partially oxidized, the state of the reaction mixture or heat generating mixture at the time of the contact treatment with an oxidizing gas may be any of a standing state, a transfer state, or a fluidizing state by stirring, etc. and may be properly selected. Furthermore, the circumstances at the time of mixing the respective components of the reaction mixture, the heat generating mixture or the heat generating composition and at the time of the contact treatment with a mixed oxidizing gas at the time of adjusting the water content are not limited, and examples thereof include those in an oxidizing gas atmosphere and those in blowing of an oxidizing gas.

A method for measuring a temperature rise of the heat generating composition is as follows.

1) A heat generating composition is allowed to stand in a state that it is sealed in an air-impermeable outer bag for one hour under a condition that the circumferential temperature is 20 ± 1 °C.

2) A magnet is provided in the vicinity of a central part of the back side of a polyvinyl chloride-made supporting plate (3 mm in thickness x 600 mm in length x 600 mm in width) of a footed supporting table so as to cover a cavity shape of a molding die.

3) A temperature sensor is placed on the central part of the supporting plate.

4) A polyethylene film (25 $\mu$m in thickness x 250 mm in length x 200 mm in width) as provided with an adhesive layer having a thickness of about 80 $\mu$m is stuck onto the supporting plate via a sticky layer such that the center of the polyethylene film is positioned at the sensor.

5) The heat generating composition is taken out from the outer bag.

6) A template (250 mm in length x 200 mm in width) having a cavity (80 mm in length x 50 mm in width x 3 mm in height) is placed above the central part of the polyethylene film; a sample is placed in the vicinity of the cavity; a force-in die plate is moved along the template; the sample is charged into the cavity while stuffing; and the sample is leveled while stuffing along the template plane (force-in die molding), thereby filling the sample in the die. Next, the magnet beneath the supporting plate is removed, and the temperature measurement is started.

With respect to the measurement of the exothermic temperature, the temperature is measured for 10 minutes at a measurement timing of 2 seconds using a data collector, and exothermic rising properties are judged in terms of the temperature after elapsing 3 minutes.

The heat generation test of the heat generating body follows the JIS temperature characteristic test.

In the iron powder or active iron powder in the oxidizing gas-treated heat generating composition, at least a part of the surface thereof is covered by an oxygen-containing film of iron. The degree of covering on the surface of the oxygen-containing film of iron is not limited so far as at least a part of the surface thereof is covered, and the surface may be entirely covered. In the case of the heat generating composition of the invention, since an ion of the reaction accelerator such as a chlorine ion is contained in the heat generating composition, there is no corrosion effect of the oxide film due to anti-corrosion effect by the ion of the reaction accelerator such as a chlorine ion. Thus, the oxidation reaction which is a sort of corrosion is not hindered. In particular, in the case where an oxygen-containing film of iron is prepared while the ion of the reaction accelerator such as a chlorine ion exists together, the subject effect is large. In the case where a metal other than iron is present on the surface, it is only required that at least other part of the metal portion other than iron is covered by the oxygen-containing film of iron.

In the iron powder of the invention, not only a region where (1) entire (uniform) corrosion, (2) pitting or crevice corrosion, (3) stress corrosion cracking, or the like is generated, but also irregularities or crevices are formed. For that reason, it is assumed that the iron powder of the invention has hydrophilicity and oxidation catalytic properties (FeO, etc.) in its own portion. In producing the heat generating composition, it is important that the iron powder has an oxygen-containing film in its own portion without relying upon mixing. In particular, in the iron component as prepared by contact treating the iron component and the reaction accelerator and water as essential components with an oxidizing gas, it is thought that a reaction active part composed mainly of an oxide, a hydroxide, a chlorine ion, a hydrogen ion, etc. is formed, whereby exothermic reactivity and hydrophilicity are improved and exothermic rising properties and moldability are remarkably improved.

With respect to (B), the amount of FeO (wustite) which is contained in the iron component containing a prescribed amount of wustite is usually from 2 to 50 % by weight, preferably from 2 to 40 % by weight, more preferably from 2 to 30 % by weight, further preferably from 5 to 30 % by weight, and still further preferably from 6 to 30 % by weight in terms of an X-ray peak intensity ratio of iron. When the amount of FeO (wustite) exceeds 50 % by weight, though the exothermic rising properties are good, the duration of heat generation becomes short. On the other hand, when it is less than 2 % by weight, the exothermic rising properties become dull.

The thickness of the oxygen-containing film of a prescribed amount or the oxygen-containing film of iron powder containing wustite and the amount of wustite are applied to the heat generating composition or the heat generating composition molded body at the time of lamination.

An iron powder containing a carbon component and/or covered by a carbon component is also preferable. Although a proportion of the carbon component is not limited so far as a ratio of the iron component to the carbon component is 50 % by weight or more, an iron powder in which the surface thereof is partially covered by from 0.3 to 3.0 % by weight of a conductive carbonaceous substance is useful. Examples of the conductive carbonaceous substance include carbon black, active carbon, carbon nanotubes, carbon nanohorns, and flullerenes. Ones which have become conductive by doping are also employable. Examples of the iron powder include reduced iron powders, atomized iron powders, and sponge iron powders. In particular, the case where the conductive carbonaceous substance is active carbon and the iron powder is a reduced iron powder is useful as a heat generating body.

Furthermore, in order to efficiently carry out covering by a conductive carbonaceous substance, an oil such as a spindle oil may be added in an amount of from 0.01 to 0.05 % by weight to such an extent that the fluidity of the iron powder is not hindered.

In the case of measuring the water mobility value of the heat generating composition in the heat generating body and the thickness and amount of wustite of the iron oxide film of iron powder in the mixture or the heat generating composition in the heat generating body, the heat generating composition or mixture may be measured according to the following items.

 1) Water mobility value:

 The heat generating composition is taken out from the heat generating body and measured according to the foregoing method of measuring a water mobility value.

 2) Thickness and amount of wustite of iron oxide film of iron powder:

 A measuring sample as prepared by dispersing the heat generating composition, the heat generating composition molded body, the heat generating composition compression molded body or the mixture in nitrogen-purged ion-exchanged water in a nitrogen atmosphere, separating the iron powder using a magnet and drying the iron powder in a nitrogen atmosphere is used.

The heat generating composition of the invention contains, as essential components, an iron powder, a carbon component, a reaction accelerator and water, and its production process is one which can be put into practical use on an industrial scale. A reaction mixture containing, as essential components, an iron powder, a reaction accelerator and water and having a water content of from 1 to 20 % by weight and a water mobility value showing a surplus water content of less than 0.01 is brought into contact with an oxidizing gas under circumstances at 0 °C or higher, a temperature rise of the reaction mixture is regulated at 1 °C or more within 10 minutes to produce a heat generating mixture, and the subject heat generating mixture is used as a raw material to form a heat generating composition. Alternatively, a heat generating composition may be formed by subsequently further adjusting the water content, or by further adding a carbon component, etc. and adjusting the water content.

In the invention, it has become possible to realize the contact treatment with an oxidizing gas within a short period of time by regulating the water content of the reaction mixture at a fixed amount or less, especially regulating the surplus water content of the reaction mixture at a fixed amount or less and carrying out an oxidizing contact treatment. By specifying the surplus water content and performing the treatment within a short period of time, adverse influences such as poor initial exothermic rising of the heat generating composition and shortening of the heat generation-retaining time can be avoided. Thus, it has become possible to establish an industrial mass-production process. Furthermore, although stirring or the like may not be achieved during the contact treatment with an oxidizing gas, when stirring or the like is achieved, the contact treatment with an oxidizing gas can be surely carried out.

Here, so far as the iron powder is partially oxidized, the state of the reaction mixture or heat generating mixture at the time of the contact treatment with an oxidizing gas may be any of a standing state, a transfer state, or a fluidizing state by stirring, etc. and may be properly selected. Furthermore, the circumstances at the time of mixing the respective components of the reaction mixture, the heat generating mixture or the heat generating composition and at the time of mixing at the time of adjusting the water content are not limited, and examples thereof include those in an oxidizing gas atmosphere and those in blowing of an oxidizing gas.

The "adjustment of the water content" as referred to herein means that after contact treating the heat generating mixture with an oxidizing gas, water or an aqueous solution of a reaction accelerator is added. Although the amount of addition of water or an aqueous solution of a reaction accelerator is not limited, examples thereof include the addition of a weight corresponding to a reduced weight by the contact treatment and the addition of a weight such that a desired water mobility value is obtained.

Whether or nor the adjustment of the water content is introduced may be properly determined depending upon the utility.

The heat generating composition of the invention contains, as essential components, an iron powder, a carbon component, a reaction accelerator and water and is started from a mixture obtained by contact treating a reaction mixture containing, as essential components, an iron powder, a reaction accelerator and water with an oxidizing gas. The heat generating composition of the invention is usually one obtained by adjusting the water content of a heat generating mixture and is a heat generating composition which is satisfactory in the exothermic rising, has a suitable amount of surplus water and has excellent moldability. Furthermore, it is possible to produce a heat generating body which can become promptly warm at the time of use.

Accordingly, at least the iron powder further including the carbon component has a history of oxidation by the contact treatment with an oxidizing gas, and it is thought that this is deeply related to excellent exothermic rising properties, exothermic endurance and excellent moldability.

When the iron powder which is contact treated with an oxidizing gas according to the invention is used, the amount of addition of the carbon component (for example, active carbon) in the heat generating composition can be reduced by, for example, 20 % or more. By reducing the amount of addition of the carbon component, the costs are lowered.

According to the production process of the heat generating mixture of the invention, it is possible to obtain a heat generating composition having excellent exothermic rising properties, excellent hydrophilicity, and excellent moldability. In particular, a heat generating composition having remarkably excellent moldability and exothermic characteristics together can be obtained while specifying the water availability value at from 0.01 to 50.

The heat generating composition as produced by the production process of the invention is remarkably improved with respect to exothermic rising properties. Thus, the amount of addition of the carbon component (such as active carbon) in the heat generating composition can be reduced by, for example, 20 % or more so that it can contribute to a reduction in costs.

Furthermore, since the hydrophilicity is remarkably improved, the moldability with a mold is remarkably improved. Thus, since after molding, collapsed pieces of the heat generating composition are not scattered on the surroundings of the heat generating composition molded body, sealing can be appropriately achieved so that a heat generating body free from sealing cut can be produced. In this way, heat generating composition molded bodies of various shapes can be produced, and heat generating bodies of various shapes are formed.

Furthermore, in view of improving the exothermic rising properties of the heat generating composition, the following are preferable.

1) A heat generating composition obtained by a contact treatment (self heat generation) of a mixture of the essential components of the heat generating composition, or a mixture of the foregoing mixture and an acidic substance or other necessary components with an oxidizing gas, a heat generating composition obtained by additionally adjusting the water content of the foregoing heat generating composition, or a heat generating composition obtained by adding and mixing other components in the foregoing heat generating composition.

2) Any one of the following active iron powders having an oxygen-containing film (for example, oxides) on at least a part of the surface thereof is used as the iron powder: (a) an iron powder having an oxygen-containing film of iron having a thickness, as measured by the Auger electron spectroscopy, of 3 nm or more on the surface thereof and (b) an iron powder having a content of wustite of from 2 to 50 % by weight in terms of an X-ray peak intensity ratio to iron.

3) A mixture of an active iron powder having an oxygen-containing film (for example, oxides) on at least a part of the surface thereof and an iron powder not having an oxygen-containing film is used as the iron powder. In this case, a mixture containing 60 % by weight or more of an active iron powder and less than 40 % by weight of an iron powder other than the active iron is preferable.

In the case of storing the heat generating composition which is treated with an oxidizing gas or the heat generating composition containing an active iron powder, or a material utilizing the same over a long period of time, it is preferred to combine a hydrogen formation inhibitor therewith. This is because in this way, a heat generating body having excellent exothermic characteristics, which is inhibited in the formation of hydrogen, is free from swelling of the outer bag at the time of storage, etc. and has satisfactory exothermic rising properties, is obtained.

Furthermore, so far as the rising characteristics are not affected, the heat generating composition having a water mobility value falling outside the range of from 0.01 to 20 can contain a water-soluble polymer, a flocculant aid, a flocculant, an agglomeration aid, a dry binding material, a dry binding agent, a dry binder, an adhesive raw material, a tackifier, an excipient, a flocculating agent, or a soluble sticky raw material.

Furthermore, since a marketed heat generating body in which a heat generating composition is accommodated in an accommodating bag is provided on the assumption that it is accommodated in an outer bag which is an air-impermeable accommodating bag and is storable over a long period of time, it is preferred to use a heat generating composition containing a hydrogen formation inhibitor. Since the heat generating composition which has passed through the contact treatment with an oxidizing gas is an active composition, it is important that the heat generating composition contains a hydrogen formation inhibitor. Also, this efficacy is further strengthened by using a pH adjusting agent together.

Furthermore, so far as the reaction characteristics and exothermic characteristics are not affected, the heat generating composition having a water mobility value of less than 0.01 may contain a flocculant aid, a flocculant, an agglomeration aid, a dry binder, a dry binding agent, a dry binding material, a sticky raw material, a thickener, an excipient, or a water-soluble polymer in an amount ranging from 0.01 to 3 parts by weight respectively.

The "flocculant aid" as referred to herein is a flocculant aid as described in Japanese Patent No. 3,161,605 (JP-T-11-508314) such as gelatin, natural gum, and corn syrup.

The "flocculant" as referred to herein is a flocculant as described in JP-T-2002-514104 such as corn syrup and maltitol syrup.

The "agglomeration aid" as referred to herein is an agglomeration aid as described in JP-T-2001-507593 such as corn syrup.

The "dry binder" as referred to herein is a dry binder as described in JP-T-2002-514104 such as microcrystalline cellulose, maltodextrin, and mixtures thereof.

The "dry binding agent" as referred to herein is a dry binding agent as described in JP-T-2001-507593 such as maltodextrin and sprayed lactose.

The "dry binding material" as referred to herein is a dry binding material as described in JP-T-11-508314 such as microcrystalline cellulose, maltodextrin, and mixtures thereof.

The "sticky raw material" or the "binder" as referred to herein is a sticky raw material or binder as described in JP-A-4-293989 such as water glass, polyvinyl alcohol (PVA), and carboxymethyl cellulose (CMC).

The "thickener" as referred to herein is a thickener as described in JP-A-6-343658 such as corn starch and potato starch.

The "excipient" as referred to herein is an excipient as described in JP-A-7-194641 such as $\alpha$-starch and sodium alginate.

As the "water-soluble polymer" as referred to herein, the water-soluble polymer in the adhesive layer can be used.

The particle size of the water-insoluble solid component constituting the moldable heat generating composition of the invention is not limited so far as the heat generating composition has moldability. In the case where any one of length, width and height as the size of the heat generating composition molded body as molded from the heat generating composition is small, the moldability is improved by making the particle size small.

In addition, it is preferable in view of molding that the particle size of the solid component constituting the moldable heat generating composition is small. A maximum particle size of the water-insoluble solid component exclusive of the reaction accelerator and water in the components constituting the moldable heat generating composition is preferably not more than 2.5 mm, more preferably not more than 930 $\mu$m, further preferably not more than 500 $\mu$m, still further preferably not more than 300 $\mu$m, even further preferably not more than 250 $\mu$m, and even still further preferably not more than 200 $\mu$m. Moreover, 80 % or more of the particle size of the solid component is usually not more than 500 $\mu$m, preferably not more than 300 $\mu$m, more preferably not more than 250 $\mu$m, further preferably not more than 200 $\mu$m, still further preferably not more than 150 $\mu$m, and even further preferably not more than 100 $\mu$m.

Incidentally, with respect to the particle size of the water-insoluble solid component, separation is conducted using a sieve, and the particle size of the component which has passed through the sieve is calculated from an opening of the sieve. That is, sieves of 8, 12, 20, 32, 42, 60, 80, 100, 115, 150, 200, 250 and 280 meshes and a receiving dish are combined in this order from up to down. About 50 g of water-insoluble solid component particles are placed on the uppermost 8-mesh sieve and shaken for one minute using an automatic shaker. Weights of the water-insoluble solid component particles on each of the sieves and the receiving dish are weighed. The total amount thereof is defined as 100 %, and the particle size distribution is determined from weight fractions. When the sum of all receiving dishes under the sieve of a specific mesh size becomes 100 % which is the total sum of the particle size distribution, the size ($\mu$m) calculated from the opening of the specific mesh is defined as the particle size of the water-insoluble solid component.

Incidentally, each of the mesh sieves may be combined with other mesh sieves. Here, the particles which have passed through a 16-mesh sieve are defined to have a particle size of not more than 1 mm; the particles which have passed through a 20-mesh sieve are defined to have a particle size of not more than 850 $\mu$m; the particles which have passed through a 48-mesh sieve are defined to have a particle size of not more than 300 $\mu$m; the particles which have passed through a 60-mesh sieve are defined to have a particle size of not more than 250 $\mu$m; the particles which have passed through a 65-mesh sieve are defined to have a particle size of not more than 200 $\mu$m; the particles which have passed through an 80-mesh sieve are defined to have a particle size of not more than 180 $\mu$m; the particles which have passed through a 100-mesh sieve are defined to have a particle size of not more than 150 $\mu$m; the particles which have passed through a 115-mesh sieve are defined to have a particle size of not more than 120 $\mu$m; the particles which have passed through a 150-mesh sieve are defined to have a particle size of not more than 100 $\mu$m; and the particles which have passed through a 250-mesh sieve are defined to have a particle size of not more 63 $\mu$m, respectively. The same is applicable to mesh sizes of less than these mesh sizes.

Furthermore, the heat generating composition can be classified into a powder, a granulate heat generating composition (having a water mobility value of less than 0.01), a moldable heat generating composition (having a water mobility value of from 0.01 to 20), and a sherbet-like heat generating composition (having a water mobility value exceeding 20 but not more than 50) depending upon the state of adjustment of the water content or surplus water. The heat generating composition as classified depending upon the water mobility value is as described previously.

The "moldability" as referred to in the invention exhibits that a laminate of the heat generating composition having a cavity or concave die shape can be formed by force-through molding using a trimming die having a cavity or cast molding using a concave die and after molding including mold release, the molding shape of the heat generating composition molded body is held. When the moldability is revealed, since the shape is held until the heat generating composition molded article is at least covered by a covering material and a seal part is formed between the substrate and the covering material, sealing can be achieved in the periphery of the shape with a desired shape. Also, since so-called "spots" which are a collapsed piece of the heat generating composition are not scattered in the seal part, sealing can be achieved without causing cutting in seal. The presence of the spots causes insufficient sealing.

Next, with respect to the moldability, a measurement device, a measurement method and a judgment method will be described below.

1) Measurement device:

With respect to the measurement device, a stainless steel-made molding die (a plate having a size of 2 mm in thickness x 200 mm in length x 200 mm in width and having a cavity as treated by R5 in four corners of 60 mm in length x 40 mm in width in a central part thereof) and a fixable leveling plate are disposed above a travelable endless belt, and magnets (two magnets having a size of 12.5 mm in thickness x 24 mm in length x 24 mm in width are disposed in parallel) are disposed under the endless belt. The magnets should cover a region of the leveling plate and the vicinity thereof and a region larger than a region covered by a cut side (40 mm) vertical to the advancing direction of the cavity of the molding die.

2) Measurement method:

With respect to the measurement method, a stainless steel plate having a size of 1 mm in thickness x 200 mm in length x 200 mm in width is placed on the endless belt of the measurement device, a polyethylene film having a size of 70 $\mu$m in thickness x 200 mm in length x 200 mm in width is placed thereon, and a stainless steel-made molding die is further placed thereon. Thereafter, a leveling plate is fixed in a position of the cavity of the molding die of 50 mm far from the end portion in the advancing direction of the endless belt, 50 g of a heat generating composition is then placed in the vicinity of the leveling plate between the leveling plate and the cavity, and the heat generating composition is filled in the cavity of the molding die while leveling it by moving the endless belt at 1.8 m/min.

After the molding die has completely passed through the leveling plate, the traveling of the endless belt is stopped. Next, the molding die is removed, and a heat generating composition molded body as laminated on the polyethylene film is observed.

3) Judgment method:

With respect to the judgment method, in the surroundings of the heat generating composition molded body, in the case where any collapsed piece of the heat generating composition molded body exceeding a maximum length of 800 $\mu$m is not present and the number of collapsed pieces of the heat generating composition molded body having a maximum length of from 300 to 800 $\mu$m is not more than 5, it is to be noted that the heat generating composition has moldability. The moldability is an essential property for a heat generating composition to be used in the molding system. If the heat generating composition does not have moldability, it is impossible to produce a heat generating body by the molding system.

The heat generating composition of the invention has resistance to compression. The "resistance to compression" as referred to herein means that a heat generating composition compressed body obtained by compressing a heat generating composition molded body as accommodated in a molding die within the die to such an extent that the thickness is 70 % of the die thickness holds 80 % or more of exothermic rising properties of the exothermic rising properties of the heat generating composition molded body before compression (a difference in temperature between one minute and 3 minutes after starting a heat generation test of the heat generating composition).

Here, the measurement method of exothermic rinsing properties for the resistance to compression will be described below.

**[0057]**   [0052]

1. Heat generating composition molded body:

1) A magnet is provided in the vicinity of a central part of the back side of a polyvinyl chloride-made supporting plate (3 mm in thickness x 600 mm in length x 600 mm in width) of a footed supporting table so as to cover a cavity shape of a molding die.

2) A temperature sensor is placed on the central part the surface of the supporting plate.

3) A polyethylene film (25 $\mu$m in thickness x 250 mm in length x 200 mm in width) as provided with an adhesive layer having a thickness of about 80 $\mu$m is stuck onto the supporting plate via a sticky layer such that the center of the polyethylene film is positioned at the sensor.

4) On an underlay plate (280 mm in length x 150 mm in width x 50 $\mu$m to 2 mm in thickness), a polyethylene film (230 mm in length x 155 mm in width x 25 $\mu$m to 100 $\mu$m in thickness) is placed such that one end of the polyethylene film is projected by about 20 mm outside the underlay plate and that one end thereof in the length direction is substantially coincident with one end of the underlay plate.

5) A template (230 mm in length x 120 mm in width x 3 mm in thickness) having a cavity (80 mm in length x 50 mm in width x 3 mm in height) is placed on the polyethylen film placed on the underlay plate; a template is placed on the polyethylene film such that one end thereof in the length direction is fitted to one end where the underlay plate and the polyethylene film are coincident with each other and that in the width direction, one end part of the width of the template is placed at a position of the central part by about 20 mm far from an opposing end to the side where the polyethylene film is projected outward from the underlay plate. Next, the resulting assembly is placed on the supporting plate together with the underlay plate.

6) A sample is placed in the vicinity of the cavity; a force-in die plate is moved along the molding die; the sample is charged into the cavity while stuffing; and the sample is leveled while stuffing along the template plane (force-in die molding), thereby filling the sample in the die.

7) Next, the magnet beneath the supporting plate is removed; the end portion of the projected polyethylene film is pressed; the underlay plate is removed; and the temperature measurement is started.

2. Heat generating composition compressed body:

1) to 6) are the same as in the case of the heat generating composition molded body.

8) A die having a convex having a thickness of 0.9 mm which can substantially tightly come into the cavity in relation of the cavity with an unevenness is fitted to the cavity and compressed by a roll press or plate press to prepare a heat generating composition compressed body having a thickness of 2.1 mm (compressed to 70 % of the die thickness) within the die.

9) The resulting assembly is placed on the supporting plate together with the underlay plate; the magnet beneath the supporting plate is removed; the end portion of the projected polyethylene film is pressed; the underlay plate is removed; and the temperature measurement is started.

With respect to the measurement of the exothermic temperature, the temperature is measured for 5 minutes at a measurement timing of 2 seconds using a data collector, and resistance to compression is judged in terms of a difference in temperature between after elapsing one minute and after elapsing 3 minutes.

The thickness after compression is preferably from 50 to 99.5 %, more preferably from 60 to 99.5 %, and further preferably from 60 to 95 % of the die thickness.

Incidentally, in the invention, it is to be noted that the heat generating composition molded body includes a heat generating composition compressed body.

In the invention, as a heat seal material constituting a heat seal layer, a single raw material may be used, or a composite raw material having a heat seal layer may be used. The heat seal material is not limited so far as at least a part thereof can be welded upon heating. Examples thereof include hot melt based resins such as polyolefins (for example, polyethylene and polypropylene) or olefin copolymer resins, ethylene based hot melt resins (for example, ethylene-vinyl acetate copolymer resins and ethylene-acrylic acid ester copolymer resins (for example, ethylene-isobutyl acrylate copolymer resins)), polyamide based hot melt resins, butyral based hot melt resins, polyester based hot melt resins, polyamide based hot melt resins, polyester based hot melt resins, polymethyl methacrylate based hot melt resins, polyvinyl ether based hot melt resins, polyurethane based hot melt resins, polycarbonate based hot melt resins,such as polyvinyl acetate, and vinyl chloride-vinyl acetate copolymers; and films or sheets thereof. Furthermore, in these hot melt based resins or films or sheets thereof, ones having various additives (for example, an antioxidant) compounded therein can be used. In particular, low density polyethylene and polyethylene obtained by using a metallocene catalyst are useful.

In the case of interposing a heat generating composition molded body between a substrate and a covering material, the "temporary adhesion" as referred to in the invention means weak pressure-sensitive bonding or adhesion for the purpose of holding the accommodated heat generating composition molded body until at least the substrate and the covering material are adhered to each other via a sticky layer made of an adhesive and heat sealed.

Furthermore, the "deadhesion" as referred to herein means that in the temporary adhering seal part after heat seal, the heat generating composition in a non-heat sealed region is transferred to the foregoing region, thereby releasing the temporary adhesion.

The temporary adhering seal part is formed via a sticky layer. An adhesive constituting the sticky layer is not limited so far as it is a layer formed of a polymer composition which is tacky at the normal temperature and can be heat sealed after the temporary adhesion.

Furthermore, although the adhesive of the foregoing adhesive layer can be used as the adhesive constituting the sticky layer to be used for the temporary adhesion, a non-hydrophilic adhesive is preferable. As the adhesive constituting the sticky layer, one which is well compatible with the heat seal material constituting the heat seal is preferable, and a melting point of a base polymer of the adhesive is preferably not higher than a melting point of the heat seal material. In particular, hot melt based adhesives are preferable. Furthermore, in the case where the heat seal material is made of an olefin based raw material, preferred examples of the adhesive include olefin based adhesives.

Incidentally, a method for providing a sticky layer for the temporary adhesion is not limited. The sticky layer may be entirely provided or partially or intermittently provided. Examples of its shape include various shapes such as a network-like shape, a stripe-like shape, a dot-like shape, and strip-like shape.

The cross-sectional shape of the seal surface may be a planar and plain surface. However, for the purposes of not only making fashionableness rich and imparting visible pleasantness but also making slipperiness small in forwarding the flexible heat generating body, it is preferred to form irregularities to provide a pattern.

It is not always required that this pattern is provided over the whole of the seal surface. A pattern may be provided only in the side of the sectional exothermic part, with the remainder being not provided with a pattern. Inversely, no pattern may be provided in the side of the sectional exothermic part, with the remainder being provided with a pattern.

The pattern on the seal surface is not particularly limited so far as the cross-sectional shape is irregular. Examples thereof include an orthogonal lattice shape, a parallel vertical linear shape, a parallel horizontal linear shape, an alternate shape, an oblique lattice shape, a broken oblique linear shape, an oblique checkerwork shape, and a scattered point shape. For example, no pattern may be provided in the side of the sectional exothermic part, with the central part being patterned. Inversely, the side of the sectional exothermic part is patterned, with the remainder as a central part being not provided with a pattern. The pattern can be arbitrarily chosen. Furthermore, a pattern in the surroundings as the circumferential part of the flexible heat generating body is the same.

A method for providing a pattern on the seal surface is not limited. Examples thereof include a method in which a pattern having an irregular cross-sectional shape in a seal part of a seal bar or a seal roll which is a seal die and sealing is carried out using the seal die.

Furthermore, the substrate and the covering material of the invention are heat sealed. In addition to heat sealing, other sealing methods can be used depending upon the utility.

As one example, sealing is carried out in a point-like (intermittent) manner or entirely by compression sealing (adhesive sealing), warm compression sealing (adhesive sealing), bonding sealing, heat bonding sealing, heat melt sealing (heat sealing), etc. by means of pressurizing, warming, heating or a combination thereof via an adhesive layer and/or a bonding agent layer and/or a heat seal layer. Selection of any one or a combination of these methods may be made depending upon the desire.

Furthermore, in the case of producing the flexible heat generating body by using a film having a heat seal layer as the substrate or the covering material, the flexible heat generating body may be produced by using a perforated heat sealable plastic film as an air-permeable substrate or the like to produce a flexible heat generating body and sticking a non-woven fabric in the air-permeable side thereof via an air-permeable sticky layer, thereby keeping warmth at the time of use or preventing leakage of collapsed pieces of the heat generating composition; or by packaging the either side thereof by a non-woven fabric or the like to form a flexible heat generating body for thermal muffler.

In the alternately disposed plural notch parts penetrating in the thickness direction, the flexibility holding member is non-stretchable, and the plural notch parts are provided with an angle against the longitudinal direction, preferably a perpendicular direction to the long axis direction.

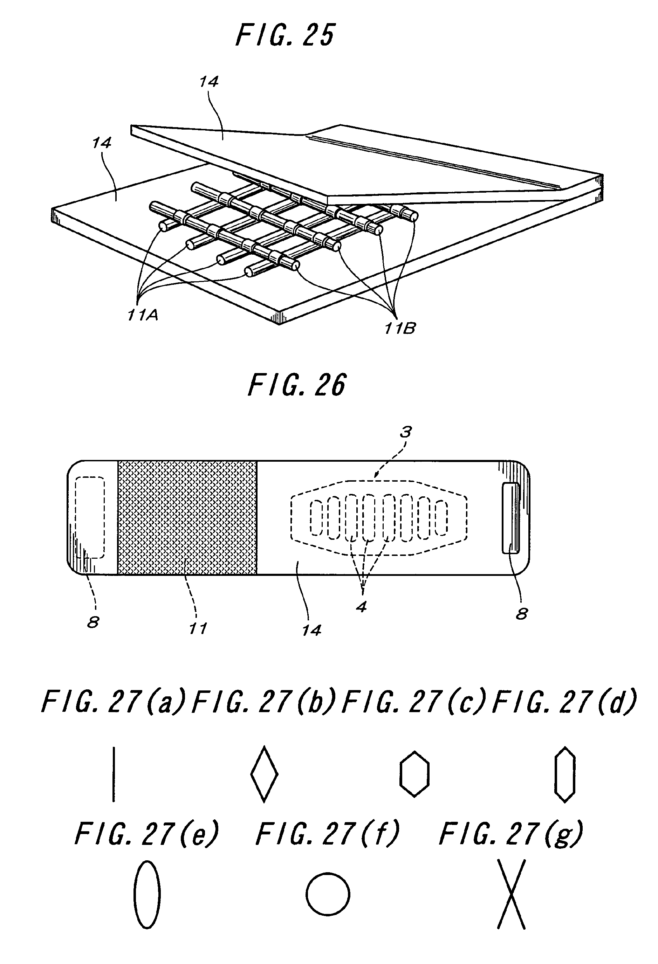

The shape of the notch part is not limited, and examples thereof include (a) a linear shape, (b) a rhombic shape, (c) a hexagonal shape, (d) an armor shape, (e) an elliptical shape, (f) a circular shape, and (g) an X shape as shown in Fig. 27.

In the case where the notch part is stretched, when the shape of the notch part causes deformation, the flexible heat generating body can stretch or extend. In particular, a portion having alternately disposed plural notch parts penetrating in the thickness direction becomes a geometric shape, especially a netlike shape so that it becomes more freely deformable.

With respect to the dimension of the notch part, the length, the longest diameter or the longest side is preferably from 1 to 50 mm, more preferably from 1.5 to 50 mm, further preferably from 2 to 30 mm, and still further preferably from 5 to 20 mm.

The width, the shortest diameter or the shortest side is preferably more than 0 mm and up to 50 mm, more preferably more than 0 mm and up to 30 mm, further preferably more than 0 mm and up to 10 mm, and still further preferably more than 0 mm and up to 5 mm.

Incidentally, a mutual space (feed width of notch) in the extending direction of an opposing notch part is preferably from 0.5 to 10 mm, more preferably from 0.5 to 8 mm, further preferably from 1 to 7 mm, and still further preferably from 2 to 6 mm. A mutual space (notch width) in the orthogonal direction to the extending direction of the notch part is preferably from 1 to 20 mm, more preferably from 2 to 15 mm, further preferably from 3 to 15 mm, and still further preferably from 5 to 15 mm.

The shape after stretching the alternate notch parts is not limited. Furthermore, with respect to the dimension of the netlike shape after stretching the alternate notch parts, when a linear notch part is taken as an example, it is preferable that the dimension (corresponding to the notch length) of the elongating direction (stretching direction) of the netlike shape is from 1 to 100 mm and that the dimension of the contracting direction (orthogonal direction to the stretching direction) is from 1.5 to 50 mm.

Incidentally, it is preferable that a mutual space (feed width of notch) in the extending direction of an opposing linear notch part is from 0.5 to 10 mm and that a mutual space (cutting width of notch) in the orthogonal direction to the extending direction of the linear notch part is from 1 to 20 mm.

In the case of using a non-extensible material as a flexible material for the flexibility holding member, when the alternately disposed plural notch parts penetrating in the thickness direction causes deformation in a geometric shape, extensibility or stretchability is revealed. The degree of extension of the flexibility holding member is influenced by selection of the geometric shape. In particular, when it is intended to form a rhombic pattern, the extensibility or stretchability is increased.

Though a measure for disposing the notch part is not particularly limited, for example, it is possible to use an expander cutter, a rotary die cutter, a die cutter, etc. In the case of using an expander cutter, it is possible to easily form a notch in a thick substrate, etc. or an adhesive tape or a double separator-provided adhesive tape; and in the case of using a rotary die cutter, not only it is possible to achieve continuous works, but also it is possible to form a notch in a substrate, etc. or from the sticky surface side.

The "alternate disposition" as referred to herein means that notches are alternately disposed such that by stretching the substrate, a netlike shape can be formed. Different from a net as prepared by subjecting strings to netlike processing, the welded portion is integral so that the netlike shape can be formed while spreading only a certain notch part length.

In the case of forming the alternately disposed plural notch parts penetrating in the thickness direction, when linear notch is taken as an example, it is described in metal lath processing of JIS-A5505 or the like. By this notch part, stretching becomes possible in the vertical direction to the longitudinal direction, and a netlike shape can be formed such that the shape can be freely changed.

It is preferable that the flexible heat generating body of the invention can be stretched in the vertical direction to the longitudinal direction of the notch part. A rate of extension varies depending upon the utility but is preferably from 1.1 to 10 times, more preferably from 1.2 to 10 times, further preferably from 1.5 to 10 times, and still further preferably from 2 to 6 times. When the rate of extension is less than 1.1 times, shape follow-up properties are insufficient, whereas when it exceeds 10 times, mesh openings are too large, leading to a lowering of the tensile strength. The "rate of extension (times)" as referred to herein means the quotient resulting from dividing a length after the extension by a length before the extension.

The tensile strength is not limited so far as it is employable for the flexible heat generating body. The tensile strength varies depending upon the utility but is preferably 3.0 N/50 mm or more, more preferably 3. 3 N/50 mm or more, further preferably 10 N/50 mm or more, still further preferably 20 N/50 mm or more, even further preferably 30 N/50 mm or more, and even still further preferably 40 N/50 mm or more.

When the tensile strength is less than 3.0 N/50 mm, when the flexible heat generating body is stretched into a netlike shape, the notch parts may possibly be cut depending upon the utility. The "tensile strength" as referred to herein means a tensile strength which the substrate or the like has after forming a linear notch. As a method for measuring a sample having alternately disposed plural notch parts penetrating in the thickness direction, a method in which according to the tensile test: JIS L1096, a sample as notched in a width of 50 mm is used and extended to the orthogonal direction to the notch direction and a strength is measured by the usual measurement of films is employable.

The "extensibility" as referred to herein refers to properties that when a tensile force is given, the material can extend, especially 1.1 times or more of the original length without causing breakage, and it does not matter whether or not when the tensile force is eliminated, it is returned to the original state.

The "stretchability" as referred to herein refers to characteristics of material that when a tensile force is given, the material stretches to the force direction without causing breakage, wherein when the tensile force is eliminated, it is returned to a range within 1.03 times of the original length.

Furthermore, it is preferable that recovery of the material or member is achieved within 30 seconds after elimination of the force from the length at the time of stretching.

On the other hand, when the material or member is not recovered within 30 seconds after elimination of the force from the length at the time of stretching, it is defined that the material or member is non-stretchable.

The thickness of the extensible material is not particularly limited so far as when a tensile force is given to a flexibility holding member as formed by using at least such a material, the flexibility holding member extends 1.1 times or more of the original length without causing breakage. An extensible material having a thickness of from 5 to 15 $\mu$m is preferable. Examples thereof include synthetic resin-made single-layered films. Furthermore, the non-extensible material is a material other than the extensible material. Specifically, examples thereof include non-woven fabrics and biaxially stretched films.

Furthermore, in the invention, since the flexible heat generating body is suitably applied to curved parts, stretching parts or bending and stretching parts of a human body, a stretchable material can also be used. In particular, stretchable films, sheets, non-woven fabrics or woven fabrics or laminates thereof are the most desirable because they are extremely stretchable, more follow up joint parts such as elbows and knees, curved parts, stretching parts or bending and stretching parts of a human body such as shoulders and arms and have excellent adhesiveness and are free from a tight feeling or an uncomfortable feeling during the use so that the feeling for use is more satisfactory and separation during the use can be surely prevented.

Examples of the stretchable material include single materials such as natural rubbers, synthetic rubbers, elastomers, and stretchable shape memory polymers; fabrics, films, spandex yarns, yarns, strands, flat plates, ribbons, slit films, expanded bodies, and non-woven fabrics, which are constituted of a mixture, a mixed material or a blended material of such a stretchable raw material and a non-stretchable raw material; and composite stretchable materials made of a laminate among these materials or with a non-stretchable material. Here, materials resulting from a processing method by entangling a non-stretchable long fiber or a continuous filament at random and bonding or melting it at random, thereby imparting stretchability as a whole are also included. Furthermore, protective stretchable yarns formed by winding nylon yarns on urethane yarns may be employed.

Here, of the elastomers, thermoplastic elastomers having thermoplastic properties are desirable because they have heat fusibility and are every easy for producing a laminate with a non-woven fabric or the like. In the case of a raw material having no heat fusibility, heat fusibility may be imparted by blending or mixing a thermoplastic resin, or bonding may be achieved by using a bonding agent (including adhesives) such as hot melt based materials. In addition, in the case where the stretchable material is impermeable to air, a flexibility holding material to which stretchability or extensibility and permeability to air are imparted by boring using boring means such as a heat pin system and an embossing system can be constituted. That is, in the case where the flexibility holding member has stretchability, it is only required that the flexibility holding member is stretchable. A single material or a composite made of a combination of stretchable materials or with a non-stretchable material may be employed.

Specific examples of the synthetic rubber include butadiene rubbers, 1,2-poybutadiene, isoprene rubbers, styrene-butadiene rubbers, styrene-butadiene-styrene copolymers, butyl rubbers, acrylonitrile-butadiene rubbers, chloroprene rubbers, isobutylene-isoprene rubbers, polyalkylene sulfides, silicone rubbers, poly(chlorotrifluoroethylene), vinylidene fluoride-hexafluoropropylene copolymers, urethane rubbers, propylene oxide rubbers, epichlorohydrin rubbers, acrylic ester-acrylonitrile copolymers, and acrylic ester-2-chloroethyl vinyl ether copolymers.

Furthermore, specific examples of the thermoplastic elastomer include olefin based elastomers, urethane based elastomers, ester based elastomers, styrene based elastomers, amide based elastomers, vinyl chloride elastomer, syndiotactic poly(1,2-butadiene), poly(trans-1,4-isoperene), and silicone based elastomers.

Specific examples of the olefin based elastomer include ethylene-propylene copolymers, ethylene-propylene-diene terpolymers, chlorosulfonated polyethylene, chlorinated polyethylene, and ethylene-vinyl acetate copolymers. Above all, cyclopentadienyl complexes, namely ethylene-$\alpha$-olefins as formed using a metallocene catalyst are especially preferable.

As the $\alpha$-olefin, 1-hexene, 1-octene, 1-heptene, 4-methylpentene-1, and so on are especially preferable.

Examples of the urethane based elastomer include urethane based elastomers composed of a urethane bond-containing block and a polycarbonate based polyol, an ether based polyol, a polyether/polyester based polyol or a caprolactone based polyester block. In particular, polyurethane films formed therefrom are characterized in that they are non-porous and have both permeability to moisture and stretchability.

Examples of the ester based elastomer include ester based elastomers composed of an aromatic polyester-containing block and an aliphatic polyester- or aliphatic polyether-containing block.

Examples of the stretchable shape memory polymer include polyisoprene bases, copolymers such as styrene-butadiene bases, polyurethane bases, and polymer alloy bases.

Though all of the foregoing materials are employable as the stretchable material, the foregoing natural rubbers, synthetic rubbers, urethane based elastomers, stretchable shape memory polymers, ethylene-$\alpha$-olefins, and composites containing the same are preferable in view of the relationship with extension recovery rate.

The non-stretchable material refers to one other than the foregoing stretchable materials. Examples thereof include polymer materials such as polyethylene, polypropylene, polyesters, polyvinyl chloride, polyvinylidene chloride, polystyrene, ethylene-vinyl acetate copolymer saponified products or ethylene-vinyl acetate copolymers, polycarbonates, aromatic or aliphatic polyamides, polysulfones, polyvinyl alcohol, polyacrylonitrile, vinyl chloride-vinylidene chloride based resins, polyimides, hydrochlorinated rubbers, polyphenylene, aromatic or aliphatic polyamides, polysulfones, polyvinyl alcohol, polyacrylonitrile, vinyl chloride-vinylidene chloride based resins, polyimides, hydrochlorinated rubbers, polyphenylene oxide, polyphenylene sulfide, polyamide-imides, epoxy resins, polyaminobismaleimide, polyacetals, polyetheretherketones, polyether sulfones, polyallylate, and polyhydroxybenzyl; natural materials such as paper, pulp, fibers, and cotton; and fabrics, woven fabrics, non-woven fabrics, films, sheets, or expanded sheets made of a combination thereof. Materials which are non-stretchable or substantially non-stretchable such as materials resulting from providing an adhesive or the like on a stretchable substrate and materials resulting from biaxial stretching are also included in the non-stretchable material. Furthermore, these materials can be used singly or as a laminate of two or more kinds thereof.

The composite stretchable material refers to a substrate which has stretchability as a whole by a combination containing the foregoing stretchable material and a material different in any one of shape, properties or kind from the foregoing material for use. The following are included.

1) One example in which a stretchable film is used can be constituted as a belt resulting from making a stretchable film such as urethane based elastomers in an extended state, bonding or melting a non-woven fabric (for example, nylon-made fabrics) in at least one side thereof and releasing the extended state, thereby imparting stretchability. By bonding or melting a non-woven fabric by an embossing or heat pin system or the like at the time of extension and simultaneously boring a stretchable material such as urethane based elastomer films, it is possible to form a belt to which stretchability and permeability to air are imparted. Here, a laminate of a resin film such as polyethylene films and a non-woven fabric may be used in place of the foregoing non-woven fabric. Furthermore, by using a previously bored stretchable raw material such as the resin films and urethane based elastomer films, it is possible to form a belt to which stretchability and permeability to air are imparted.

2) As a stretchable material, a plurality of yarn-like urethane made of a yarn-like stretchable material (0.5 mm$\phi$) or a 0.8 mm$\phi$-thick yarn-like acrylonitrile-butadiene rubber provided with a hot melt bonding agent (or an adhesive) were disposed at fixed intervals (for example, intervals of 5 mm), stretched 100 % or more in the longitudinal direction and fixed at that position; a substrate made of a resin film such as a non-woven fabric-laminated polyethylene film was placed on one side thereof such that the resin film and the stretchable substrate were faced at each other; and the resin film and the stretchable substrate were intermittently bonded (adhered) to each other. Thereafter, the extension is released, thereby returning it to an original state. By returning to an original state, the non-woven fabric becomes narrower and is lined with pleats so that engagement with a male fastener of a hook and loop fastener becomes good.

3) By changing the thickness of the non-woven fabric at certain intervals (for example, intervals of 2 mm), a holding material made of a stretchable non-woven fabric is prepared, and the non-woven fabric is stretched and fixed. On the other hand, stretchable yarns such as adhesive-provided polyurethane yarns which are a stretchable material having a length at the time of releasing the extension of the foregoing holding material made of a stretchable non-woven fabric is extended to a length equal to that the extended holding material and subjected to point heat bonding to the extended and fixed holding material at certain intervals (for example, intervals of 2 mm). After heat bonding, the extension is released to form a stretchable material which is less in pleats. Incidentally, point contact bonding may be carried out by using an adhesive.

4) Plural non-woven fabrics in a strip-like form are disposed; a hot melt based adhesive such as SIS is coated in a cobweb-like state thereon by a melt blow method, etc.; a yarn-like stretchable substrate made of plural bare urethane yarns, etc. in an extended state is placed thereon such that it intersects the non-woven fabrics; non-woven fabrics in a strip-like form are placed thereon; and after heat contact bonding by a heat roll processed with TEFLON (a registered trademark), the extended state is released to form a stretchable material.

5) After laminating a stretchable lattice-like (netlike) sheet and a card web (precursor of fibrous laminate) in at least one side of the lattice-like sheet, the respective materials are entangled by a high-pressure water flow, etc. and integrated by bonding or melting at random, thereby forming a stretchable part. Here, with respect to the lattice-like netlike sheet, an elastic yarn as produced from an elastic raw material such as a thermoplastic elastomer, for example, ethylene-$\alpha$-olefins or an elastic body in a thin belt-like form as obtained by slitting the foregoing film is used as a warp or a weft and disposed, and points of intersection are welded by heat melting or bonding using a bonding agent or other methods, thereby obtaining a stretchable material. Examples of a fiber which constitutes the fibrous laminate include polyolefins, polyesters, polyamides, cotton, and rayon. The lattice-like sheet can be produced from an elastic body yarn by methods as disclosed in JP-A-49-118963, JP-A-49-62771, etc. Examples of a method for preparing the lattice-like sheet include a method in which a cylindrical net having prescribed strand diameter and pitch is prepared from a raw material to be used for the foregoing stretchable material or non-stretchable material by using a uniaxial extruder and a circular net die and cut open, followed by cutting in a certain fixed direction in fixed width and length. In addition, a sheet as prepared by heating this at a fixed temperature lower than the melting point, extending, cooling to room temperature as it is and releasing a tension may be used. Since this heating and extension treated sheet is not substantially stretchable in the temperature range lower than the heating and extension treatment temperature, the original stretchability can be exhibited by processing this sheet by sticking a non-woven fabric or the like and then holding it for ten-odd seconds at a temperature exceeding the heating and extension treatment temperature. The cross-sectional shape of the netlike wire may be any shape of a circular shape, an elliptical shape, a triangular shape, a square shape, a hexagonal shape, etc., or plural wires made of twisted wires may be employed. The diameter is preferably from 0.01 to 5.0 mm, and it is not required that all of them are uniform. Furthermore, in the netlike mesh, it is desired that a width in the extension direction is from 0.1 to 15 mm and that a width in the perpendicular direction thereto is from 0.1 to 15 mm. Furthermore, the shape of the netlike opening is not limited to a square but may be a substantially circular shape or an elliptical shape. Furthermore, the cross point of the netlike wire may be bonded or intersected in a knitted state. In addition, it is desired that an apparent thickness of the netlike sheet (as generated due to overlap or deformation of the wire) is from about 0.5 to 5.0 mm. Furthermore, the shape of its molded article may be kept by extending the molded article in an extending direction at a temperature lower than the melting point, cooling while applying a tension, releasing the tension and having a 5 % strain tensile stress in the extending direction of from 5 to 40 times of an apparent 5 % strain tensile stress before the extension. A stretchable material may be prepared in such a manner that a yarn-like material or a strand-like material in the MD direction of the stretchable material of the lattice-like sheet has a softening point higher than that of a yarn-like material or a strand-like material in the TD direction; the yarn-like material or the strand-like material in the TD direction is at least thermoplastic and heat fusible, and an outside substrate such as a non-woven fabric, etc. is provided on at least one surface in the state that the stretchable material of the lattice-like sheet is extended in the MD direction, the yarn-like material or the strand-like material in the TD direction and the outside substrate, etc, are molten upon heating under pressure, and the extending state is then released.

6) The substrate is composed of two layers of a cardet heat fused thermoplastic resin (for example, polypropylene) non-woven fabric and a netlike stretchable sheet as interposed therebetween, in which a pressure sensitive hot melt adhesive is coated between the non-woven fabric and the netlike stretchable sheet in a level of from 0.31 to 0.93 mg/cm$^2$ by a spiral coating method. This three-layered structure is assembled by using a netlike stretchable sheet under a tension of zero, the assembled three-layered structure is passed through one pair of ring rolls as combined at a fixed interval (for example, an interval of 2 mm), and cut lines are provided on the non-woven fabric by a thin scale. Alternatively, after assembling by using a netlike stretchable sheet, a portion of a region on the entire surface on which the flexible heat generating body is provided is entirely contact bonded, thereby forming an intermittently stretchable region or a non-stretchable region, the assembled three-layered structure is then passed through one pair of ring rolls as combined at an interval of 2 mm, the processed part is removed, and cut lines are provided on the non-woven fabric by a thin scale. At the time of use, the cut line-incorporated non-woven fabric-provided netlike stretchable sheet is stretched and then used.

7) A laminate in which a mesh is disposed between a first substrate, etc. and a second substrate, etc. is not limited so far as the mesh has a mesh form and is stretchable. Examples thereof include a mesh in which plural first strands intersecting plural second strands are present; the first and second strands have a softening temperature under an applied pressure; and at least about 10 % of the first strands are integrally coupled with the first substrate, etc. and the second substrate, etc. as substrates by applying a coupling pressure in the softening temperature of the first stands. The laminate is a flexible material member in which the mesh is integrally coupled with the first substrate, etc. and the second substrate, etc. and is a laminate capable of extending the flexible material member in the longitudinal direction.

Fig. 25 is an exploded view of constitutional parts of a stretchable material using an elastic mesh.

The stretchable material is formed of a stretchable mesh having plural first strands and plural second strands as disposed therebetween and integrally thermally coupled therewith and a first substrate, etc, and a second substrate, etc. as two substrates, etc.

Coupling of the substrates, etc, and the mesh can be achieved by using an adhesive, a bonding agent, heat melting, etc., and it is desired that coupling is partially achieved. In particular, in order to have extensibility at least on the longitudinal direction, it is desired that coupling is provided at a certain angle against the extending direction, preferably at intervals in the extending direction against the orthogonal direction. In the case where the substrates, etc. are non-extensible, stretchability may be given by a processing method. Examples of the processing method include a method in which the mesh is extended in a prescribed amount and coupled, the substrates, etc. are bent in a proportion corresponding to the extension and in regions other hand the coupled places, the substrates, etc. are cut in a direction with an angle against the extending direction, preferably one or more plural places are cut in the orthogonal direction.

The stretchability of the mesh may be made different depending upon the direction by making the stretchability different between the first strand and the second strand.

The first substrate, etc. and the second substrate, etc, may be different from each other with respect to the shape and size.

Furthermore, integrally coupled plural stretchable meshes may be incorporated between the first substrate, etc. and the second substrate, etc.

With respect to the stretchable material, it is desired that a structural direction which is partially or wholly stretchable over the length thereof is obtained at least in the longitudinal direction.

Furthermore, a structural direction in which a part of the length is stretchable, whereas a part thereof is non-stretchable may be provided.

The materials which form the first and second strands of the stretchable materials constituting the mesh or scrim can be selected from polymers such as polyolefins, polyamides, polyesters, and natural rubbers or synthetic rubbers (for example, SBR, polybutadiene rubbers, polychloroprene rubbers, and nitrile rubbers); or a number of polymer materials which can be extended or returned to the original state. Suitable materials are not limited thereto but include styrene based block copolymers, rubbers, polyethylene inclusive of one as produced by using a metallocene catalyst, and expanded bodies containing polyurethane or polyester.

8) A pasty or liquid rubber material is bonded in a band-like state on a pleat-provided part as provided in a required place of a non-stretchable material and solidified to form a stretchable material.

Furthermore, bonding (including adhesion) at the time of processing of a raw material into the flexibility holding member is carried out by a single method or a combination of known methods such as use of a hot melt bonding agent (or an adhesive) and fusion (for example, heat fusion, pressure fusion, heat pressurizing fusion, and ultrasonic melting). The bonding or fusion between the materials may be partially or wholly achieved and can be achieved even in the case where the material is an integrated material or one raw material is sandwiched by materials of other kind. However, it is preferable in view of texture, etc. that bonding is intermittently carried out such that the stretchability of the material is not hindered. In the case of coating an adhesive, it is recommended that the adhesive or the like is provided in a partially continuous manner such that the adhesive is linearly coated in a striped form in the orthogonal direction to the longitudinal direction of the flexibility holding member or an intermittent manner such that it is coated in a netlike or point-like state. So far as the material is soft and keeps texture, all entire bonding or entire fusion may be employed.

Examples of the non-woven fabric include non-woven fabrics, stretchable non-woven fabrics, and non-stretchable non-woven fabrics. Examples of such a material include vegetable fibers such as pulp, hemp, cotton, rayon, and acetates; synthetic pulps made of polyethylene, etc. as the raw material; single fibers or composite fibers of thermoplastic polymer substances including polyolefin bases aiming a self welding type, such as polyethylene, polypropylene, copolymers composed mainly of propylene and ethylene, and propylene-ethylene-butene ternary random copolymers, polyamide bases such as nylon 6, and polyester bases such as polyethylene terephthalate; mixed fibers thereof; and mixtures thereof with cellulose fibrous pulp, etc. With respect to the non-woven fabric, though short fiber non-woven fabrics, long fiber non-woven fabrics, and continuous filament non-woven fabrics can be used, long fiber non-woven fabrics and continuous filament non-woven fabrics are preferable in view of mechanical properties. Examples of the stretchable non-woven fabric include polyurethane based non-woven fabrics.

It is preferable that the flexibility holding member has permeability to air. In that case, the air permeability of the air-permeable flexibility holding member is preferably not more than 1,000 seconds/100 cc, more preferably not more than 500 seconds/100 cc, and further preferably not more than 100 seconds/100 cc according to the Gurley method. When the air permeability exceeds 1,000 seconds/100 cc, an unwell feeling such as sweat becomes strong.

Furthermore, the film or sheet in the side faced at the skin of a human body, which constitutes the flexibility holding member, may be provided with an ability and/or water absorbing properties for absorbing secretions such as sweat and body fluids from the mouth of a wound, or water may be previously contained so as to supply water to the skin. Specific examples thereof include materials in which a water absorptive paper, woven fabric, non-woven fabric or expanded sheet is laminated on the inside of an air-permeable film or sheet; materials in which a water retaining agent is sprayed or laminated on the inside of an air-permeable film or sheet; and materials in which a paper, woven-fabric, non-woven fabric or expanded sheet carried with a water absorptive polymer or a water retaining agent such as bentonite is laminated on the inside of an air-permeable film or sheet.

A portion stretching at least in the intersecting direction with the longitudinal direction may be constituted by a belt which stretches in the both directions. Furthermore, a material which stretches at least in one direction may be installed on a raw material or flexible heat generating body which stretches in the longitudinal direction. The foregoing material which stretches at least in the intersecting direction with the longitudinal direction may be installed by entire fixation or partial fixation such as bonding (including adhesion) or fusion so far as it can be stretched in the intersecting direction with the longitudinal direction over a void. Furthermore, an installing angle is not limited so far as a fixing function can be kept. Furthermore, the foregoing respective material or flexible heat generating body may be rounded by trimming. As a material in the case of installing the material separately, for example, stretchable materials, materials which are stretchable in only one direction thereof and processed products thereof can be used.

Furthermore, the non-extensible non-woven fabric is disclosed in JP-T-2002-501127, the disclosures of which can be all incorporated in this description by reference.

Furthermore, the extensible material or stretchable material is disclosed in JP-A-8-58007, JP-A-2002-501127, JP-A-2003-520705, JP-A-2004-76178, JP-A-2002-307627, JP-A-2002-283479, JP-A-2000-514730, JP-T-2001-514093, and JP-A-2003-533374, the disclosures of which can be all incorporated in this description by reference.

Each of the flexible material, the extensible material, the stretchable material and the non-stretchable material may be transparent, opaque, colored, or colorless. Furthermore, each of these materials may be provided with symbols, pictures, photographs, figures, patterns, etc.

In addition, in the flexible heat generating body of the invention, for the purposes of realizing high-speed heat sealing, making the heat seal width thin and securing heat sealing, it is preferable that the substrate and the covering material are subjected to temporary adhesion via a sticky layer and then heat sealed.

That is, the substrate and the covering material of the air-permeable accommodating bag have a heat seal layer, the heat seal part is formed by heat sealing after temporary adhesion by a sticky layer, and adhesive components which constitute the sticky layer and heat sealing material components which constitute the heat seal layer are copresent in the heat seal part.

By making the heat seal part width for sectioning the flexible exothermic part thin, a space between the sectional exothermic parts becomes narrow, a heat insulating effect can be mutually kept, and the exothermic part can be sectioned without causing a lowering of exothermic characteristics such as a lowering of heat generation time due to sectioning of the exothermic part. Thus, it is possible to prepare a flexible heat generating body which is pliable and excellent in exothermic characteristics.

A thermal relieving sheet may be provided in the side of the flexible exothermic part coming into contact with the skin.

It is preferable that the exothermic parts of the invention are provided in a striped form at intervals. The terms "provided in a striped form at intervals" as referred to herein mean that elongated and continuous sectional exothermic parts and/or the adhesive layers are disposed at intervals and that stripes are disposed in, for example, a parallel stripe form, a radial form, or a folding fan form. In providing the stripes at intervals, it is preferable that the stripes have a width and a gap of from approximately 1 to 20 mm and from approximately 0.5 to 30 mm, respectively. These widths may be selected depending upon the utility. Furthermore, the orientation of the stripes can be adjusted in, for example, a parallel stripe form, a radial form, or a folding fan form depending on the distribution of the muscle or tendon of the affected part to apply.

In this way, in sticking to an affected part such that a muscle or tendon of the affected part is in parallel to the sectional exothermic parts, a reverse physical tension is intermittently given to the tension of the muscle or tendon, whereby the tension of the muscle or tendon is relieved.

Furthermore, since a fault strain is brought between the adjacent muscles or tendons and the tension of the muscles or tendons is relieved, the physical tension is reinforced. As a result, residence of vital energy and blood is dissolved and lightened.

When the flexible exothermic part is held by the body due to the flexibility holding member, uneven distribution of the heat generating composition does not occur during the use. Thus, it is possible to keep desired exothermic characteristics without being influenced by the posture or movement or the like during wearing. Besides, since heat of a heating generating bag can be efficiently transferred without needs of direct sticking to the skin, the amount of the heat generating composition of the flexible heat generating body can be reduced so that a light-weight and thin flexible heat generating body with a desired duration can be obtained.

Furthermore, in the flexible heat generating body of the invention, by adjusting the size and space of the sectional exothermic parts, a flexible exothermic part which is pliable and exhibits a uniform temperature distribution and a flexible exothermic part which exhibits a pattern-like temperature distribution are obtained. By this pattern-like temperature distribution, it is possible to improve a meridian point effect of the warming part.

Furthermore, since a packaging material on the surface of the flexible heat generating body coming into contact with the skin does not have an adhesive layer, it is possible to set up it relatively arbitrarily. Taking into consideration of feelings such as a touch feeling, heat transfer properties, water absorptivity, and so on, it becomes possible to employ an arbitrary raw material and an arbitrary shape so that an unwell feeling during the use can be overcome.

The flexible heat generating body is accommodated in an air-tight air-impermeable accommodating bag (outer bag), stored and transported. Examples thereof include a flexible heat generating body prepared by mediating a produced flexible heat generating body between two sheets of an air-impermeable film or sheet, punching the two sheets of film or sheet into a size larger than that of the flexible heat generating body at the same time with or after this mediation, and sealing the two sheets of film or sheet in the surroundings exceeding the size of the flexible heat generating body at the same time with or after this punching. The outer bag is not limited so far as it is impermeable to air and may be made of a laminate. Usually, an outer bag prepared from an air-impermeable raw material is used. Incidentally, for example, when the flexible heat generating body is bent centering on the flexible exothermic part and sealed in an outer bag, it becomes very compact and is convenient because saving of the outer bag, excellent portable properties, and the like are produced.

Though a method for using the flexible heat generating body of the invention is not limited, it can be used as a method for relaxing symptoms such as stiff shoulder, low-back pain, and muscular fatigue, thereby relieving the tension of a muscle or tendon which hinders the flow of vital energy and blood. The sectional exothermic parts in a striped form continuously give a physical tension in a striped form in parallel to a muscle or tendon which hinders the flow of vital energy and blood and in the opposite direction to the tension, whereby extremely effective effects can be expected. Furthermore, with respect to an ejector of a drug, a function for penetrating the drug into an affected part may be brought by providing the drug in a portion of the flexible heat generating body coming into contact with the skin.

Furthermore, at least one member of the heat generating composition molded body, the substrate, the covering material, the air-permeable adhesive layer and the underlay material may be subjected to an entire or partial pressurization treatment or may be formed with irregularities. In this way, the movement of the heat generating composition molded body between the substrate and the covering material may also be prevented.

Furthermore, when a molded body prepared by appropriately compressing the heat generating composition molded body under pressure is used, moldability is markedly improved. For example, even when a perforated film which is difficult with respect to the pressure adjustment is used as a raw material of the air-permeable part in place of the porous film, or even when an inner pressure of the accommodating bag becomes equal to or more than the outer pressure, shape collapse hardly occurs so that the use of a perforated film is possible. Accordingly, not only the range for selecting an air-permeable raw material is widened so that the costs can be lowered, but also a body to be warmed can be uniformly warmed at an appropriate temperature over a long period of time.

In the flexible exothermic part, a magnetic substance such as a magnet can also be accommodated therein for the purpose of containing a magnetic substance and improving circulation of the blood and improvement of stiff shoulder by the magnetic effect.

Furthermore, by covering the flexible exothermic part by an air permeability adjusting material via a sticky layer or a bonding layer, the permeability to air into the sectional exothermic parts may be adjusted. In the air permeability adjusting material, by covering the flexible exothermic part by the air permeability adjusting material while utilizing a difference of altitude between the sectional exothermic part and the sectioned part, a partitioned space is formed in at least a part of the periphery of the sectional exothermic part, thereby adjusting the permeability to air between the outside and the sectional exothermic part and also imparting a heat insulating effect. The permeability to air of the air permeability adjusting material is not limited so far as it is able to adjust air retention or permeability to air in at least a part of the periphery of the sectional exothermic part. However, it is preferable that the permeability to air of the air permeability adjusting material is lower than that on the air-permeable surface of the sectional exothermic part as a covering part for covering the heat generating composition molded body.

Furthermore, a region where the permeability to air is higher than that in the covering part for covering the heat generating composition molded body may be provided in a local region of the air permeability adjusting material by perforation, etc., thereby keeping the permeability to air of other region lower than that on the air-permeable surface of the sectional exothermic part. In this way, it is possible to control an air passage for air, etc.

As the raw material which constitutes the air permeability adjusting material, the raw materials which are used for the substrate of chemical body warmer or flexible heat generating body, the substrate which is used in the air-impermeable accommodating bag for sealing and accommodating the covering material and the flexible heat generating body therein, and the like can be used.

Though usually used adhesives or bonding agents can be used as the raw material which constitute the sticky layer, adhesives which are used in chemical body warmers or flexible heat generating bodies are preferable.

The fixing means is not limited so far as it has capability for fixing a thermal packaging body for joint surroundings or a material having an exothermic part to a prescribed part.

As the fixing means, an adhesive layer, a hook and eye, a hook and button, a hook and loop fastener such as Velcro, a magnet, a band, a string, and combination thereof can be arbitrarily used.

Incidentally, in the case of a band, fixing means for adjustment may be further constructed by a combination of a hook and loop fastener and an adhesive layer.

Here, the "hook and loop fastener" as referred to herein has a fastening function by a combination of a loop as a female fastener with a male fastener capable of fastening the female fastener thereto, which is known as trade names such as Magic Tape (a registered trademark), Magic Fastener (a registered trademark), Velcro Fastener, and Hook and Loop Tape. Examples of the material having a loop function include non-woven fabrics and woven fabrics of napped or hole-containing yarns. Such a material having a loop function (female fastener function) may be covered on the surface of a paddling forming the band, or the band may be constructed of such a material itself. Although the hook member which is the male fastener member is not particularly limited, examples thereof include hook members formed of a polyolefin based resin (for example, polyethylene and polypropylene), a polyamide, a polyester, etc. Although the shape of the hook is not particularly limited, a hook having a cross-sectional shape such as an I type, an inverted L type, an inverted J type, and a so-called mushroom type is preferable because it is easily hooked by the loop and does not give an extreme stimulus to the skin. Incidentally, the hook may be adhered to the entire area of a fastening tape, and only the hook may be used as a fastening tape while omitting a tape substrate.

The adhesive layer may contain at least one member selected from additional components consisting of a water retaining agent, a water absorptive polymer, a pH adjusting agent, a surfactant, an organosilicon compound, a hydrophobic polymer compound, a pyroelectric substance, an antioxidant, an aggregate, a fibrous material, a moisturizer, a functional substance, and a mixture thereof.

The adhesive of the invention is classified into a non-hydrophilic adhesive, a mixed adhesive, and a hydrophilic adhesive (for example, a gel).

The adhesive constituting the adhesive layer is not limited so far as it has an adhesive strength necessary for adhering to the skin or clothes. Adhesives of every form such as a solvent based adhesive, an aqueous adhesive, an emulsion type adhesive, a hot melt type adhesive, a reactive adhesive, a pressure-sensitive adhesive, a non-hydrophilic adhesive, and a hydrophilic adhesive are employable.

The adhesive layer includes one layer of a non-hydrophilic adhesive constituted of the non-hydrophilic adhesive and non-hydrophilic adhesive layers constituted of the non-hydrophilic adhesive.

It is to be noted that a material whose water absorption properties are improving by containing a water absorptive polymer or a water retaining agent in the non-hydrophilic adhesive layer is dealt as the non-hydrophilic adhesive layer.

A hot melt based adhesive may be provided between the hydrophilic adhesive layer and a substrate or a covering material. Furthermore, in the case where the hydrophilic adhesive is provided in a thermal packaging body for joint surroundings, there is no limitation. After seal treating a thermal packaging body for joint surroundings, a hydrophilic adhesive layer may be provided in the thermal packaging body for joint surroundings.

Furthermore, the adhesive layer may or may not have air permeability and may be properly selected depending upon the utility. With respect to the air permeability, the adhesive layer may be air-permeable as a whole. Examples thereof include an adhesive layer having air permeability as a whole of a region in which an adhesive is partially present and a portion where no adhesive is present is partially present.

In laminating an adhesive on an air-permeable substrate and/or a covering material in a stratiform state as it is, examples of a method for keeping its air permeability include a method in which an adhesive layer is partially laminated by printing or transferring an adhesive, thereby forming a non-laminated part as an air-permeable part; a method in which an adhesive is transferred in one direction while drawing a circle in a filament-like form or properly moved in the two-dimensional directions by transferring in a zigzag manner, whereby a space of the filament-like adhesive keeps air permeability or moisture permeability or the adhesive is foamed; and a method for forming a layer by a melt blow system.

Examples of the adhesive which constitutes the non-hydrophilic adhesive layer include acrylic adhesives, polyvinyl acetate based adhesives (for example, vinyl acetate resin based emulsions and ethylene-vinyl acetate resin based holt melt adhesives), polyvinyl alcohol based adhesives, polyvinyl acetal based adhesives, vinyl chloride based adhesives, polyamide based adhesives, polyethylene based adhesives, cellulose based adhesives, chloroprene (neoprene) based adhesives, nitrile rubber based adhesives, polysulfide based adhesives, butyl rubber based adhesives, silicone rubber based adhesives, styrene based adhesives (for example, styrene based hot melt adhesives), rubber based adhesives, and silicone based adhesives. Of these, rubber based adhesives, acrylic adhesives, and adhesives containing a hot melt based polymer substance for the reasons that they are high in the adhesive strength, are cheap, are good in long-term stability, and are small in reduction of the adhesive strength even by providing heat.

In addition to the base polymer, if desired, the adhesive may be compounded with other components such as tackifiers (for example, petroleum resins represented by rosins, chroman-indene resins, hydrogenated petroleum resins, maleic anhydride-modified rosins, rosin derivatives, and C-5 based petroleum resins), phenol based tackifiers (especially, tackifiers having an aniline point of not higher than 50 °C; for example, terpene phenol based resins, rosin phenol based resins, and alkylphenol based resins), softeners (for example, coconut oil, castor oil, olive oil, camellia oil, and liquid paraffin), softeners, anti-aging agents, fillers, aggregates, adhesion adjusting agents, adhesion modifiers, coloring agents, anti-foaming agents, thickeners, and modifiers, thereby improving performance such as an improvement in adhesion to nylon-made clothes and mixed yarn clothes.

Examples of the hot melt based adhesive include known hot melt based adhesives imparted with adhesion. Specific examples thereof include styrene based adhesives made of, as a base polymer, an A-B-A type block copolymer (for example, SIS, SBS, SEBS, and SIPS), vinyl chloride based adhesives made of, as a base polymer, a vinyl chloride resin, polyester based adhesives made of, as a base polymer, a polyester, polyamide based adhesives made of, as a base polymer, a polyamide, acrylic adhesives made of, as a base polymer, an acrylic resin, polyolefin based adhesives made of, as a base polymer, a polyolefin (for example, polyethylene, super low density polyethylene, polypropylene, ethylene-α-olefin copolymers, and ethylene-vinyl acetate copolymers), 1,2-polybutadiene based adhesives made of, as a base polymer, 1,2-polybutadiene, and polyurethane based adhesives made of, as a base polymer, polyurethane; adhesives made of a modified body of the foregoing adhesive whose adhesion is improved or whose stability is changed; and mixtures of two or more kinds of these adhesives. Adhesive layers constituted of a foamed adhesive and adhesive layers constituted of a crosslinked adhesive can also be employed.

The non-aromatic hot melt based adhesive is not limited so far as it is made of, as a base polymer, a hot melt based adhesive not containing an aromatic ring. Examples thereof include olefin based hot melt based adhesives and acrylic hot melt based adhesives. As the non-aromatic polymer which is the base polymer not containing an aromatic ring, there are enumerated polymers or copolymers of an olefin or a diene. Examples thereof include olefin polymers. The olefin polymer includes polymers or copolymers of ethylene or an α-olefin. Also, polymers resulting from adding a diene (for example, butadiene and isoprene) as other monomer thereto may be employed.

The α-olefin is not limited so far as it is a monomer having a double bond in the terminal thereof. Examples thereof include propylene, butene, heptane, hexene, and octene.

The "aromatic hot melt based adhesive" as referred to herein is a hot melt based adhesive whose base polymer contains an aromatic ring. Examples thereof include styrene based hot melt based adhesives represented by A-B-A type block copolymers.

In the foregoing A-B-A type block copolymers, the A block is a non-elastic polymer block made of a monovinyl substituted aromatic compound A such as styrene and methylstyrene; and the B block is an elastic polymer block made of a conjugated diene such as butadiene and isoprene. Specific examples thereof include a styrene-butadiene-styrene block copolymer (SBS), a styrene-isoprene-styrene block copolymer (SIS), and hydrogenated types thereof (for example, SEBS and SIPS), and mixtures thereof.

As a countermeasure for preventing a lowering of adhesive strength caused due to an increase of water of the non-hydrophilic adhesive layer, an adhesive layer obtained by further compounding a water absorptive polymer in the non-hydrophilic adhesive can be used.

The hydrophilic adhesive which constitutes the hydrophilic adhesive layer is not particularly limited so far as it contains a hydrophilic polymer or a water-soluble polymer as the major component, has adhesion and is hydrophilic as an adhesive. Examples of the constitutional components of the hydrophilic adhesive include hydrophilic polymers (for example, polyacrylic acid), water-soluble polymers (for example, poly(sodium acrylate) and polyvinylpyrrolidone), crosslinking agents (for example, dry aluminum hydroxide and meta-silicic acid aluminic acid metal salts), softeners (for example, glycerin and propylene glycol), higher hydrocarbons (for example, soft liquid paraffin and polybutene), primary alcohol fatty acid esters (for example, isopropyl myristate), silicon-containing compounds (for example, silicone oil), fatty acid glycerin esters (for example monoglycerides), oily components (for example, vegetable oils such as olive oil), antiseptics (for example, methyl p-hydroxybenzoate and propyl p-hydroxybenzoate), solubilizing agents (for example, N-methyl-2-pyrrolidone), thickeners (for example, carboxymethyl cellulose), surfactants (for example, polyoxyethylene hardened castor oil and sorbitan fatty acid esters), hydroxycarboxylic acid (for example, tartaric acid), excipients (for example, light silicic anhydride, water absorptive polymers, and kaolin), moisturizers (for example, D-sorbitol), stabilizers (for example, sodium edetate, p-hydroxybenzoic acid esters, and tartaric acid), crosslinking type water absorptive polymers, boron compounds (for example, boric acid), and water. They may be used as an arbitrary combination.

A temporary adhering seal part is formed via a sticky layer. An adhesive which constitutes the sticky layer is a layer formed of a polymer composition which is tacky at the normal temperature and is not limited so far as it can be heat sealed after temporary adhesion.

Furthermore, the foregoing adhesives of the sticky layer can be used as the adhesive which constitutes the sticky layer as used for temporary adhesion. Of these, non-hydrophilic adhesives are preferable. With respect to the adhesive constituting the adhesive layer, it is preferable that the adhesive is well compatible with a heat seal material constituting a heat seal and that a melting point of the base polymer of the adhesive is not higher than a melting point of the heat seal material. Hot melt based adhesives are especially preferable for hot melt based bonding agents. Furthermore, in the case where the heat seal material is an olefin based raw material, preferred examples thereof include olefin based adhesives.

A bonding layer for fixing the air permeability adjusting material is constituted of a bonding agent or an adhesive which is usually used. In particular, an adhesive is useful, and the foregoing adhesives for constituting the adhesive layer can be used.

Furthermore, a method for providing a bonding layer is not limited so far as the air permeability adjusting material can be fixed. The bonding layer may be entirely provided or partially or intermittently provided. Examples of its shape include various shapes such as a network-like shape, a stripe-like shape, a dot-like shape, and strip-like shape.

Furthermore, in the case where an adhesive layer is employed as the hydrophilic adhesive layer, if there is a difference in a water retaining force between the hydrophilic adhesive layer and the heat generating composition molded body, transfer of water occurs via a packaging material present therebetween such as a substrate, thereby causing in-conveniences against the both. In particular, the transfer of water occurs during the storage. In order to prevent this, it is preferable that the packaging material present therebetween at least has a moisture permeability of not more than 2 $g/m^2/day$ in terms of a moisture permeability according to the Lyssy method. By using this, in the case where the heat generating body is accommodated in an outer bag as an air-impermeable accommodating bag and stored, the transfer of water can be prevented.

In the case where a hydrophilic adhesive layer is used as the adhesive layer, the moisture permeability of a moisture-proof packaging material provided between the heat generating composition molded body and the hydrophilic adhesive layer is not limited so far as the transfer of water can be prevented within the range where the exothermic performance is not affected. The moisture permeability according to the Lyssy method is usually not more than 2 $g/m^2/day$, preferably not more than 1. 0 $g/m^2/day$, more preferably not more than 0.5 $g/m^2/day$, and further preferably from 0.01 to 0.5 $g/m^2/day$. These values are a value under a condition under an atmospheric pressure at 40 °C and 90 % RH. Incidentally, the moisture-proof packaging material can be used as a substrate or a covering material and may be laminated singly on a substrate, a covering material, or the like.

The moisture-proof packaging material is not limited so far as the transfer of water between the heat generating composition molded body and the hydrophilic adhesive layer can be prevented. Examples thereof include metal vapor deposited films, vapor deposited films of a metal oxide, metal foil-laminated films, EVOH (ethylene/vinyl alcohol copolymer or ethylene/vinyl acetate copolymer saponified product) based films, biaxially stretched polyvinyl alcohol films, polyvinylidene chloride coated films, polyvinylidene chloride coated films obtained by coating polyvinylidene chloride on a substrate film (for example, polypropylene), metal foils such as an aluminum foil, air-impermeable packaging materials obtained by vapor depositing or sputtering a metal (for example, aluminum) on a polyester film substrate, and packaging laminates using a transparent barrier film of a structure in which silicon oxide or aluminum oxide is provided on a flexible plastic substrate. The air-impermeable packaging materials which are used in the outer bag, etc. can also be used.

Furthermore, packaging materials such as moisture-proof packaging materials as described in JP-A-2002-200108, the disclosures of which can be incorporated herein by reference, can be used.

In the case of using a water-containing hydrophilic adhesive (for example, a gel) in the adhesive layer, in order to adjust the moisture equilibrium between the heat generating composition and the adhesive layer, the content of a reaction accelerator (for example, sodium chloride) or a substance having a water holding power (for example, a water absorptive polymer) in the heat generating composition may be adjusted within the range of from 10 to 40 % by weight, preferably from 15 to 40 % by weight, and more preferably from 15 to 30 % by weight based on the heat generating composition.

Furthermore, as the adhesive having good moisture permeability and low stimulation to the skin, water-containing adhesives (for example, hydrophilic adhesives and gels) as described in JP-A-10-265373 and JP-A-9-87173, adhesives which can be subjected to hot melt coating as described in JP-A-6-145050 and JP-A-6-199660, and rubber based adhesives as described JP-A-10-279466 and JP-A-10-182408, the disclosures of which are totally incorporated herein by reference, are useful.

The functional substance which is contained in the adhesive layer is not limited so far as it is a substance having any function. There can be enumerated at least one member selected from aromatic compounds, vegetable extracts, crude drugs, perfumes, slimming agents, analgesics, blood circulation promoters, swelling improvers, antibacterial agents, sterilizers, mold inhibitors, odor eaters, deodorants, percutaneously absorptive drugs, fat-splitting components, minus ion generators, far infrared ray radiants, magnetic bodies, fomentations, cosmetics, bamboo vinegar, and wood vinegar.

Specific examples thereof include aromatic compounds (for example, menthol and benzaldehyde), vegetable extracts (for example, mugwort extract), crude drugs (for example, moxa), perfumes (for example, lavender and rosemary), slimming agents (for example, aminophylline and tea extract), analgesic drugs (for example, indomethacin and *dl*-camphor), blood circulation promoters (for example, acidic mucopolysaccharide and chamomile), swelling improvers (for example, horse chestnut extract and flavone derivatives), fomentations (for example, aqueous boric acid, physiological saline, and aqueous alcohols), fat-splitting components (for example, jujube extract, caffeine, and tonalin), cosmetics (for example, aloe extracts, vitamin preparations, hormone preparations, anti-histamines, and amino acids), antibacterial agents and sterilizers (for example, carbolic acid derivatives, boric acid, iodine preparations, invert soaps, salicylic acid based substances, sulfur, and antibiotics), and mold inhibitors.

The percutaneously absorptive drug is not particularly limited so far as it has percutaneous absorption. Examples thereof include corticosteroids, anti-inflammatory drugs, hypertension drugs, anesthetics, hypnotic sedatives, tranquillizers, antibacterial substances, antifungal substances, skin stimulants, inflammation inhibitors, anti-epileptics, analgesics, antipyretics, anesthetics, mold inhibitors, antimicrobial antibiotics, vitamins, antiviral agents, swelling improvers, diuretics, antihypertensives, coronary vasodilators, anti-tussive expectorants, slimming agents, anti-histamines, antiarrhythmic agents, cardiotonics, adrenocortical hormones, blood circulation promoters, local anesthetics, fat-splitting components, and mixtures thereof. However, it should not be construed that the invention is limited thereto. These drugs are used singly or in admixture of two or more kinds thereof as the need arises.

The content of such a functional substance is not particularly limited so far as it falls within the range where the effect of a medicine can be expected. However, from the viewpoints of adhesive strength as well as pharmacological effect and economy, the content of the functional substance is preferably from 0.01 to 25 parts by weight, and more preferably from 0.5 to 15 parts by weight based on 100 parts by weight of the adhesive.

Furthermore, a method for providing the adhesive layer is not limited so far as a thermal packaging body for joint surroundings can be fixed. The adhesive layer may be entirely provided or partially or intermittently provided. Examples of its shape include various shapes such as a network-like shape, a stripe-like shape, a dot-like shape, and strip-like shape.

As the fixing measure, a non-slip layer may be provided. The non-slip layer is not limited so far as it is able to prevent divergence or falling off of the flexible heat generating body due to the friction, thereby always tightly fixing the flexible heat generating body in a required place. Examples thereof include a non-slip agent layer constituted of a non-slip agent.

With respect to the non-slip layer having permeability to air according to the invention, after providing a non-slip agent on the entire surface on the flexibility holding member, plural bores may be provided, thereby keeping the permeability to air; a porous resin formed by fibrillating a non-slip agent may be provided on the flexibility holding member; a porous weakly sticky layer on a separator may be transferred and fixed onto the surface of the flexibility holding member; or a non-slip agent may be provided directly on the surface of the flexibility holding member in a cobweb state by a melt blow method, etc. Furthermore, by printing or uniformly dispersing a granular weakly sticky substance or a soft vinyl chloride resin and fixing upon heating, a number of protrusions may be formed. Furthermore, a non-slip agent resulting from spraying a solution or dispersion of an elastomer or a plastisol in a stripped form or polka-dotted form can be used. Furthermore, the non-slip layer may be formed by sticking a film having friction or the like. The non-slip layer may be provided entirely or partially on the flexibility holding member. In addition, the adhesiveness between the non-slip agent and the flexibility holding member may be improved by applying heat or pressure by using a roll, etc. Furthermore, as the non-slip agent, all of hot melt bases, solvent bases and emulsions can be used. Furthermore, the non-slip layer may be provided in at least a part of the flexible exothermic part.

A coefficient of friction of the non-slip layer is preferably 20° or more, more preferably from 20 to 80°, further preferably from 30 to 70°, and still further preferably from 40 to 60°. When the coefficient of friction is less than 20°, the duty as the non-slip layer is not fulfilled. When it exceeds 80°, the duty as the non-slip layer is thoroughly fulfilled, and it can be used for non-slip of the flexibility holding member or between the flexible exothermic parts. However, in the case where it is used so as to come into contact with the skin, the adhesiveness to the skin is too well so that when the flexible heat generating body is peeled away or the body is moved, unwell feelings such as pains are accompanied therewith. The moisture permeability is preferably not more than 1,000 seconds/100 cc, more preferably not more than 500 seconds/100 cc, and further preferably not more than 100 seconds/100 cc according to the Gurley method. When the moisture permeability exceeds 1,000 seconds/100 cc, an unwell feeling such as sweat becomes strong.

The coefficient of fraction was measured by using a slip inclination analyzer in a method according to the inclination method as defined in JIS P8147. Here, the slip inclination analyzer has an inclination plate as installed by a hinge such that it is parallel to a fixed table having a level installed thereon. A sample (width: 100 mm, length: 200 mm) was fixed to the inclination plate; a weight (width: 60 mm, length: 100 mm, weight: 400 g) was placed on an upper end of the sample; an inclination angle was increased at a rate of 1° per second; and when the weight started to slip, an inclination angle was read out. In a gather-provided substrate, it was fixed in a fully extended state, and its coefficient of friction was measured.

As the non-slip agent, all of solvent bases, hot melt bases and emulsions can be used. Weakly sticky substances are especially preferable, and hot melt bases and emulsion bases thereof are preferable.

Examples of the weakly sticky substance which can be preferably used include styrene based elastomers such as SIS, SBS, SEBS, and SIPS; acrylic elastomers containing, as a component, an acrylic acid based or methacrylic acid based alkyl ester; olefin based elastomers such as polyethylene, super low density polyethylene, polypropylene, and ethylene-vinyl acetate copolymers; and urethane based elastomers. Examples of other non-slip agents include mixtures of a capsule containing an expanding agent (a volatile solvent such as isobutane) in a core of a resin such as a soft vinyl chloride based resin, a natural rubber, a styrene-butadiene rubber, an isoprene rubber, a chloroprene rubber, a urethane rubber, an ethylene-vinyl acetate copolymer, a hydrolyzate of an ethylene-vinyl acetate copolymer, an ethylene-acrylic acid copolymer, an ethylene-acrylic ester copolymer, a polyamide resin, a hot melt resin such as polyester resins, an ethylene-methyl methacrylate copolymer (EMMA) whose tackiness is weakened, a polyurethane resin composition which is an expandable resin composition containing, as an expanding agent, water or a low boiling neutral liquid, and an acrylic resin (for example, thermosetting acrylic resins such as vinylidene chloride-acrylonitrile copolymers) with a binder resin.

These substances may be used singly or as a blend of two or more kinds thereof or may be used as an aqueous emulsion. In addition, the following may be employed.

1) A solution or dispersion of a resin or an elastomer, or a plastisol is uniformly coated on the surface of the flexibility holding member and dried, followed by opening plural bores. Alternatively, the solution or dispersion or plastisol is coated in a linear or pattern-like state and dried.

In the following, in the case of uniformly coating, etc., bores are ultimately opened, thereby ensuring permeability to air.

2) A resin material of a hot melt type is coated uniformly or in a pattern-like state on the surface of the flexibility holding member by a hot melt coating method.

3) A heat fusible resin is extruded on the surface of the flexibility holding member to form a laminate.

4) An expandable resin composition is coated or impregnated uniformly or in a pattern-like state on the surface of a flexibility holding member and then expanded, thereby forming an irregular surface due to the expanded layer of the resin.

5) A synthetic resin film with friction is laminated on the surface of the flexibility holding member. Incidentally, in order to coat the respective resin material in a pattern-like state, a gravure coater or the like can be used. Furthermore, in the case of hot melt resins or soft vinyl chloride resins or the like, by uniformly spraying a granular substance on the surface of the flexibility holding member and fixing upon heating, a number of protrusions can be formed on the surface of the flexibility holding member.

Furthermore, it may be arbitrarily achieved to provide characters, patterns, etc. in a desired position of the flexible heat generating body by printing or the like, to color a part or the whole thereof, or to stick a material having a different color thereto.

The heat generating body of the invention is able to give various shapes, thicknesses and temperature zones and therefore, can be used for various utilities such as use for a joint, facial esthetic use, use for eyes, slimming use, use for heating or warming a dripping solution, use for a wet compress pack, use for a medical body warmer, use for a neck, use for a waist, use for a mask, use for a glove, use for hemorrhage, use for relaxation of symptoms such as shoulder pain, muscular pain, and menstrual pain, use for a cushion, use for heating or warming a human body during the operation, use for a thermal sheet, use for thermally volatilizing an aroma, use for an abdomen, insecticidal use by thermal volatilization, and use for treating cancer in addition to common warming of a human body. In addition, the heat generating body of the invention can be used for heating or warming machines, pets, etc.

**[0111]** [0109] For example, in the case of using for relaxation of symptoms, the heat generating body of the invention is applied directly in a necessary site of the body or indirectly via a cloth, etc. Furthermore, in the case of using for heating or warming a human body during the operation, a method for using the heat generating body of the invention includes the following methods.

(1) The heat generating body is directly applied to a body requiring heating or warming.
(2) The heat generating body is fixed on a covering, etc. and covered on the body.
(3) The heat generating body is fixed on a cushion to be placed beneath the body, etc.
(4) The heat generating body is used as a covering or a cushion which is a product having the heat generating body provided therein in advance.

Incidentally, examples of the pain of muscles or bones include acute muscle pain, acute bone pain, acute reference pain, previous muscle pain, previous bone pain, chronic reference pain, and join pain of knee, elbow, etc.

The holding time is not limited but is preferably from 20 seconds to 24 hours, more preferably from one hour to 24 hours, and further preferably from 8 hours to 24 hours.

The holding temperature is preferably from 30 to 50 °C, more preferably from 32 to 50 °C, further preferably from 32 to 43 °C, still further preferably from 32 to 41 °C, and even further preferably from 32 to 39 °C.

The invention will be described below in more detail with reference to the Examples, but it should not be construed that the invention is limited thereto.

[Brief Description of the Drawings]

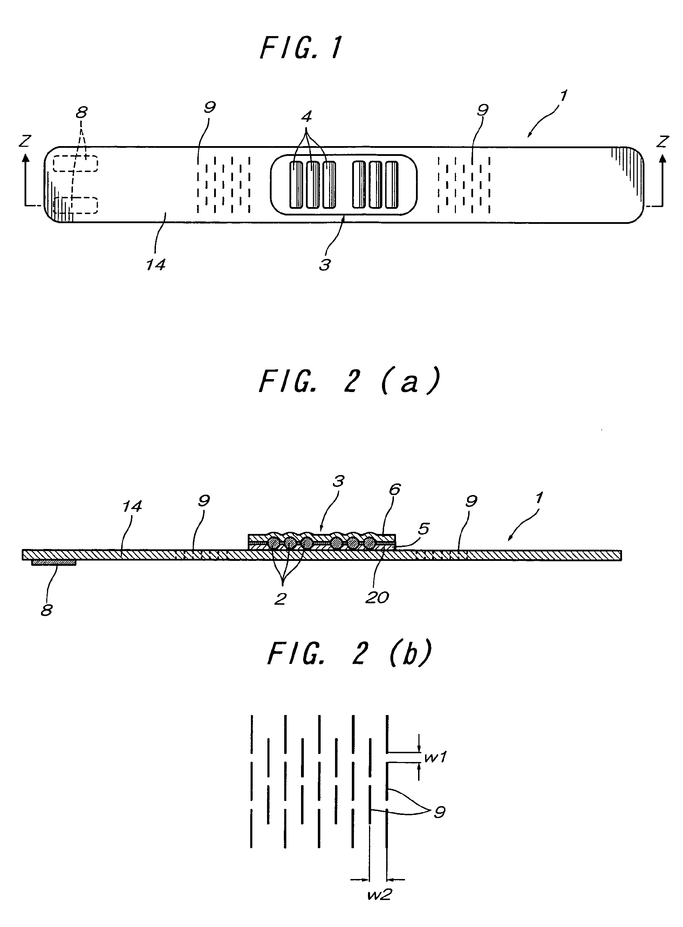

Fig. 1 is a plan view of an embodiment of the flexible heat generating body of the invention.
Fig. 2(a) is a cross-sectional view along the line Z-Z of the same; and Fig. 2(b) is an explanatory view to show a notch part of the same.

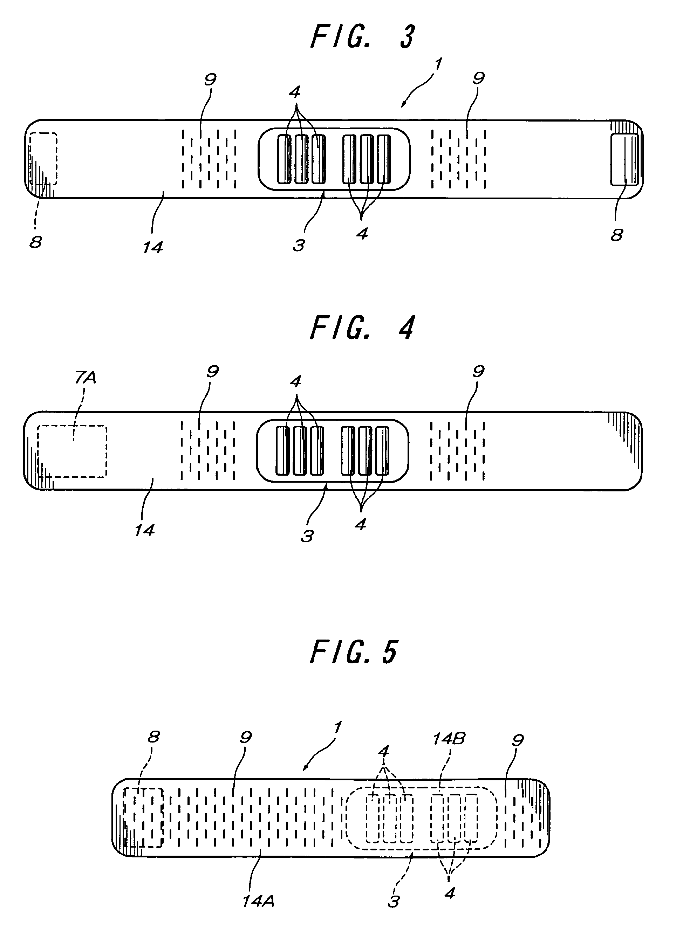

Fig. 3 is a plan view of other embodiment of the flexible heat generating body of the invention.
Fig. 4 is a plan view of other embodiment of the flexible heat generating body of the invention.
Fig. 5 is a plan view of other embodiment of the flexible heat generating body of the invention.

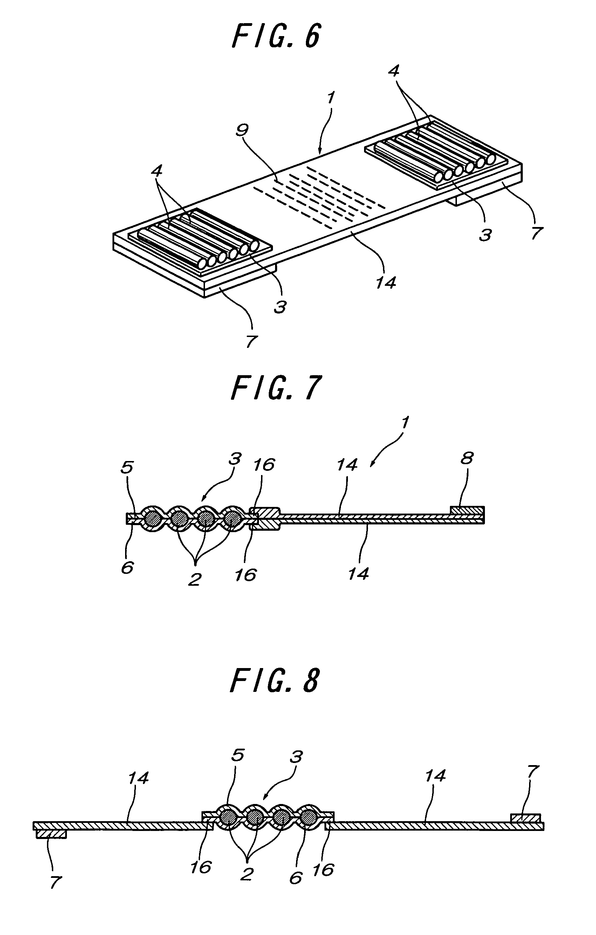

Fig. 6 is an oblique view of other embodiment of the flexible heat generating body of the invention.
Fig. 7 is a cross-sectional view of other embodiment of the flexible heat generating body of the invention.
Fig. 8 is a cross-sectional view of other embodiment of the flexible heat generating body of the invention.

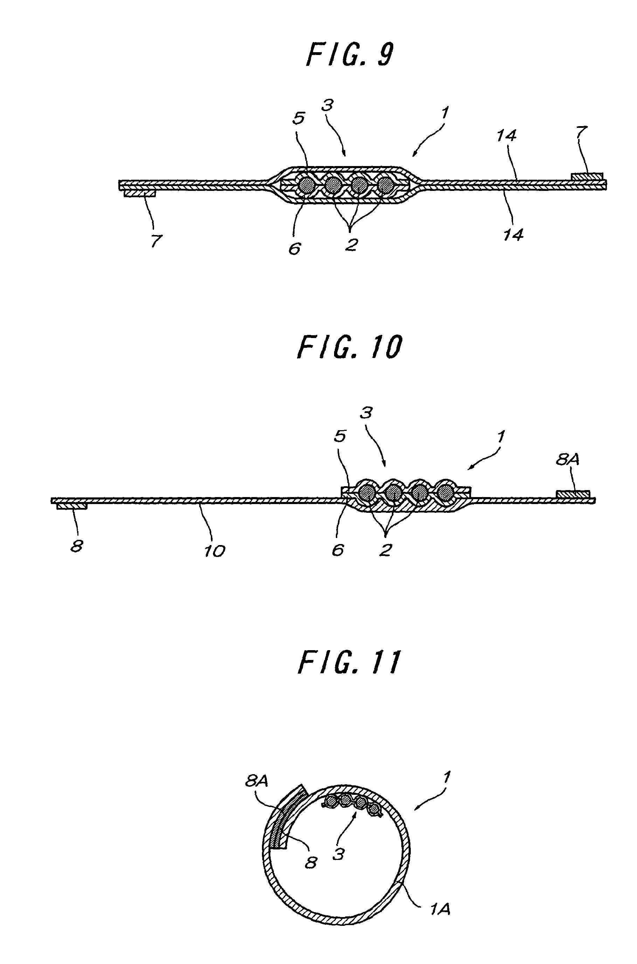

Fig. 9 is a cross-sectional view of other embodiment of the flexible heat generating body of the invention.

Fig. 10 is a cross-sectional view of other embodiment of the flexible heat generating body of the invention.

Fig. 11 is an explanatory view to show the state of use of the same.

Fig. 12 (a) and Fig. 12 (b) are each a cross-sectional view of other embodiment of the flexible heat generating body of the invention.

Fig. 13 is an explanatory view to show the state of use of the same.

Fig. 14 is a cross-sectional view of other embodiment of the flexible heat generating body of the invention.

Fig. 15 is a cross-sectional view of other embodiment of the flexible heat generating body of the invention.

Fig. 16 is a cross-sectional view of other embodiment of the flexible heat generating body of the invention.

Fig. 17 is a cross-sectional view of other embodiment of the flexible heat generating body of the invention.

Fig. 18 is a cross-sectional view of other embodiment of the flexible heat generating body of the invention.

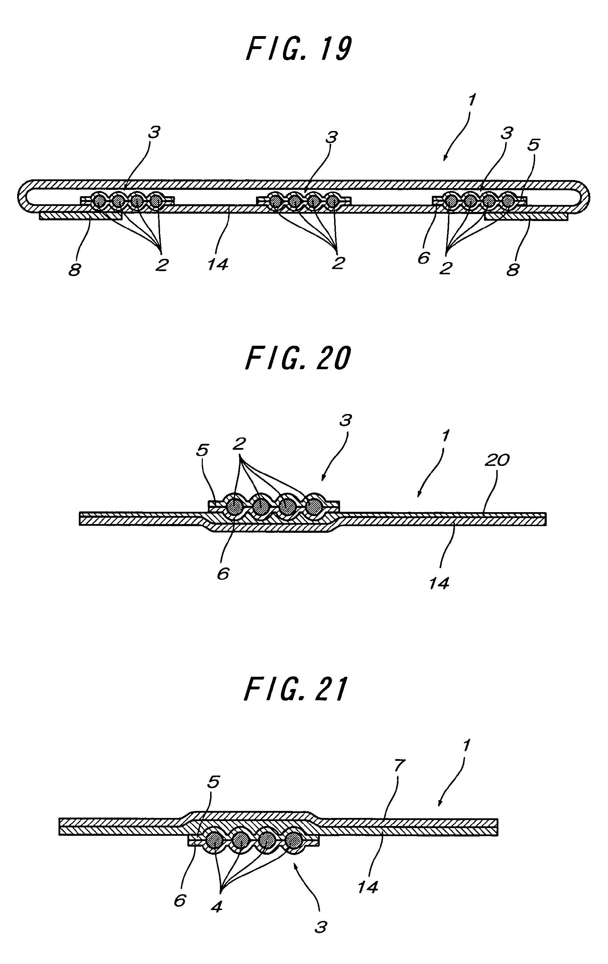

Fig. 19 is a cross-sectional view of other embodiment of the flexible heat generating body of the invention.

Fig. 20 is a cross-sectional view of other embodiment of the flexible heat generating body of the invention.

Fig. 21 is a cross-sectional view of other embodiment of the flexible heat generating body of the invention.

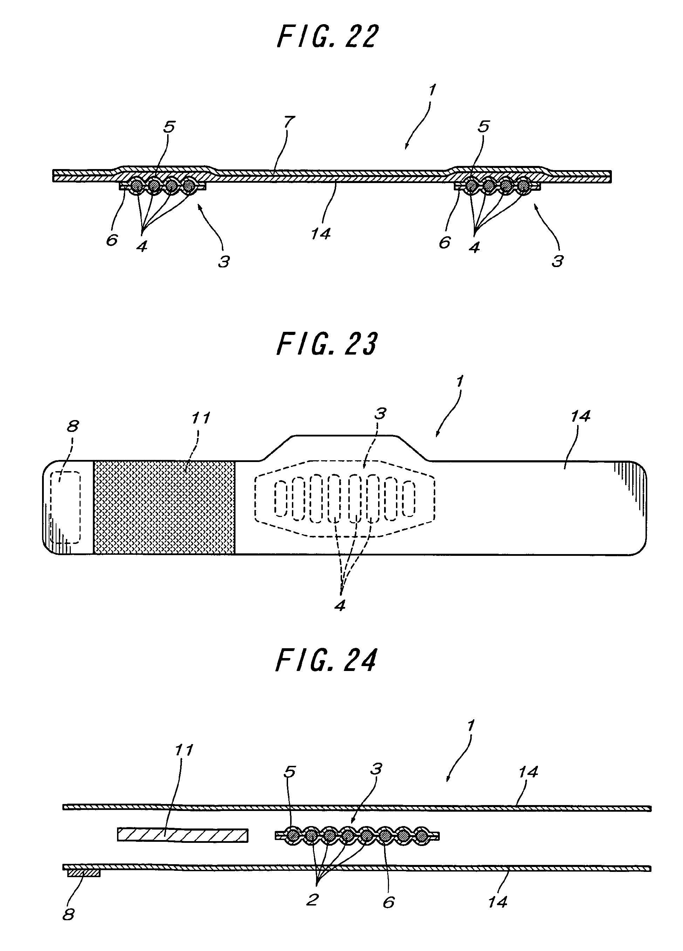

Fig. 22 is a cross-sectional view of other embodiment of the flexible heat generating body of the invention.

Fig. 23 is a plan view of other embodiment of the flexible heat generating body of the invention.

Fig. 24 is an exploded view of the same.

Fig. 25 is an oblique view for explaining a mesh.

Fig. 26 is a plan view of other embodiment of the flexible heat generating body of the invention.

Fig. 27 is an explanatory view of modifications of a notch part.

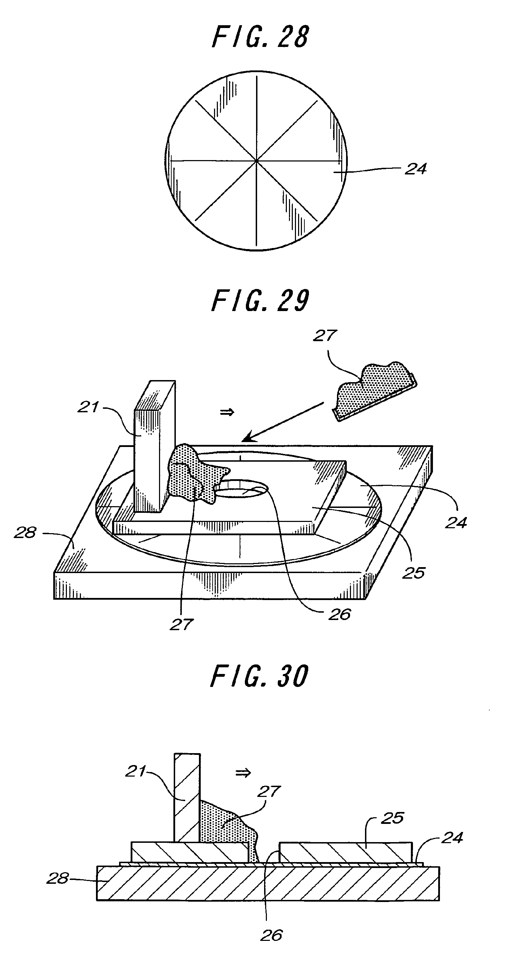

Fig. 28 is a plan view of a filter paper for the measurement of water mobility value in the invention.

Fig. 29 is an oblique view for carrying out the measurement of water mobility value in the invention.

Fig. 30 is a cross-sectional view of the same.

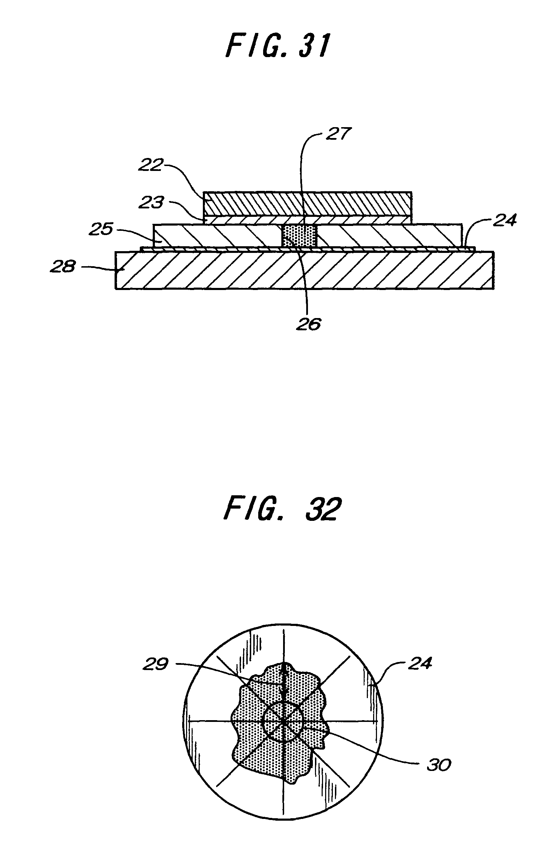

Fig. 31 is a cross-sectional view of the same.

Fig. 32 is a plan view of a filter paper after carrying out the measurement of water mobility value in the invention.

[Description of Reference Numerals and Signs]

1: Flexible heat generating body

3: Flexible exothermic part

4: Heat generating composition molded body

7: Adhesive layer

8: MAGIC TAPE (registered trademark)

9: Notch part

14: Flexibility holding member

15: Body joint part

21: Leveling plate

22: Flat plate

23: Non-water absorptive film (for example, a polyethylene film)

24: Filter paper in which eight lines are drawn radiating from the center point with an interval of 45°

25: Template

26: Hole

27: Sample

28: Stainless steel plate

29: Distance to the oozed-out locus of water or aqueous solution

30: Position corresponding to a hollow cylindrical hole on filter paper

[Examples]

(Example 1)

This Example will be described with reference to Fig. 1 and Fig. 2. Fig. 1 is a plan view of a flexible heat generating body 1; and Fig. 2 (a) is a cross-sectional view along the line Z-Z of the same, and Fig. 2(b) is a plan view of a notch part 9.

A heat generating composition having a water mobility value of 8, which is a mixture consisting of 100 parts by weight of a reduced iron powder (particle size: not more than 300 $\mu$m), 7.0 parts by weight of active carbon (particle size: not more than 300 $\mu$m), 2.1 parts by weight of a wood meal (particle size: not more than 300 $\mu$m), 2. 0 parts by weight of a water absorptive polymer (particle size: not more than 300 $\mu$m), 0.2 parts by weight of calcium hydroxide, 0.7 parts by weight of sodium sulfite and 11 % of salt water, was used as the heat generating composition.

Next, the heat generating composition was molded by using a trimming die provided with three cavities in a striped form at intervals in each of the right and left sides thereof. Incidentally, the cavities were provided at intervals of 10 mm only in the central part and at intervals of 5 mm in other part, respectively. Also, the dimensions of the cavities were 9 mm in width x 80 mm in length.

With respect to the heat generating composition as molded by the trimming die, as shown in Fig. 2, six heat generating composition molded bodies 2 each constituting a sectional exothermic part 4 were provided on a substrate 5 made of a polyethylene film on which a non-woven fabric had been laminated; next, an air-permeable covering material 6 having a nylon-made non-woven fabric with a basis weight of 40 g/m$^2$ laminated on a polyethylene-made porous film was put thereon; and the periphery of each of the heat generating composition molded bodies 2 and the outer surroundings as a flexible exothermic part 3 were sealed. A seal part of the periphery of each of the heat generating composition molded bodies 2 was heat sealed in a seal width of 3 mm. Also, the outer surroundings of the exothermic part 3 were sealed in a seal width of 8 mm.

Incidentally, a loop side of a hook and loop fastener was provided on either surface of the flexible exothermic part 3. Also, in the flexible exothermic part 3, the air permeability of the covering material 6 was 400 g/m$^2$/24 hr in terms of a moisture permeability by the Lyssy method.

Also, the bending resistance of the flexible exothermic part 3 was 30 mm in the longitudinal direction and 80 mm or more in the short side direction (stripe direction). The respective values were a minimum value and a maximum value of the bending resistance of the flexible exothermic part 3. Also, the bending resistance ratio was 2 or more.

Next, in a non-extensible flexibility holding member 14 made of an air-permeable non-woven fabric of 100 cm in length x 15 cm in width, notch parts 9 were provided within the range of 50 mm in width in the longitudinal direction in the vicinity of either side of a site to which the flexible exothermic part 3 was to be stuck. The notch parts 9 were provided in a length of 10 mm in the orthogonal direction (vertical direction to the stretching direction) at intervals of 2 mm (w1) in the longitudinal direction and alternately disposed in a width of 10 mm (w2) in the stretching direction. Furthermore, the notch parts 9 were formed penetrating in the thickness direction of the flexibility holding member 14.

A maximum degree of extension (in the central part) in a region where the notch parts 9 of the resulting stretchable flexibility holding member 14 were provided (one of two regions where the notch parts 9 were provided) was 1.3 times. The flexible exothermic part 3 was stuck via a sticky layer 20 made of an adhesive between the two regions where the notch parts 9 were provided such that the air-permeable surface of the flexible exothermic part 3 was faced externally.

Next, a hook and loop fastener (having a hook function) 8 as a fixing measure was provided via a sticky layer on the surface in the opposite side to the flexible exothermic part 3 in one end side of the longitudinal direction of the flexibility holding member 14. Incidentally, the flexibility holding member 14 had a female function of a hook and loop fastener on the both surfaces.

The thus obtained flexible heat generating body 1 had a very low bending resistance as 30 mm in the longitudinal direction so that it was very excellent in handling and a feeling for use.

Next, the flexible heat generating body 1 was sealed and accommodated in an air-impermeable accommodating bag (hereinafter referred to as "outer bag") and allowed to stand at room temperature for 24 hours. After 24 hours, the flexible heat generating body 1 was taken out from the outer bag, wound around the body such that the flexible exothermic part 3 came into contact with the waist and then fixed by the hook and loop fastener 8.

As a result, it was felt warm within 3 minutes, and comfortable warmth was continued for 7 hours. During the use, the flexible heat generating body was free from slack of the flexibility holding member 14 and coming off of the flexible heat generating body and was superior in all of curved surface fitness, winding properties and usefulness.

(Example 2)

An example of preparing a flexible heat generating body 1 by using a batchwise stirrer-equipped oxidizing gas contact treatment device composed of a mixer equipped with a rotary blade for stirring as an oxidizing gas contact treatment device will be described. Incidentally, air was used as an oxidizing gas.

A reaction mixture consisting of 100 parts by weight of an iron powder (particle size: not more than 300 $\mu$m), 5. 5 parts by weight of active carbon (particle size: not more than 300 $\mu$m), 2.3 parts by weight of a wood meal (particle size: not more than 300 $\mu$m), 2.3 parts by weight of a water absorptive polymer (particle size: not more than 300 $\mu$m), 0.5 parts by weight of calcium hydroxide, 0.7 parts by weight of sodium sulfite and 5 parts by weight of 11 % salt water and having a water mobility value of less than 0.01 was charged in the batchwise stirrer-equipped oxidizing gas contact treatment device.

Next, the upper portion of the contact treatment device vessel was opened to air, and the reaction mixture was subjected to self heat generation with stirring in the opened state to air under circumstances at 20°C. When the temperature rise of the reaction mixture reached 40°C, the reaction mixture was sealed in an air-impermeable accommodating bag and cooled to room temperature to obtain a heat generating mixture. 11 % salt water was mixed in the heat generating mixture to obtain a heat generating composition having a water mobility value of 12. This heat generating composition was stuck to a flexibility holding member 14 provided with notch parts 9 in the same manner as in Example 1, thereby obtaining a flexible heat generating body 1 provided with a hook and loop fastener 8 in either end part as shown in Fig. 3. This flexible heat generating body had functions substantially the same as in the flexible heat generating body 1 of Example 1 and was superior in both exothermic performance and usefulness.

(Example 3)

**[0117]** [0110] A batchwise stirring tank composed of a mixer equipped with a rotary blade in a blade form of a ventilation fan was used as an oxidizing gas contact treatment device, and air was used as an oxidizing gas. First of all, a reaction mixture consisting of 100 parts by weight of a reduced iron powder (particle size: not more than 300 $\mu$m) and 5 parts by weight of 11 % salt water and having a water mobility value of less than 0.01 was charged in the contact treatment device vessel.

Next, the upper portion of the contact treatment device vessel was opened to air, and the reaction mixture was subjected to self heat generation with stirring in the opened state to air under circumstances at 20°C. When the temperature rise of the reaction mixture reached 10°C, the reaction mixture was sealed in an air-impermeable accommodating bag and cooled to room temperature to obtain a heat generating mixture. The heat generating mixture was mixed with 5. 3 parts by weight of active carbon (particle size: not more than 300 $\mu$m), 5 parts by weight of a wood meal (particle size: not more than 300 $\mu$m), 1.2 parts by weight of a water absorptive polymer (particle size: not more than 300 $\mu$m), 0.2 parts by weight of calcium hydroxide, 0.7 parts by weight of sodium sulfite and 11 % salt water to obtain a heat generating composition having a water mobility value of 8.

By using this heat generating composition, a flexible heat generating body provided with a non-slip layer 7A as shown in Fig. 4 was obtained. This flexible heat generating body was subjected to a warmth taking test of back of the neck as a thermal muffler. As a result, the vicinity of back of the neck was warmed and felt pleasant. Furthermore, this flexible heat generating body was superior in both exothermic performance and usefulness.

(Example 4)

A batchwise stirrer-equipped oxidizing gas contact treatment device composed of a mixer equipped with a rotary blade for stirring was used as an oxidizing gas contact treatment device, and air was used as an oxidizing gas. A mixture consisting of 100 parts by weight of an iron powder (particle size: not more than 300 $\mu$m) and 3.2 parts by weight of active carbon (particle size: not more than 300 $\mu$m) was charged in the batchwise stirrer-equipped oxidizing gas contact treatment device. Next, 5 parts by weight of 11 % salt water (corresponding to a water mobility value of less than 0.01) was added thereto while stirring the mixture, and the reaction mixture was subjected to self heat generation with stirring in the opened state to air under circumstances at 20°C.

After 20 seconds, when the temperature rise of the reaction mixture reached 20°C, the reaction mixture was sealed in an air-impermeable accommodating bag and cooled to room temperature to obtain a heat generating mixture.

The heat generating mixture was mixed with 5.3 parts by weight, 5 parts by weight of a wood meal (particle size: not more than 300 $\mu$m), 1.2 parts by weight of a water absorptive polymer (particle size: not more than 300 $\mu$m), 0.2 parts by weight of calcium hydroxide, 0.7 parts by weight of sodium sulfite and 11 % salt water to obtain a heat generating composition having a water mobility value of 8.

Next, a flexible heat generating body as shown in Fig. 5 was obtained in the same manner as in Example 1, except that a longitudinal flexibility holding member 14A and a short flexibility holding member 14B were provided while sandwiching a flexible exothermic part 3 therebetween and that a hook and loop fastener 8 was provided in the vicinity of an end part of the longitudinal flexibility holding member 14A on the surface on which the flexible exothermic part 3 was not provided. This flexible heat generating body had functions substantially the same as in the flexible heat generating body of Example 1 and was superior in both exothermic performance and usefulness.

(Example 5)

Fig. 6 shows a plan view of a flexible heat generating body 1 in which flexible exothermic parts 3, 3 are each provided via a sticky layer in the vicinity of either end part of a flexibility holding member 14 and plural notch parts 9 penetrating in the thickness direction are alternately disposed in the vicinity of the central part thereof.

(Example 6)

By using a heat generating composition the same as the heat generating composition of Example 1, a flexible heat generating body 1 was prepared in the same manner as in Example 3, except for changing the reduced iron powder of Example 1 to an iron powder (particle size: not more than 300 $\mu$m) containing 0.2 % by weight of a carbon component resulting from a coating treatment of sponge iron with 1 % by weight of active carbon.

In this Example, a flexibility holding member 14 as prepared by using expanded polyurethane (dimensions: 150 mm in width, 100 cm in length, 1.0 mm in width) and forming linear notch parts having a length of 8 mm on the orthogonal direction to the longitudinal direction (vertical direction to the stretching direction) in the vicinity of either end of a region where a flexible exothermic part 3 was to be stuck in a cut width of 2 mm and a feed width of 5.5 mm in the stretching direction was used. This flexibility holding member had a rate of extension of 3.0 times, a tensile strength of 6.5 N/25 mm and a peel strength of 3.0 N/25 mm.

The flexible heat generating body 1 was sealed and accommodated in an air-impermeable accommodating bag (hereinafter referred to as "outer bag") and allowed to stand at room temperature for 24 hours. After 24 hours, the flexible heat generating body 1 was taken out from the outer bag, wound around the body such that the exothermic part came into contact with a waist, and a hook and loop fastener was installed in the flexibility holding member 14 while drawing. As a result, it was felt warm within 3 minutes, and comfortable warmth was continued for 7 hours. During the use, the flexible heat generating body was free from slack of the flexibility holding member 14 and coming off of the flexible heat generating body. As a result of evaluation with respect to curved surface fitness, winding properties and usefulness, the flexible heat generating body was supper in all of these evaluations.

(Example 7)

In Fig. 7 to Fig. 22, any one of a stretchable elastomer-containing film, an elastomer-containing expanded body, an elastomer-containing non-woven fabric or woven fabric, or a stretchable non-woven fabric or fabric was used as a flexibility holding member 14.

In Fig. 7, two sheets of a flexibility holding member 14 are bonded to each other, and a flexible exothermic part 3 is sandwiched by one end 16 thereof, whereas a hook and loop fastener 8 is provided in the other end.

(Example 8)

In a flexible heat generating body as shown in Fig. 8, a substrate 6 and a covering material 5 of a flexible exothermic part 1 are each constituted of a stretchable material, and both end parts 16, 16 thereof are provided such that adhesive layers 7 are installed on the surface opposite to each other in a flexibility holding member 14 made of a stretchable material.

(Example 9)

In a flexible heat generating body as shown in Fig. 9, a flexible exothermic part 3 is wrapped in the central part between flexibility holding members 14, 14 made of a stretchable material, and adhesive layers 7 are provided in either end part such that they are provided in the opposite side to each other.

(Example 10)

A flexible heat generating body as shown in Fig. 10 is an example in which a flexible exothermic part 3 is provided near one end of a flexibility holding member 14 made of a stretchable material and hook and loop fasteners 8, 8A (each having either one of a hook function and a loop function) are provided in either end part of the same material such that they are provided in the opposite side to each other.

This flexible heat generating body 1 is wound around an arbitrary place such as feet and arms and fixed by the hook and loop fasteners 8, 8A.

(Example 11)

Fig. 12 (a) is an example in which a flexible exothermic part 3 is provided in either end part of a flexibility holding member 14 made of a stretchable material and an adhesive layer 7 is provided on the surface of this flexible exothermic part 3.

Fig. 12(b) is an example in which the adhesive layer 7 is provided on the surface of the flexibility holding member 14 made of a stretchable material opposite to the region where the flexible exothermic part 3 is provided.

A flexible heat generating body as shown in Fig. 13 is an example in which it is put on a bending and stretching part 15 such as feet and arms and bonded to the skin by an adhesive layer 7.

(Example 12)

In a flexible heat generating body as shown in Fig. 14, a flexible exothermic part 3 is provided on one surface of either end part of a flexibility holding member 14 made of a stretchable material; a flexible exothermic part 3 is provided in the central part of the surface in the opposite side thereof; and an adhesive layer 7 is provided on the opposite surface to the flexible exothermic part 3 of either end of the flexibility holding member 14.

(Example 13)

A flexible heat generating body as shown in Fig. 15 is an example in which a hook and loop fastener (having a hook function) 8 is installed in either end of a flexibility holding member 14 made of a stretchable material and a hook and loop fastener (having a loop function) 8A as a counterpart surface of the hook and loop fastener (having a hook function) 8 is installed in a flexible exothermic part 3.

(Example 14)

A flexible heat generating body as shown in Fig. 16 is an example in which two flexible exothermic parts 3, 3 are provided in either end of a flexibility holding member 14.

(Example 15)

A flexible heat generating body as shown in Fig. 17 is an example in which two flexibility holding members 14, 14 made of a stretchable material are bonded; a part of each of flexible exothermic parts 3, 3 is sandwiched and fixed in either end part thereof; and an adhesive layer 7 is provided on one of the surfaces of the flexible exothermic parts 3, 3.

(Example 16)

A flexible heat generating body as shown in Fig. 18 is an example in which a flexible exothermic part 3 is wrapped in either end part of a flexibility holding member 14 made of a stretchable material.

(Example 17)

A flexible heat generating body as shown in Fig. 19 is an example in which plural flexible exothermic parts 3, 3, 3 are charged and fixed in a flexibility holding member 14 made of a bag-like stretchable material and a hook and loop fastener 8 is provided on the external surface.

(Example 18)

A flexible heat generating body as shown in Fig. 20 is an example in which a sticky layer 20 made of an adhesive is entirely provided on the back surface of a flexibility holding member 14 made of a stretchable material and a flexible exothermic part 3 is installed in the central part of the sticky layer 20.

(Example 19)

A flexible heat generating body as shown in Fig. 21 is an example in which an adhesive layer 7 is provided on the back surface of a flexibility holding member 14 made of a stretchable material and a flexible exothermic part 3 is provided in the central part of the flexibility holding member 14 made of a stretchable material.

(Example 20)

A flexible heat generating body as shown in Fig. 22 is an example in which an adhesive layer 7 is provided on the back surface of a flexibility holding member 14 made of a stretchable material and a flexible exothermic part 3 is provided on the surface of the flexibility holding member 14 made of a stretchable material.

(Example 21)

A flexible heat generating body as shown in Fig. 23 and Fig. 24 is a stretchable flexible heat generating body 1 in which a polymer-made mesh 11 and a flexible exothermic part 3 having eight sectional exothermic parts 2 in a striped form are coupled and fixed while sandwiching by two sheets of a flexibility holding member 12 and a hook and loop fastener 8 having a hook function is provided in one end part thereof. The flexible exothermic part 3 is disposed in the central part, and the mesh 11 is disposed in one side thereof. The flexibility holding member 14 is made of a non-woven fabric, and the surface thereof has a loop function of a hook and loop fastener 8A.

Furthermore, the mesh 11 has plural stretchable second strands 11A and plural first strands 11B intersecting therewith and is integrally coupled with the two flexibility holding members 14, 14 as shown in Fig. 25. The mesh 11 has stretchability and can extend in the longitudinal direction of the flexibility holding member 14.

(Example 22)

A flexible heat generating body as shown in Fig. 26 is an example of a flexible heat generating body 1 in which a flexible exothermic part 3 is provided near one end thereof in the same manner as in Example 21.

(Example 23)

An example of preparing a flexible heat generating body 1 by using a batchwise stirrer-equipped oxidizing gas contact treatment device composed of a mixer equipped with a rotary blade for stirring as an oxidizing gas contact treatment device will be described. Incidentally, air was used as an oxidizing gas.

A reaction mixture consisting of 100 parts by weight of an iron powder (particle size: not more than 300 $\mu$m), 5.5 parts by weight of active carbon (particle size: not more than 300 $\mu$m), 2.3 parts by weight of a wood meal (particle size: not more than 300 $\mu$m), 1.8 parts by weight of a water absorptive polymer (particle size: not more than 300 $\mu$m), 0.5 parts by weight of calcium hydroxide, 0.7 parts by weight of sodium sulfite and 5 parts by weight of 11 % salt water and having a water mobility value of less than 0.01 was charged in the batchwise stirrer-equipped oxidizing gas contact treatment device.

Next, the upper portion of the contact treatment device vessel was opened to air, and the reaction mixture was subjected to self heat generation with stirring in the opened state to air under circumstances at 20°C. When the temperature rise of the reaction mixture reached 40°C, the reaction mixture was sealed in an air-impermeable accommodating bag and cooled to room temperature to obtain a heat generating mixture. 11 % salt water was mixed in the heat generating mixture to obtain a heat generating composition having a water mobility value of 12. A napped, i.e., fluffy, non-woven fabric and a polyethylene-made porous film were used as a covering material; and a laminate of a non-fluffy non-woven fabric and polyethylene was used as a substrate. A trimming die provided with five (ten in total) cavities having a width of 5 mm and a length of 80 mm as provided in a striped form at intervals of 7 mm while interposing a central part of a width of 10 mm therebetween was used as a trimming die.

Next, an air-permeable adhesive layer in a netlike form was provided in the porous film side of the air-permeable covering material by a melt blow method using an olefin based hot melt based adhesive and put on the heat generating composition molded body and the substrate.

Thereafter, by using a temporary adhering plate, the outside of 8 mm from the two heat generating composition molded bodies of the top and outermost side of the heat generating composition molded bodies was subjected to linear temporary adhesion in a width of 10 mm in the longitudinal direction. Next, by using a heat seal plate, the surroundings of the sectional exothermic parts were sealed in a seal width of 3 mm, and the outer circumference which will become a flexible heat generating body was sealed in a seal width of 8 mm, thereby obtaining a flexible heat generating body (153 m in length x 98 mm in width).

Next, a hydrophilic adhesive layer made of a hydrophilic adhesive (gel) was provided on the substrate, and a separator was placed thereon, followed by cutting to obtain a flexible heat generating body. The separator had a bending resistance of not more than 20 mm.

The bending resistance of the flexible heat generating body was not more than 30 mm in the long side direction (direction orthogonal to the stripe direction) of the exothermic part and 80 mm or more in the short side direction (stripe direction), respectively. The bending resistance ratio was 2 or more. Since the bending resistance in one direction is very high and the bending resistance in the direction substantially perpendicular thereto is very low, handling and a feeling for use are very excellent.

Furthermore, since this flexible heat generating body could be wound up, it became compact and was convenient for accommodation. Incidentally, in this Example, since a separator having a low bending resistance was used, even a separator-provided flexible heat generating body could be wound up.

The flexible heat generating body was sealed and accommodated in an air-impermeable outer bag and allowed to stand at room temperature for 24 hours. After 24 hours, the flexible heat generating body was taken out from the outer bag; the separator was eliminated; and the flexible heat generating body was subjected to a test for the body while sticking the hydrophilic adhesive layer side thereof to a waist. Temperature characteristics, curved surface fitness, winding properties and usefulness were evaluated. As a result, the flexible heat generating body was superior in all of these evaluations.

Incidentally, the hydrophilic adhesive which constitutes the hydrophilic adhesive layer is made of 2.5 % by weight of polyacrylic acid (average molecular weight: 150,000), 4.5 % by weight of poly(sodium acrylate), 1.0 % by weight of sodium carboxymethyl cellulose, 18.0 % by weight of glycerin, 0.2 % by weight of magnesium metasilicate aluminate, 8.0 % by weight of a wood meal, 0.1 % by weight of sodium edetate, 0.1 % by weight of citric acid and 0.1 % by weight of tartaric acid, with the remainder being purified water (100 % by weight in total).

**Claims**

1. A flexible heat generating body provided with a flexible exothermic part which is prepared by mediating a plural number of a heat generating composition molded body resulting from molding a heat generating composition containing surplus water as a connecting substance between a substrate and a covering material and heat sealing the periphery of the heat generating composition molded body, thereby constituting a sectional exothermic part containing the heat generating composition molded body and a sectioned part as heat sealed, **characterized in that**:

   1) the heat generating composition contains, as essential components, an iron powder, a carbon component, a reaction accelerator and water and does not contain a flocculant aid, a flocculant, an agglomeration aid, a dry binder, a dry binding agent, a dry binding material, a sticky raw material, a thickener and an excipient;
   2) the surplus water has a water mobility value of from 0.01 to 20;
   3) the water in the heat generating composition does not function as a barrier layer;
   4) the heat generating composition molded body has a volume of from 0.01 to 30 cm$^3$;
   5) a ratio of the capacity of the sectional exothermic part to the volume of the heat generating composition molded body is from 0.6 to 1.0;
   6) the sectioned exothermic part has a maximum height of from 0.1 to 10 mm;
   7) the sectioned part has a width of from 0.3 to 30 mm;
   8) a flexibility holding member is provided in at least one side of the flexible exothermic part;
   9) a bending resistance in at least one direction of the flexible exothermic part is not more than 100 mm;
   10) a rate of bending resistance in at least one direction of the flexible exothermic part is not more than 50;
   11) a bending resistance in at least one direction of the flexibility holding member is not more than 200 mm;
   12) a bending resistance in at least the longitudinal direction of the flexible heat generating body is not more than 200 mm;
   13) the substrate is substantially planar and does not have a pocket, an accommodating division or an accommodating zone; and
   14) the substrate and/or the covering material has permeability to air in at least a part thereof.

2. The flexible heat generating body according to claim 1, **characterized in that** the flexibility holding member is constituted of a non-stretchable portion and a stretchable portion.

3. The flexible heat generating body according to claim 2, **characterized in that** the flexibility holding member is constituted of a sheet-like material in which a non-stretchable portion and a stretchable portion are integrally formed in a sheet-like form; and that the flexible exothermic part is provided in the non-stretchable portion of the sheet-like material.

4. The flexible heat generating body according to claim 1, **characterized in that** the flexible exothermic part is sandwiched by the flexibility holding members constituted of a flexible material; and that a part of the flexibility holding member is constituted of a stretchable material.

5. The flexible heat generating body according to claim 1, **characterized in that** the flexibility holding member is constituted of a longitudinal non-stretchable raw material; and that plural notch parts are alternately provided in the crossing direction to the longitudinal direction of the non-stretchable raw material, thereby forming the stretchable portion.

6. The flexible heat generating body according to claim 5, **characterized in that** the alternate notch parts have a rate of extension in the notch direction of from 1.1 to 10 times and a tensile strength of 3 N/50 mm or more.

7. The flexible heat generating body according to claim 5, **characterized in that** the alternate notch parts are provided in the vicinity of either side of the flexible exothermic part.

8. The flexible heat generating body according to claim 2, **characterized in that** the stretchable portion is constituted of a film to which stretchability is imparted by an elastomer, an expanded body, a non-woven fabric, a fabric, a laminate thereof, or a flexible material body of a scrim-supported laminate.

9. The flexible heat generating body according to claim 1, **characterized in that** the flexible heat generating body is constituted such that when wound up around a prescribed site, the both end parts thereof are superposed on each other.

10. The flexible heat generating body according to claim 1, **characterized in that** a thermal buffer sheet is provided in a portion coming into contact with an adherend of the flexible exothermic part.

11. The flexible heat generating body according to claim 1, **characterized in that** a fixing measure for fixing to an adherend is provided in the flexible heat generating body.

## FIG. 1

## FIG. 2 (a)

## FIG. 2 (b)

# FIG. 3

# FIG. 4

# FIG. 5

## F I G. 6

## F I G. 7

## F I G. 8

## FIG. 9

## FIG. 10

## FIG. 11

## FIG. 12 (a)

## FIG. 12 (b)

## FIG. 13

## FIG. 14

## FIG. 15

## FIG. 16

## FIG. 17

## FIG. 18

FIG. 19

FIG. 20

FIG. 21

## FIG. 22

## FIG. 23

## FIG. 24

## FIG. 25

## FIG. 26

## FIG. 27(a) FIG. 27(b) FIG. 27(c) FIG. 27(d)

## FIG. 27(e) FIG. 27(f) FIG. 27(g)

## FIG. 28

24

## FIG. 29

27

21

⇒

28

24

25

26

27

## FIG. 30

21

⇒

27

25

24

28

26

# F I G. 31

# FIG. 32

## INTERNATIONAL SEARCH REPORT

| | International application No. |
|---|---|
| | PCT/JP2005/013017 |

A. CLASSIFICATION OF SUBJECT MATTER
   Int.Cl$^7$ A16F7/08, 13/00, 13/02, C9K5/16

According to International Patent Classification (IPC) or to both national classification and IPC

B. FIELDS SEARCHED

Minimum documentation searched (classification system followed by classification symbols)
   Int.Cl$^7$ A16F7/08, 13/00, 13/02, C9K5/16

Documentation searched other than minimum documentation to the extent that such documents are included in the fields searched
   Jitsuyo Shinan Koho          1922-1996   Jitsuyo Shinan Toroku Koho   1996-2005
   Kokai Jitsuyo Shinan Koho    1971-2005   Toroku Jitsuyo Shinan Koho   1994-2005

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2003-334212 A  (Maikoru Kabushiki Kaisha),<br>25 November, 2003 (25.11.03),<br>Full text; Fig. 2<br>(Family: none) | 1-11 |
| A | JP 2003-336042 A  (Maikoru Kabushiki Kaisha),<br>28 November, 2003 (28.11.03),<br>Full text; Figs. 1 to 16<br>& US 2004/0217325 A      & EP 1516900 A<br>& WO 2003/097764 A1      & CA 2439044 A<br>& CN 1496395 A | 1-11 |
| E,A | JP 2005-219313 A  (Kao Corp.),<br>18 August, 2005 (18.08.05),<br>Full text; Figs. 1 to 3<br>(Family: none) | 1-11 |

[X] Further documents are listed in the continuation of Box C.      [ ] See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 09 September, 2005 (09.09.05) | 11 October, 2005 (11.10.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

## INTERNATIONAL SEARCH REPORT

| International application No. |
|---|
| PCT/JP2005/013017 |

C (Continuation).    DOCUMENTS CONSIDERED TO BE RELEVANT

| Category* | Citation of document, with indication, where appropriate, of the relevant passages | Relevant to claim No. |
|---|---|---|
| P,A | JP 2005-87719 A  (Kao Corp.),<br>07 April, 2005 (07.04.05),<br>Full text; Figs. 1 to 2<br>& WO 2005/011543 A1 | 1-11 |
| P,A | JP 2004-306560 A  (Idemitsu Unitech Co., Ltd.),<br>04 November, 2004 (04.11.04),<br>Full text; Figs. 1, 2, 4, 6<br>(Family: none) | 1-11 |
| P,A | JP 2004-208978 A  (MYCOAL PRODUCTS CORP.),<br>29 July, 2004 (29.07.04),<br>Full text; Figs. 1 to 20<br>& WO 2004/061045 A1 | 1-11 |
| A | JP 2003-334211 A  (Maikoru Kabushiki Kaisha),<br>25 November, 2003 (25.11.03),<br>Full text; Figs. 1 to 23<br>& US 2004/0149732 A1     & EP 1506756 A1<br>& WO 2003/096942 A1     & CA 2468331 A<br>& CN 1518435 A | 1-11 |
| A | JP 2003-509120 A  (The Procter & Gamble Co.),<br>11 March, 2003 (11.03.03),<br>Full text; Figs. 1, 2<br>& US 6336935 B1          & EP 1212020 A<br>& WO 2001/019302 A1     & AU 7116900 A<br>& BR 14044 A               & CN 1373649 A<br>& CA 2381812 A          & AU 768363 B | 1-11 |
| A | JP 11-512954 A  (The Procter & Gamble Co.),<br>09 November, 1999 (09.11.99),<br>Full text; Figs. 1 to 3<br>& JP 3545769 B             & WO 1997/049361 A1<br>& AU 735088 B | 1-11 |
| A | JP 11-508786 A  (The Procter & Gamble Co.),<br>03 August, 1999 (03.08.99),<br>Full text<br>& US 5918590 A1          & EP 835087 A<br>& WO 1997/001313 A2     & DE 69619111 T<br>& AT 212819 T             & DK 835087 T<br>& ZA 9605463 A           & ES 2167572 T | 1-11 |

Form PCT/ISA/210 (continuation of second sheet) (January 2004)

**INTERNATIONAL SEARCH REPORT**

| | International application No. |
|---|---|
| | PCT/JP2005/013017 |

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2001-513394 A (The Procter & Gamble Co.), 04 September, 2001 (04.09.01), Page 12, line 14 to page 33, line 12; Figs. 1 to 6 & US 5904710 A1 & EP 1021145 A & WO 1999/009918 A1 & DE 69820661 T & NO 20000846 A & BR 9811970 A & CA 2301137 A & CN 1271265 A & HU 2689 A & CZ 20000585 A & AU 739013 B & AT 256446 T & DK 1021145 T & ES 2209165 T | 2-4 |
| P,A | JP 2004-330573 A (Idemitsu Unitech Co., Ltd.), 25 November, 2004 (25.11.04), Full text; Figs. 1, 2, 5, 6, 7 & WO 2004/030904 A1 | 1 |
| Y | JP 2003-204983 A (Asahi Kasei Corp.), 22 July, 2003 (22.07.03), Column 4, line 32 to 46; page 5, line 16 to column 6, line 40 (Family: none) | 1 |
| A | JP 11-89869 A (Asahi Kasei Corp.), 06 April, 1999 (06.04.99), Full text (Family: none) | 1 |
| A | CD-ROM of the specification and drawings annexed to the request of Japanese Utility Model Application No. 53753/1993(Laid-open No. 24907/1995) (Japan Gals Co., Ltd.), 12 May, 1995 (12.05.95), Full text; Figs. 1, 10 (Family: none) | 5-7 |

Form PCT/ISA/210 (continuation of second sheet) (January 2004)

**EP 1 782 767 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001513394 A
- JP 2001507593 T
- JP 3161605 B
- JP 11508314 T
- JP 2002514104 T
- JP 4293989 A
- JP 6343658 A
- JP 7194641 A
- JP 49118963 A
- JP 49062771 A
- JP 2002501127 T
- JP 8058007 A
- JP 2002501127 A
- JP 2003520705 A
- JP 2004076178 A
- JP 2002307627 A
- JP 2002283479 A
- JP 2000514730 A
- JP 2001514093 T
- JP 2003533374 A
- JP 2002200108 A
- JP 10265373 A
- JP 9087173 A
- JP 6145050 A
- JP 6199660 A
- JP 10279466 A
- JP 10182408 A